(19) Europäisches Patentamt
European Patent Office
Office européen des brevets

(11) Publication number: **0 226 381 B1**

(12) **EUROPEAN PATENT SPECIFICATION**

(45) Date of publication of patent specification: **05.02.92**

(51) Int. Cl.5: **C07H 13/04**, C07H 15/04, C07D 309/14, A61K 31/70, A61K 31/35

(21) Application number: **86309377.9**

(22) Date of filing: **02.12.86**

(54) **Novel glucopyranose derivatives.**

(30) Priority: **06.12.85 JP 273440/85**
**06.09.86 JP 210379/86**

(43) Date of publication of application:
**24.06.87 Bulletin 87/26**

(45) Publication of the grant of the patent:
**05.02.92 Bulletin 92/06**

(84) Designated Contracting States:
**AT BE CH DE ES FR GB GR IT LI LU NL SE**

(56) References cited:
**WO-A-84/04526**
**US-A- 4 374 831**

**AGRICULTURAL AND BIOLOGICAL CHEMISTRY, vol. 48, no. 1, January 1984, pages 251-252; The Agricultural Chem. Soc. of Japan, JP M. KISO et al.: "Synthesis of biologically active, novel monosaccharide analogs of lipid A"**

**CHEMICAL & PHARMACEUTICAL BULLETIN, vol. 33, no. 10, October 1985, pages 4621-4624; Pharm. Soc. of Japan, JP T. SHIMIZU et al.: "Antitumor activity and biological effects of chemically synthesized monosaccharide analoques of lipid A in mice"**

(73) Proprietor: **ONO PHARMACEUTICAL CO., LTD.**
**No. 14, Doshomachi 2-chome Higashi-ku**
**Osaka-shi Osaka(JP)**

(72) Inventor: **Toda, Masaaki**
**3-16-12 Shimamotocho Todaiji**
**Mishima-gun Osaka(JP)**
Inventor: **Shimoji, Katsuichi**
**1-3-305 Takano Tadehara-cho**
**Sakyo-ku Kyoto(JP)**
Inventor: **Sasaki, Yutaro**
**No. 11-88-504 Okayamatecho**
**Hirakata-shi Osaka(JP)**

(74) Representative: **Pearce, Anthony Richmond et al**
**MARKS & CLERK Alpha Tower Suffolk Street**
**Oueensway Birmingham B1 1TT(GB)**

**EP 0 226 381 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Summary

This invention is related to novel glucopyranose derivatives.

More particularly, this invention is related to

(1) novel glucopyranose derivatives;

(2) processes for the preparation of them; and

(3) enhancing agent of immunity and/or anti-tumor agent containing them as active ingredient.

### Background

Gram-negative germ (e.g. cholera germ, salmonella germs, colon bacillus) have compounds so called as lipopolysuccharide (abbreviated as LPS) on the outer cell membrane, and it was thought that the compounds induced endotoxin shock.

It was known that LPS have various bioactivities included fetal toxicity this is why it is so named endotoxin. For example, LPS have a pyrogenetic action, a hemorrhage action, induce encephalomyelitis, arthritis, and have a blastogenic action (macrophage activating action, B cell mitogenic activity, producing action of non-specific antibody, enhancing activity of cellular immunity etc.) and anti-tumor action (INF (interferon) inducing action, TNF (tumor necrosis factor) inducing action etc.).

Especially, LPS is effective as non-specific immunity agent, and have an action inducing hemorrhage necrosis of tumor cell specifically by its TNF inducing activity, and therefore be useful for anti-tumor agent.

And LPS also useful for its inducing activity of IL-1 (interleukin-1) or interferon, not only their enhancing activity of cellular immunity but also stimulating of NK (natural killer) activity.

On the other hand, LPS is constituted from three kinds of materials i.e. acidic protein, macromolecular polysuccharides and phospholipid, and phospholipid have been found as its active site by Westphal, Lüdertz et al.

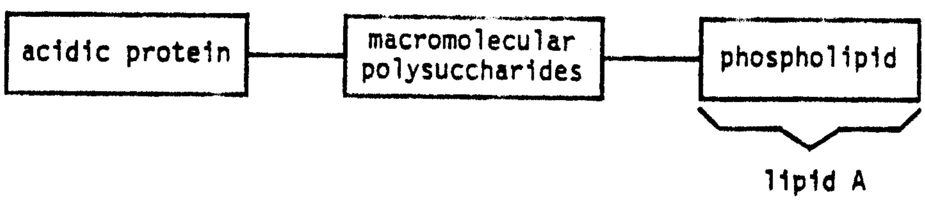


The phospholipid shown above is called lipid A, and it was known that the compound alone possess various activities like LPS.

The absolute structure of lipid A was unknown for long years, but recent studies cleared that its structure is disucchariedeamine combined with fatty acids and phosphoric acids as the following [See Nippon Saikin-gaku Zasshi 40(1), 57 (1985) and Proc. Natl. Acad. Sci. U.S.A. 80, 4624 (1983)].

Lipid A of E. Coli

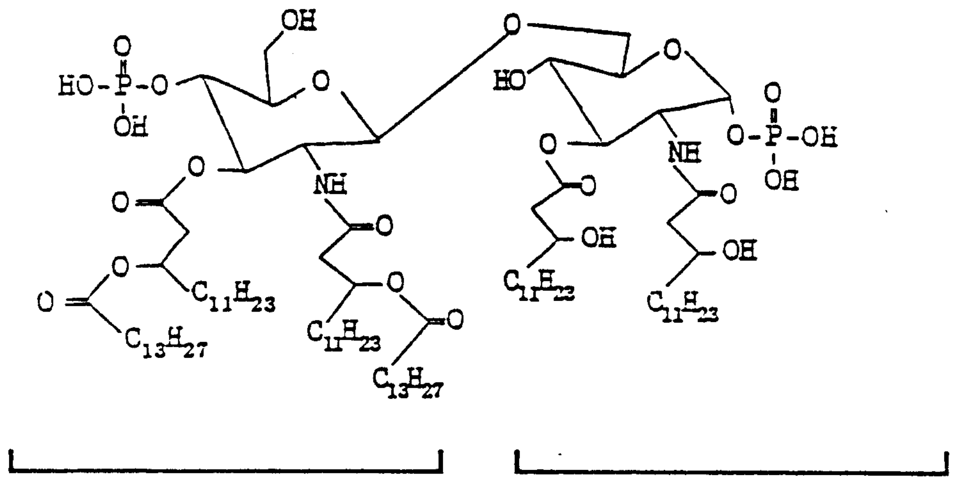

non-reducing subunit　　　reducing subunit

Result of the recent studies discovered that each subunits above possess activities like Lipid A. And especially, reducing subunit was isolated as a biosynthesis precursor and was named to lipid X [See Biol. Chem. 256, 10690 (1981) and Proc. Natl. Acad. Sci. 80, 4624 (1983)].

And compound combined with hexadecanoyl group on the hydroxy group of β-hydroxytetradecanoyl group on the 2nd position of lipid X as ester is named lipid Y.

About the above lipid A, lipid X and lipid Y, Wisconsin Alumni Research Foundation was patent applicated [See WO-8404526 or EP-143840].

Non-reducing subunits were not found in natural, and some of them were synthesized chemically, i.e. compounds of general formula:

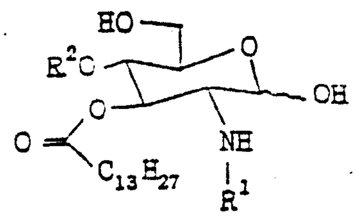

Compound 1:

$R^1 = -CO-CH_2-CH-C_{11}H_{23}$
|
$O-CO-C_{13}H_{27}$

$R^2 = -H$

Compound 2: $R^1 = -CO-C_{13}H_{27}$

$R^2 = -PO(OH)_2$

Compound 3:

$R^1 = -CO-CH_2-CH-C_{11}H_{23}$
|
$O-CO-C_{13}H_{27}$

$R^2$ = $-PO(OH)_2$

[See Agric. Biol. Chem., 48(1), 251 (1984) and FEBS LETT., 167, 226 (1984)].

Patent applications related to the above compounds were published [See Japanese Patent Publication No. 61-126093 and 61-126094].

Further, compounds replaced the hydroxy group on the 1st position into a hydrogen atom were published, i.e. compounds of general formula:

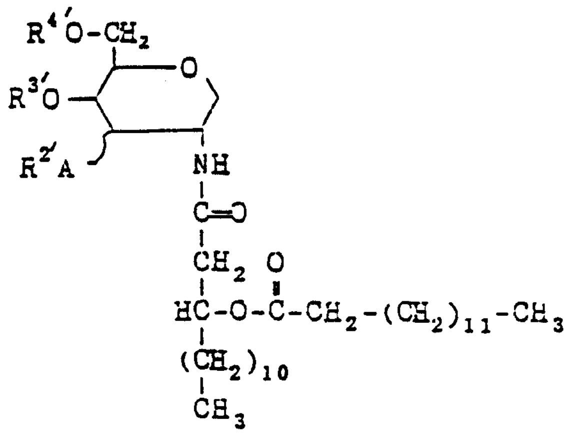

[wherein A represents O or NH, $R^{2'}$ represents $C_{14}$ or $C_{14}$-O-$C_{14}$, $R^{3'}$ and R4' represent a hydrogen atom or P.

$C_{14}$ represents tetradecanoyl group, $C_{14}$-O-$C_{14}$ represents -(3-tetradecanoyl)-tetradecanoyl group, and P represents phosphoryl group, respectively. Stereo configulation of $AR^{2'}$ on the 3rd position is $\alpha$-configulation or $\beta$-configulation.]

(See Japanese Patent Publication No. 61-172867).

Disclosure of the Invention

We, the present inventors, were successed to synthesize novel compounds which are ones introduced the following chemical modifications into non-reducing subunit:

(1) conversion of the phosphoric acid residue on the 4th position into a sulfuric acid residue,

(2) introduction an aryl group (benzene ring or naphthalene ring) into the terminal of the acyl chain on the 2nd and 3rd positions, and

(3) conversion of the hydroxy groups on the 1st and/or 6th position(s) into hydrogen atom(s) in some compounds, and we confirmed that they possess pharmacological activities like Lipid-A, and then achieved the present invention.

That is the present invention is related to the novel glucopyranose derivatives of general formula:

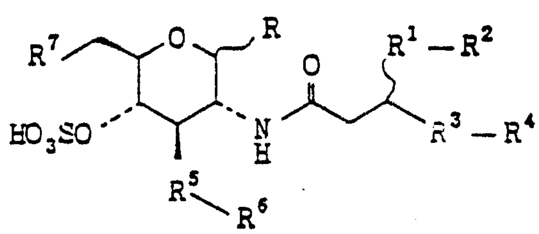

(I)

[wherein, R represents a hydrogen atom, a hydroxy group or an alkoxy group of from 1 to 4 carbon atom(s), $R^1$ represents a single bond or an oxycarbonylalkyl group of from 2 to 20 carbon atoms,

$R^2$ and $R^6$, independently, represent a hydrogen atom or a general formula:

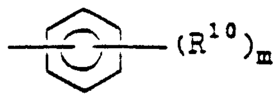 , 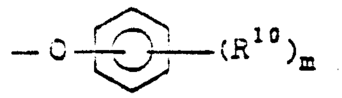 ,

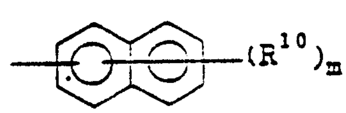 or 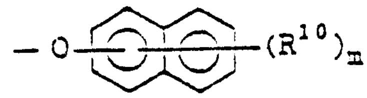

(wherein, $R^{10}$ represents a hydrogen atom, an alkyl or alkoxy group of from 1 to 7 carbon atom(s) or a halogen atom, and m represents 1, 2 or 3.), respectively,
$R^3$ represents an alkylene group of from 1 to 20 carbon atom(s),
$R^4$ represents a hydrogen atom or a general formula:

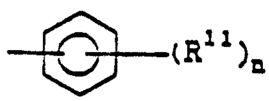 , 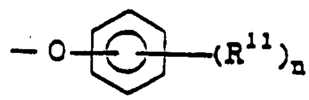 ,

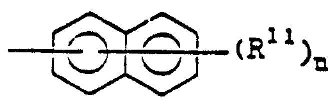 or 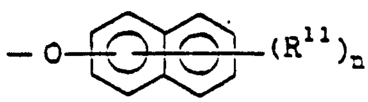

(wherein, $R^{11}$ represents a hydrogen atom, an alkyl or alkoxy group of from 1 to 7 carbon atom(s) or a halogen atom, and n represents 1, 2 or 3.),
$R^5$ represents an oxycarbonyl group of from 2 to 20 carbon atoms,
$R^7$ represents a hydrogen atom or a hydroxy group.
With the proviso that, $R^2$, $R^4$ and $R^6$ do not represent hydrogen atoms at the same time.]
or non-toxic salts thereof, processes for the preparation of them and enhancing agent of immunity and/or anti-tumor agent containing them as active ingredient.

In the general formula (I), examples of the alkoxy group of from 1 to 4 carbon atom(s) as R are methoxy, ethoxy, propoxy and butoxy groups and isomeric groups thereof, and preferable groups as R are a hydrogen atom, a hydroxy group and a methoxy group.

In the general formula (I), the oxycarbonylalkyl group of from 2 to 20 carbon atoms as $R^1$ and $R^5$ is a group of general formula:

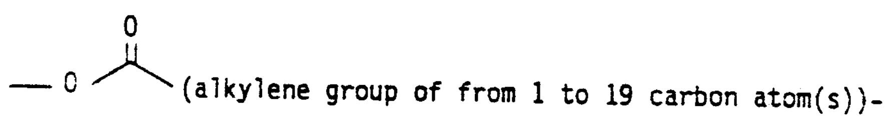

and alkylene chain side is bonded to $R^2$ group.
Alkylene group of 1 to 19 carbon atom(s) said foregoing are methylene, ethylene, trimethylene, tetramethylene, pentamethylene, hexamethylene, heptamethylene, octamethylene, nonamethylene, decamethylene, undecamethylene, dodecamethylene, tridecamethylene, tetradecamethylene, pentadecamethylene, hexadecamethylene, heptadecamethylene, octadecamethylene and nonadecamethylene groups and isomeric groups thereof, and preferable groups as $R^1$ are a single bond (a hydrogen atom as $R^2$ is preferable, in this case.), oxycarbonyltrimethylene, oxycarbonyltetramethylene, oxycarbonylhexamethylene, oxycarbonylheptamethylene, oxycarbonyloctamethylene, oxycarbonylnonamethylene, oxycarbonyldecamethylene and oxycarbonyldodecamethylene groups.

In the general formula (I), examples of the alkyl or alkoxy group of from 1 to 7 carbon atom(s) as $R^{10}$ in the group represented by $R^2$ and $R^6$, and as $R^{11}$ in the group represented by $R^4$ are methyl, ethyl, propyl, butyl, pentyl, hexyl, heptyl, methoxy, ethoxy, propoxy, butoxy, pentyloxy, hexyloxy and heptyloxy groups and isomeric groups thereof. Halogen atoms represented by $R^{10}$ and $R^{11}$ are fluorine, chlorine, bromine and iodine atoms, and preferable groups as $R^{10}$ and $R^{11}$ are a hydrogen atom, chlorine atom, methoxy group

and pentyl group.

In the general formula (I), examples of the alkylene group of from 1 to 20 carbon atom(s) are methylene, ethylene, trimethylene, tetramethylene, pentamethylene, hexamethylene, heptamethylene, octamethylene, nonamethylene, decamethylene, undecamethylene, dodecamethylene, tridecamethylene, tetradecamethylene, pentadecamethylene, hexadecamethylene, heptadecamethylene, octadecamethylene and nonadecamethylene and eicosamethylene groups and isomeric groups thereof, and preferable groups as $R^3$ especially are hexamethylene and undecamethylene groups.

In the general formula (I), all of the groups as $R^4$ are preferable.

The alkyl, alkylene and alkenylene groups foregoing described mean straight ones and branched ones, and preferable group especially are straight ones.

The compounds of the present invention of the general formula (I) possess activities like lipid A, and therefore be useful for enhancing agent and anti-tumor agent. Concretely, the compounds of the present invention possess action of activating macrophages, B cell mitogenic activity, producing action of non-specific antibody, enhancing activity of cellular immunity, as blastogenic action, and possess INF (interferon) inducing action, TNF (tumor necrosis factor) inducing action etc. as anti-tumor action. And it is expected that the compounds also possess producing action of interleukin and stimulating action of NK (natural killer) activity.

## Processes for the Preparation

The present invention is not only chemical compounds *per se* and non-toxic salts thereof but also processes for the preparation.

According to the present invention, among the compounds of the present invention of the general formula (I), compounds wherein $R^7$ is a hydroxy group, of the general formula:

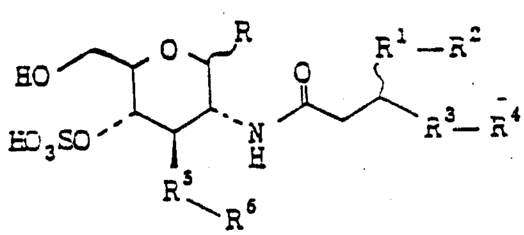

(Ia)

[wherein, all of the symbols are the same meaning as hereinbefore defined.]
may be prepared by hydrolizing a compound of general formula:

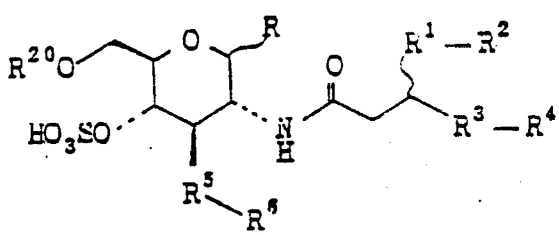

(II)

[wherein, $R^{20}$ represents a trialkylsilyl group, and the other symbols are the same meaning as hereinbefore defined.]
in an acidic condition.

Hydrolysis is well known, for example, with using an acid (acetic acid, oxalic acid, trifluoroacetic acid, hydrochloric acid etc.), in a water-miscible organic solvent (THF, methanol, ethanol, dioxane etc.), with or without water, at a temperature of from 0 °C to 70 °C.

On the other hand, among the compounds of the general formula (I), compounds wherein $R^7$ is a hydrogen atom, of general formula:

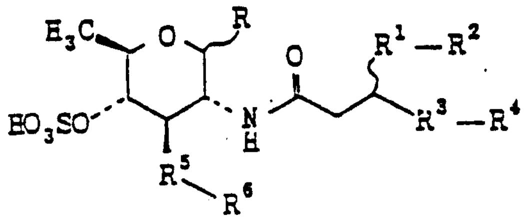

(Ib)

[wherein, all of the symbols are the same meaning as hereinbefore defined.]
may be prepared by introducing a sulfo group into the hydroxy group of the 4th-position of a compound of general formula:

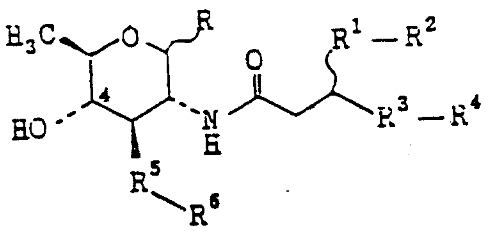

(III)

[wherein, all of the symbols are the same meaning as hereinbefore defined.].

The reaction is well known, it may be carried out, for example, in the presence of a tertiary amine (pyridine, triethylamine etc.), in an inert organic solvent (THF, methylene chloride, ethyl acetate etc.) or without solvent used, using with sulfur trioxide-pyridine complex, at a temperature of from -10°C to 60°C.

Among the compounds of the general formula (II) and (III), compounds wherein R is a hydroxy group, of general formula (IIa) and (IIIa) may be prepared by the series of reaction steps described in the following scheme [A].

Scheme [A]

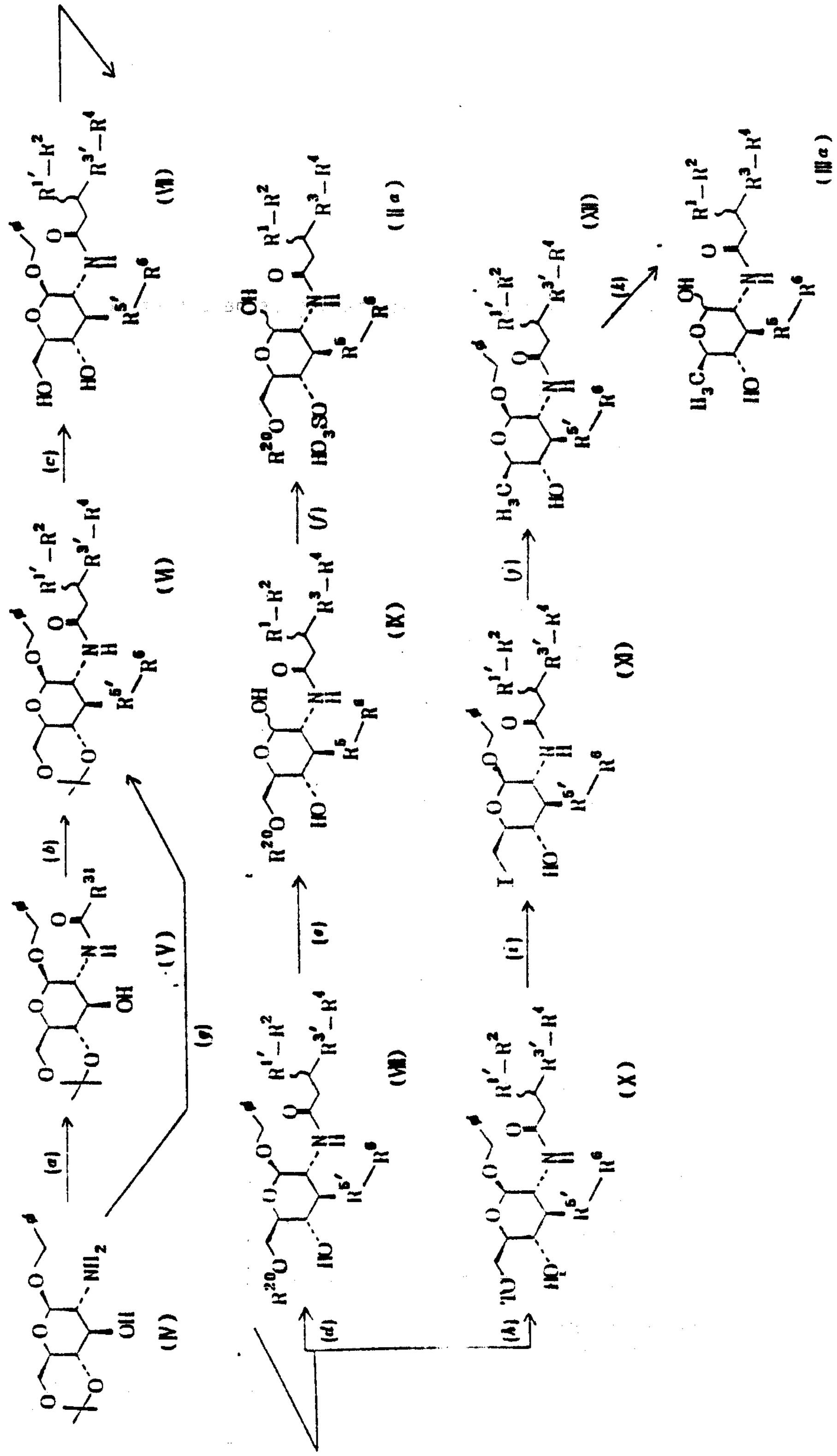


(IV) (V) (VI) (VII) (VIII) (IX) (IIa) (X) (XI) (XII) (IIIa)

Each symbols in the scheme [A] represent the following meanings or as defined hereinbefore respectively.

$R^{1'}$ - a single bond or an oxycarbonylalkyl or oxycarbonylalkenyl group of from 2 to 20 carbon atoms,

$R^{3'}$ - an alkylene or alkenylene group of from 1 to 20 carbon atom(s),

$R^{5'}$ - an oxycarbonylalkyl or oxycarbonylalkenyl group of from 2 to 20 carbon atoms,
$R^{31}$ - a group of general formula:

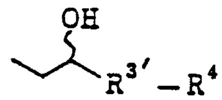

or

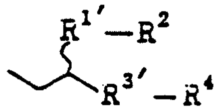

,

T - methanesulfonyl group or p-toluenesulfonyl group,
I - iodine atom.

In the scheme [A], each reaction steps are known per se, and summarized descriptions are the followings.

Step (a) is an introducing reaction of an acyl group into the amino group, i.e. N-acylation, and it may be carried out, for example, using with a mixed acid anhydride which was prepared from a carboxylic acid of general formula:

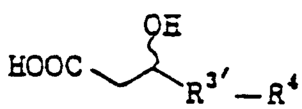

(XIII)

or

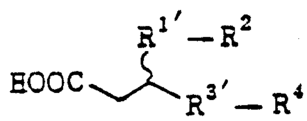

(XIV)

by reacting with pivaloyl chloride, in the presence of a suitable base (triethylamine, pyridine etc.), in an inert organic solvent (methylene chloride, toluene, benzene, hexane, THP, THF, etc.), at a temperature of from -30 °C to 30 °C, in the same condition without purification.

And, N-acylation described above may also be carried out by reacting a compound of the general formula (IV) and a carboxylic acid of the general formula (XIII) or (XIV), in the presence of a suitable base (triethylamine, pyridine, 4-(N,N-dimethylamino) pyridine etc.) in an inert organic solvent (methylene chloride, acetonitrile etc.), using with 2-chloro-N-methylpyridinium iodide, at a temperature of from -30 °C to 30 °C.

And, further, N-acylation also may be carried out by reacting a carboxylic acid of the general formula (XIII) and (XIV) in the presence of triphenylphosphine, in an inert organic solvent (methylene chloride, THF, ethyl acetate etc.), using with 2,2'-pyridyldisulfide, at a temperature of from -10 °C to 50 °C.

And, N-acylation may be carried out by other activating method of carboxylic acid, for example, using with dicyclohexylcarbodiimide.

In this N-acylation, to use the compound of the general formula (XIII) produce a compound of the general formula (IIa) wherein the substituents $-R^1-R^2$ and the substituents $-R^5-R^6$ are the same groups.

Step (b) is an introducing reaction of acyl group into the hydroxy group, i.e. O-acylation, and it may be carried out, for example, in the presence of a suitable base (triethylamine, 4-(N,N-dimethylamino)pyridine, pyridine etc.), in an inert organic solvent (methylene chloride, toluene, benzene, ethyl acetate, hexane, THF, THP etc.) or without solvent used, using with a corresponding acyl halide, at a temperature of from -10 °C to 60 °C.

And O-acylation may be carried out by reacting a corresponding acid, in the presence of a suitable amine (triethylamine, 4-(N,N-dimethylamino)pyridine, pyridine etc.) in an inert organic solvent (methylene chloride, acetonitrile etc.), using with 2-chloro-N-methylpyridinium iodide as a condencing agent, at a temperature of from -30 °C to 30 °C.

And further, O-acylation may also be carried out by other activating method of carboxylic acid, for example, using with dicyclohexylcarbodiimide.

Step (c) is a removing reaction of isopropylidene group on the 4th and 6th positions of the sugar as protecting group, and it may be carried out, for example in water or a water miscible organic solvent (THF, methanol, ethanol etc.) and water, using an acid (acetic acid, p-toluenesulfonic acid, camphorsulfonic acid, trichloroacetic acid, oxalic acid etc.), at a temperature of from 0 ° C to 80 ° C.

Step (d) is an introducing reaction of a protecting group into the hydroxy group on the 6th position selectively, and it may be carried out, for example, in the presence of a suitable base (4-(N,N-dimethylamino)pyridine, pyridine, triethylamine etc.), in an inert organic solvent (methylene chloride, toluene, benzene, ethyl acetate, hexane, THF, THP etc.) or without solvent used, using with a corresponding trialkylsilyl halide (t-butyldimethylsilyl chloride, trimethylsilyl chloride etc.), at a temperature of from -10 ° C to 60 ° C.

Step (a) is a removing reaction of benzyl group, and it may be carried out, for example, in an atmosphere of hydrogen, in an organic solvent (methanol, ethanol, THF, THP etc.), using with a hydrogenating catalyst (palladium-carbon, platinum black, nickel etc.), at a temperature of from -10 ° C to 80 ° C.

Step (f) is an introducing reaction of sulfo group into the hydroxy group on the 4th position, and it may be carried out, for example, in the presence of a tertiary wine (pyridine, triethylamine etc.), in an inert organic solvent (THF, methylene chloride, ethyl acetate etc.) or without solvent used, using sulfur trioxide-pyridine complex, at a temperature of from -10 ° C to 60 ° C.

Step (g) is acylation of the amino group on the 2nd position and the hydroxy group on the 3rd position at the same time, and it may be carried by the same procedure as described in the step (b).

Step (h) is tosylation (an introducing reaction of p-toluenesulfonyl group) or mesylation (an introducing reaction of methanesulfonyl group), and it may be carried out, for example, in an inert organic solvent (methylene chloride etc.) or without solvent used, in the presence of tertiary amine (triethylamine, pyridine, 4(N,N-dimethylamino)pyridine etc.), using with tosyl halide or mesyl halide, at a temperature of from -40 ° C to 40 ° C.

Step (i) is a replacing reaction of tosyloxy or mesyloxy group into an iodine atom, it may be carried out, for example, in an inert organic solvent (acetone etc.), using an iodide (sodium iodide, potassium iodide etc.), at a temperature of from 0 ° C to 60 ° C.

Step (j) is a removing reaction of the iodine atom, and it may be carried out, for example, in an inert organic solvent (toluene, benzene, xylene etc.), in the presence of tributylstannane and $\alpha,\alpha'$-azobisisobutyronitrile, by irradiation of light.

Step (k) is a removing reaction of benzyl group, and it may be carried out by the same procedure as described in the step (e).

Compounds of general formula (II) or (III) wherein R is a hydrogen atom or an alkoxy group of from 1 to 4 carbon atom(s) could be prepared by using compounds of general formula:

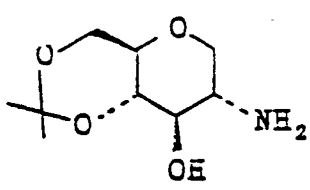

(XV)

or

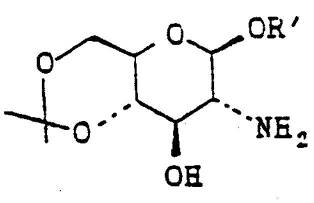

(XVI)

[wherein R' represents an alkyl group of from 1 to 4 carbon atom(s).]
as starting material instead of the compounds of the general formula (IV) shown in the scheme [A].

In this case, reaction step (e) or (k) should be omitted, and substituents with prime ($R^{1'}$, $R^{3'}$ and $R^{5'}$) should be read each substituents without prime.

Throughout the specification, in each reactions, products may be purified by conventional methods, for example, distillation at atmospheric or reduced pressure, high performance liquid chromatography, thin layer chromatography using silica gel or magnesium silicate or washing or recrystallization. Purification may be carried after each reactions or a series of reactions.

Starting materials

Starting materials and reagents using in the present invention are known compounds per se or may be prepared by known methods per se.

For example, the compounds of the general formula (IV) were described in Agric. Biol. Chem., 48(1), 251 (1984).

Salts

The compounds of the present invention of the general formula (I) may be formed salts at the sulfo group.

By converting into salts, solubility of the compounds of the present invention against water can be increased, and therefore useful at the administration as pharmaceuticals.

The compounds of the present invention may easily be converted into corresponding salts by the methods known per se, e.g. methods described hereafter.

The salts in the present invention are preferably non-toxic ones. The non-toxic salts herein referred mean salts of cations such that it is relatively innoxious to living body (animals including human beings) tissues and that the effective pharmacological properties of the compounds of the general formula (I) are not impaired by side effect(s) resulting from the cations when used in an amount required for the prevention and/or treatment. And water-soluble salts are preferable.

Suitable salts include, for example, a salt of an alkali metal such as sodium, potassium, a salt of an alkaline earth metal such as calcium, magnesium, an ammonium salt and a pharmaceutically acceptable (non-toxic) amine salt.

Amines suitable for forming such salts with sulfo group are well known and include, for example, those amines which are theoretically obtained by substituting one or more of hydrogen atom(s) of ammonia by other group(s). These groups, which may be the same or different when one or more hydrogen atom(s) are substituted, are selected from, for example, alkyl group(s) of from 1 to 6 carbon atom(s) and hydroxyalkyl group(s) of from 1 to 3 carbon atom(s). Suitable non-toxic amine salts include salts of a tetraalkylammonium group, such as tetramethylammonium salt and salts of an organic amine, such as methylamine, dimethylamine, cyclopentylamine, benzylamine, phenetylamine, piperidine, monoethanolamine, diethanolamine, lysine and alginine.

Salts can be obtained from the compounds of the present invention of the general formula (I), by methods known per se, for example, by reacting the compound of the general formula (I) and a suitable base such as a hydroxide or carbonate of an alkali metal or alkaline earth metal, ammonium hydroxide, ammonia or an organic amine in theoretical amounts in an appropriate solvent.

The salts can be isolated by freeze-drying the solution, or by filtration if the salts are sufficiently insoluble to the reaction solution, or if necessary, by removing part of the solvent followed by filtration.

Pharmacological activities

The compounds of the present invention of the general formula (I) possess lipid A like activities described hereinbefore, for example, in a standard laboratory test, results in the followings are given.

(1) Mitogenic activity in vitro

The compound of the present invention showed activities as in the following Table I, with the test system described hereafter.

Table I

| Example No. of the compounds | Blastgenic Index |
|---|---|
| 1(c) | 30 |
| 1(d) | 28 |
| 1(f) | 23 |
| 1(h) | 37 |
| 1(j) | 25 |
| 1(r) | 17 |
| 1(o) | 17 |
| 1(u) | 10 |
| 1(s) | 58 |
| 1(t) | 50 |
| 2 | 33 |

Mitogenic activity means activity of activating mitogenic cell division, and in this test system, taking amount of tymidine into lympha cells was measured and ratio to the one of blank control cell (Blastgenic index) was calculated.

For example, adding the compound prepared in example 1(c) to the test system induce 30 times amount of tymidine was taken into the lympha cells.

The amounts were measured by dencity of radiolable.

Mytogenic activity in vitro was measured by the following method (See Procedure Method of Immunoexperiment pp 315 published by Japan Immonology Society).

C3H/He male mice of 6 weeks old were killed by bloodletting, and spleens were isolated and homoginised. Homoginising liquids were filtered with gauze, and suspending cells were prepared with PBS solution. After washing, lymphacytes fractions were gathered with lympholight M. Lymphacytes were suspended in RPMI 1640 cultivation solution (10% FEBS), and the solution was divided into 5 x $10^5$ cells/180 μℓ/wells. Ten times concentration of the terminal concentration (1 μg/mℓ) of the compounds of the present invention were added with 20 μℓ portions to the each wells.

In an atmosphere of a mixed gas of 5% $CO_2$- 95% $O_2$, the cells were cultivated for 24 hrs at 37° C. After cultivation, $^3$H-tymidine 0.5 μCi/20 μℓ was added to the each wells. And more 24 hrs cultivation was continued at 37° C, and taking amounts of $^3$H-tymidine into the cells were measured. Mitogenic activities were showed by Blastogenic index in the followings.

$$\text{Blastgenic index} = \frac{\text{Taking amount of } ^3\text{H-tymidine into the lymphacytes with adding the compound of the present invention}}{\text{Taking amount of } ^3\text{H-tymidine into the blank control lymphacytes}}$$

(2) Inducing activities of TNF in vitro

The compounds of the present invention showed activities as in the following Table II, with the test system described hereafter.

Table II

| Example No. of the compounds | Cytotoxicities (%) |
|---|---|
| 1(c) | 86 |
| 1(d) | 95 |
| 1(h) | 81 |
| 1(j) | 69 |
| 1(r) | 86 |
| 1(s) | 100 |

Inducing activity of TNF in vitro was measured by the following test system.

Corye parvum (manufactured by Ribi Immuno Chem. Research Inc.) was administered to ICR male mice of 6-week old from tail vein as priming agent. Ten days' after, the compounds of the present invention (10 $\mu$g) were administered from tail vein as eliciting agent. 90 mins' after of the administration, bloods were collected from heart, and blood serums were separated.

Mice L-M cells ($10^4$/100 $\mu\ell$/well) suspended in a mixture of the diluted serum solution (100 $\mu\ell$/well) and RPMI 1640 cultivating solution (10% FEBS and 0.2% glucose; manufactured by Nissui Pharmaceutical Co.) were cultivated in an atmosphere of a mixed gas of 5% $CO_2$ - 95% $O_2$, for 48 hrs at 37° C, after adding $^3$H-tymidine (0.5 $\mu$Ci/20 $\mu\ell$/well). Taking amounts of $^3$H-tymidine into cells during the cultivation were measured.

Inducing activities of TNF were showed by cytotoxicity in the followings.

$$\text{Cytotoxicity (\%)} = \left(1 - \frac{\text{Taking amount of } ^3\text{H-tymidine into the lymphacytes with adding the compound of the present invention}}{\text{Taking amount of } ^3\text{H-tymidine into the blank control lymphacytes}}\right) \times 100$$

Toxicity

On the other hand, it was confirmed that the acute toxicity ($LD_{50}$) of the compounds of the present invention were more than 100 mg/kg animal body weight by intravenous administration. Therefore, the compounds of the present invention may be considered to be sufficiently safe and suitable for pharmaceutical use.

For example, the values of $LD_{50}$ of the compound prepared in example 1(c) was more than 300 mg/kg animal body weight by intravenous administration in mice.

Application for Pharmaceuticals

In mammals including human beings, especially human beings, decrease of immunity by ageing, disorders including immuno deficiency induce fatal infections e.g. opportunistic infection.

The compounds of the present invention of the general formula (I) possess enhancing activity of cellular immunity (e.g. mitogenic activity) to living tissue as shown in the experiment hereinbefore, and therefore be useful for enhancing agent of immunity.

And, the compounds of the present invention of the general formula (I) possess and inducing activity of TNF, an inducing activity of IL-1 and an inducing activity of IFN, and therefore be useful for anti-tumor agent.

For the purpose hereinbefore described, the compounds of the present invention of the general formula (I) or non-toxic salts thereof may normally be administered systemically or partially; usually by oral or parenteral administration.

The doses to be administered are determined depending upon age, body weight, symptom, the desired therapeutic effect, the route of administration, and the duration of the treatment etc. In the human adult, the doses per person per dose are generally between 0.1 mg and 100 mg, by oral administration, up to several times per day, and between 10 $\mu$g and 10 mg, by parenteral administration up to several times per day.

As mentioned above, the doses to be used depend upon various conditions. Therefore, there are cases in which doses lower than or greater than the ranges specified above may be used.

Solid compositions according to the present invention for oral administration include compressed tablets, dispersible powders and granules. In such solid compositions, one or more of the active compound(s) is or are, admixed with at least one inert diluent such as lactose, mannitol, glucose, hydroxypropylcellulose, microcrystalline cellulose, starch, polyvinylpyrrolidone or magnesium metasilicate aluminate. The compositions may also comprise, as is normal practice, additional substances other than inert diluents e.g. lubricating agents such as magnesium stearate, disintegrating agents such as cellulose calcium gluconate, and assistant for dissolving e.g. arginine, glutamic acid or amino-acid such as aspartic acid. The tablets or pills may, if desired, be made into gastric film-coated or enteric film-coated tablets or pills, such as sugar-coated, gelatin-coated, hydroxypropyl cellulose-coated or hydroxypropylmethyl cellulose phthalate-coated tablets or pills and, two or more of layers may be used. The compositions for oral administration also include capsules of absorbable material such as gelatin.

Liquid compositions for oral administration include pharmaceutically-acceptable emulsions, solutions, suspensions, syrups and elixirs containing inert diluents commonly used in the art such as distilled water or ethanol. Besides inert diluents such compositions may also comprise adjuvants such as wetting and suspending agents, and sweetening, flavouring, perfuming and preserving agents.

Other compositions for oral administration include spray compositions which may be prepared by known methods and which comprise one or are of the active compound(s).

Preparations for injection according to the present invention for parenteral administration include sterile aqueous or non-aqueous solutions, suspensions or emulsions. Example of aqueous solvents or suspending media are distilled water for injection and physiological salt solution. Examples of non-aqueous solvents or suspending media are propylene glycol, polyethylene glycol, vegetable oils such as olive oil, alcohols such as ethanol, POLYSORBATE 80 (registered Trade Mark). These compositions may also include adjuvants such as preserving, wetting, emulsifying, dispersing agents and assistant agent for dissolving (e.g. arginine, glutamic acid or amino-acid such as aspartic acid). They may be sterilized, for example, by filtration through a bacteria-retaining filter, by incorporation of sterilizing agents in the compositions or by irradiation. They may also be manufactured in the form of sterile solid compositions which can be dissolved in sterile water or some other sterile injectable medium immediately before use.

Other compositions for parenteral administration include liquids for external use, and endermic liniments such as ointments, suppositories and pessaries which comprise one or more of the active compound(s) and may be prepared by known methods.

Nomenclature

Throughout the specification including claims, the compounds of the present invention and named as derivatives of glucopyranose or 1,5-anhydro-glucitol having the following structure.

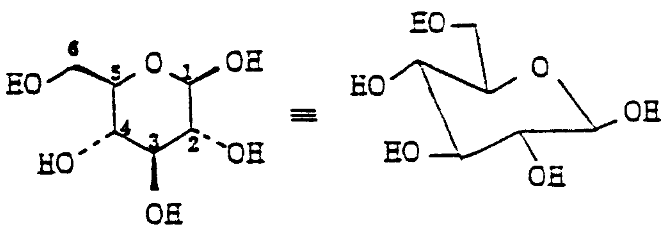

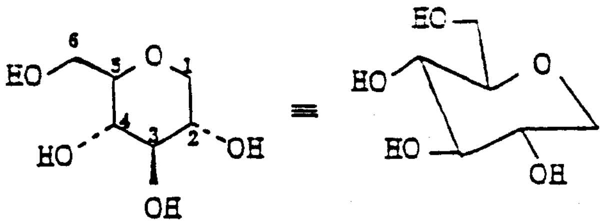

In the structural formula of the specification including claims, the dotted line (---), the thickened line (◢) and the wavy line (~~) indicate that the respective group attached thereto is in the backside of the plane, i.e. in α-configulation, in the front of the plane, i.e. in β-configulation, and in α- or β-configulation or a mixture thereof, respectively, according to the generally accepted nomenclatural rule.

Reference examples and Examples

The following reference examples and examples are illustrate the present invention, but not limit the present invention.

In the reference examples and examples, "TLC" and "IR" represent "Thin layer chromatography" and "Infrared absorption spectrum", respectively.

The solvents in the parentheses show the developing or eluting solvents and the ratios of the solvents used are by volume in chromatographic separations.

Unless otherwise specified, "IR" was measured by KBr tablet method.

Reference example 1 and 1(a)

Synthesis of benzyl 2-deoxy-2-(3R-hydroxytetradecanoyl)amino-4,6-0-isopropylidene-β-D-glucopyranoside (reference example 1) and benzyl 2-deoxy-2-(3S-hydroxytetradecanoyl)amino-4,6-0-isopropylidene-β-D-glucopyranoside (reference example 1(a)).

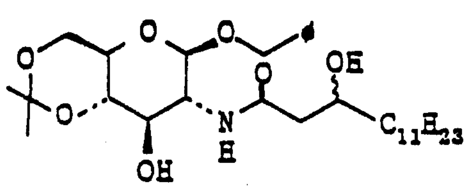

Pivaloyl chloride (1.37 mℓ) was added to an ice-cooled solution of β-hydroxymiristic acid (2.60 g) in methylene chloride (20 mℓ), and the mixture was stirred for 30 mins. The solution was added slowly to an ice-cooled solution of benzyl 2-deoxy-2-amino-4,6-0-isopropylidene-β-D-glucopyranoside (Agric. Biol. Chem., 48(1), 251 (1984); 2.6 g) and triethylamine (1.67 mℓ) in methylene chloride (20 mℓ).

The reaction mixture was stirred for 30 mins with ice-cooling and further for 2 hrs at room temperature

and then diluted with a mixture of hexane and ethyl acetate (n-$C_6H_{14}$:EtOAc = 1:2; 200 mℓ). The solution was washed with 1N hydrochloric acid, water and brine, successively dried and evaporated. The residue was purified by column chromatography on silica gel (EtOAc:$CH_2Cℓ_2$ = 3:2) to give the title compounds (3R-compound: 1.71 g; 3S-compound: 2.69 g) having the following physical data.

3R-compound:

TLC: Rf 0.31 (EtOAc:$CH_2Cℓ_2$ = 3:2);
IR : ν 3500, 3300, 1645, 1630, 1540, 1460, 1450, 1380, 1370, 1260, 1200, 1170, 1080, 1035, 935, 855, 735, 694 $cm^{-1}$

3S-compound:

TLC: Rf 0.22 (EtOAc:$CH_2Cℓ_2$ = 3:2);
IR : same as 3R-compound

Reference example 1(b) - 1(f)

By the same procedure as reference example 1, following compounds having the physical data shown in the Table III were given.

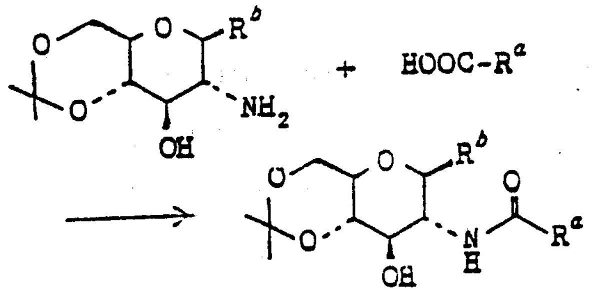

Table III

| No. | $-R^b$, $-R^a$ | Name | TLC | IR ($cm^{-1}$) |
|---|---|---|---|---|
| 1 (b) | $R^b$ : 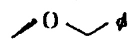<br>$R^a$ : 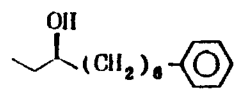 | benzyl 2-deoxy-2-(3R-hydroxy-9-phenylnonamoyl)amino-4,6-O-isopropylidene-β-D-glucopyranoside | Rf 0.45<br>(EtOAc) | ν (liquid film)<br>3370, 3330, 2910, 1730, 1630 |
| 1 (c) | $R^b$ : 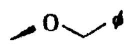<br>$R^a$ : 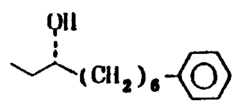 | benzyl 2-deoxy-2-(3S-hydroxy-9-phenylnonanoyl)amino-4,6-O-isopropylidene-β-D-glucopyranoside | Rf 0.36<br>(EtOAc) | ν 3400, 2940, 2860, 1650, 1600 |
| 1 (d) | $-R^b$ : -H<br>$-R^a$ : 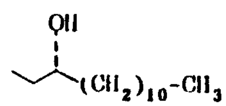 | 2-deoxy-2-(3R-hydroxytetradecanoyloxy)amino-4,6-O-isopropylidene-1,5-anhydro-D-glucitol | Rf 0.24<br>(EtOAc) | ν 3300, 2925, 2850, 1640, 1550, 1465, 1380 |
| 1 (e) | $-R^b$ : -H<br>$-R^a$ : 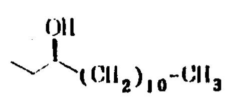 | 2-deoxy-2-(3S-hydroxytetradecanoyloxy)amino-4,6-O-isopropylidene-1,5-anhydro-D-glucitol | Rf 0.21<br>(EtOAc) | |

| No. | $-R^b$, $-R^a$ | Name | TLC | IR ($cm^{-1}$) |
|---|---|---|---|---|
| 1 (f) | $-R^b$ : $-R^a$ : 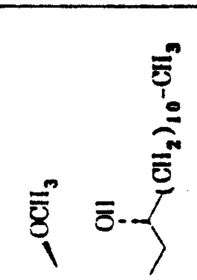 | methyl 2-deoxy-2-(3R-hydroxytetradecanoyloxy) amino-4,6-O-isopropylidene-β-D-glucopyranoside | Rf 0.21 (EtOAc) | ν 3470-3280, 2920, 2850, 1640, 1545, 1465, 1380 |

Reference example 2

Synthesis of benzyl 2-deoxy-2-[3R-(4-phenylbutoxy)tetradecanoyl] amino-3-0-(4-phenylbutyryl)-4,6-0-isopropylidene-β-D-glucopyranoside

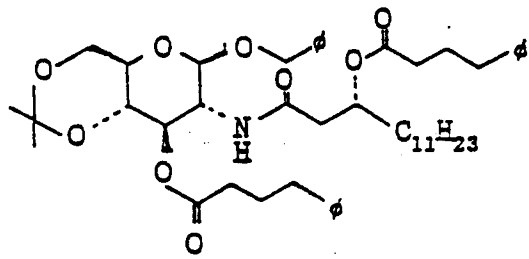

Phenylbutanoic acid (361 mg) and then 2-chloro-1-methylpyridinium iodide (767 mg) and triethylamine (0.84 mℓ) were added to a solution of the compound (535 mg) prepared in reference example 1 dissolved into methylene chloride (10 mℓ). The mixture was stirred for 30 mins with ice-cooling. 4-(N,N-dimethylamino) pyridine (61 mg) was added to the reaction solution at room temperature and the mixture was stirred for 18 hrs.

The reaction solution was washed with water, dil. hydrochloric acid and an aqueous saturated solution of sodium bicarbonate, dried and evaporated. The residue was purified by column chromatography on silica gel (EtOAc:$CH_2C\ell_2$ = 1:9) to give the title compound having the following physical data.

TLC: Rf 0.55 (EtOAc:$CH_2C\ell_2$ = 1:9);

IR : ν 3320, 2900, 2840, 1720, 1645, 1515, 1445, 1370, 1190, 1165, 1080, 1020, 850, 725, 690 $cm^{-1}$

Reference example 2(a) - 2(z)

By the same procedure as reference example 2, using with each starting materials, following compounds having the physical data shown in the Table IV were given.

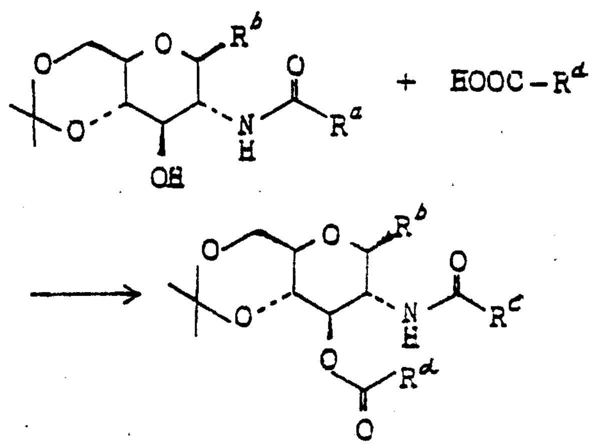

Table IV

| No. | $R^b$, $R^c$, $R^d$ | Name | Starting Material | TLC | IR ($cm^{-1}$) |
|---|---|---|---|---|---|
| 2 (a) | 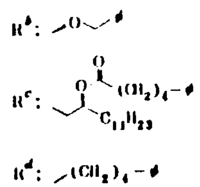 | benzyl 2-deoxy-2-[3S-(5-phenylpentanoyloxy)tetradecanoyl]amino-3-O-(5-phenylpentanoyl)-4,6-O-isopropylidene-β-D-glucopyranoside | Reference Example 1 (a) | Rf 0.78 ($CH_2Cl_2$: EtOAc = 9:1) | |
| 2 (b) | 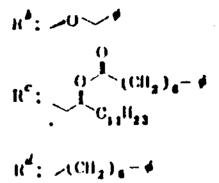 | benzyl 2-deoxy-2-[3S-(7-phenylheptanoyloxy)tetradecanoyl]amino-3-O-(7-phenylheptanoyl)-4,6-O-isopropylidene-β-D-glucopyranoside | Reference Example 1 (a) | Rf 0.79 ($CH_2Cl_2$: EtOAc = 9:1) | |
| 2 (c) | 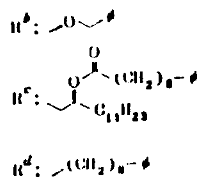 | benzyl 2-deoxy-2-[3S-(9-phenylnonanoyloxy)tetradecanoyl]amino-3-O-(9-phenylnonanoyl)-4,6-O-isopropylidene-β-D-glucopyranoside | Reference Example 1 (a) | Rf 0.78 ($CH_2Cl_2$: EtOAc = 9:1) | |
| 2 (d) | 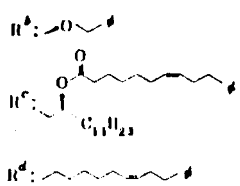 | benzyl 2-deoxy-2-[3S-(10-phenyl-7Z-decenoyloxy)tetradecanoyl]amino-3-O-(10-phenyl-7Z-decenoyl)-4,6-O-isopropylidene-β-D-glucopyranoside | Reference Example 1 (a) | Rf 0.60 ($CH_2Cl_2$: EtOAc = 95:5) | |

| No. | $R^b$, $R^c$, $R^d$ | Name | Starting Material | TLC | IR ($cm^{-1}$) |
|---|---|---|---|---|---|
| 2 (e) | 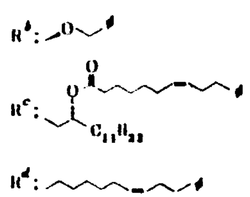 | benzyl 2-deoxy-2-[3R-(10-phenyl-7Z-decenoyloxy)tetradecanoyl]amino-3-O-(10-phenyl-7Z-decenoyl)-4,6-O-isopropylidene-β-D-glucopyranoside | Reference Example 1 | | ν 3460, 2980, 2860, 1730, 1680, 1600, 1510, 1500, 1450, 1380, 1310, 1270, 1180, 1090, 860, 700 |
| 2 (f) | 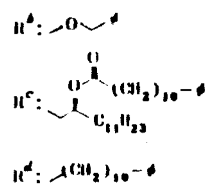 | benzyl 2-deoxy-2-[3S-(10-phenylundecanoyloxy)tetradecanoyl]amino-3-O-(10-phenylundecanoyl)-4,6-O-isopropylidene-β-D-glucopyranoside | Reference Example 1 (a) | | |
| 2 (g) | 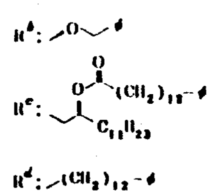 | benzyl 2-deoxy-2-[3S-(13-phenyltridecanoyloxy)tetradecanoyl]amino-3-O-(13-phenyltridecanoyl)-4,6-O-isopropylidene-β-D-glucopyranoside | Reference Example 1 (a) | Rf 0.86 ($CH_2Cl_2$: $CH_3OH$ = 95:5) | |
| 2 (h) | 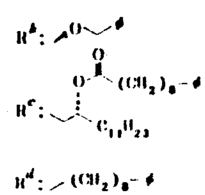 | benzyl 2-deoxy-2-[3R-(9-phenylnonanoyloxy)tetradecanoyl]amino-3-O-(9-phenylnonanoyl)-4,6-O-isopropylidene-β-D-glucopyranoside | Reference Example 1 | Rf 0.69 (EtOAc: n-$C_6H_{14}$ = 3:5) | ν 3350, 2910, 1850, 1730, 1680, 1520 |

| No. | $R^b$, $R^c$, $R^d$ | Name | Starting Material | TLC | IR ($cm^{-1}$) |
|---|---|---|---|---|---|
| 2 (i) | 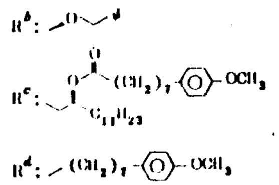 | benzyl 2-deoxy-2-[3S-[8-(4-methoxyphenyl)octanoyloxy]tetradecanoyl]amino-3-O-[8-(4-methoxyphenyl)octanoyl]-4,6-O-isopropylidene-β-D-glucopyranoside | Reference Example 1 (a) | Rf 0.78 (EtOAc: n-$C_6H_{14}$ = 1:1) | ν 3410, 3350, 1730, 1710, 1650, 1600 |
| 2 (j) | 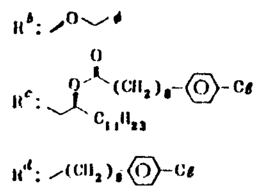 | benzyl 2-deoxy-2-[3S-[9-(4-chlorophenyl)nonanoyloxy]tetradecanoyl]amino-3-O-[9-(4-chlorophenyl)nonanoyl]-4,6-O-isopropylidene-β-D-glucopyranoside | Reference Example 1 (a) | | ν 3350, 2900, 2840, 1720, 1660, 1520, 1490 |
| 2 (k) | 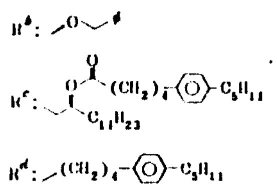 | benzyl 2-deoxy-2-[3S-[5-(4-pentylphenyl)pentanoyloxy]tetradecanoyl]amino-3-O-[5-(4-pentylphenyl)pentanoyl]-4,6-O-isopropylidene-β-D-glucopyranoside | Reference Example 1 (a) | Rf 0.82 (EtOAc: n-$C_6H_{14}$ = 3:5) | ν 3350, 2910, 2850, 1730, 1650, 1520 |
| 2 (l) | 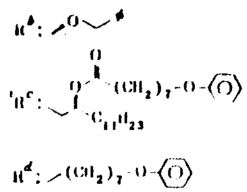 | benzyl 2-deoxy-2-[3S-(8-phenoxyoctanoyloxy)tetradecanoyl]amino-3-O-(8-phenoxyoctanoyl)-4,6-O-isopropylidene-β-D-glucopyranoside | Reference Example 1 (a) | Rf 0.69 (EtOAc | ν (liquid film) 3300, 2920, 2850, 1730, 1660, 1600 |

0226381

| No. | $R^b$, $R^c$, $R^d$ | Name | Starting Material | TLC | IR ($cm^{-1}$) |
|---|---|---|---|---|---|
| 2 (m) | 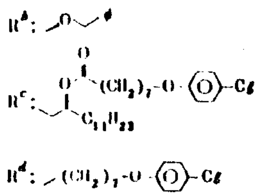 | benzyl 2-deoxy-2-[3S-[8-(4-chlorophenoxy)octanoyloxy]tetradecanoyl]amino-3-O-[8-(4-chlorophenoxy)octanoyl]-4,6-O-isopropylidene-β-D-glucopyranoside | Reference Example 1 (a) | | ν (liquid film) 3320, 2920, 2850, 1730, 1660, 1590 |
| 2 (n) | 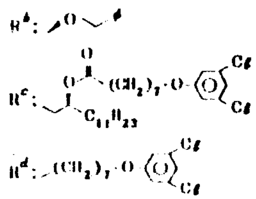 | benzyl 2-deoxy-2-[3S-[8-(3,5-dichlorophenoxy)octadecanoyloxy]tetradecanoyl]amino-3-O-[8-(3,5-dichlorophenoxy)octanoyl]-4,6-O-isopropylidene-β-D-glucopyranoside | Reference Example 1 (a) | Rf 0.65 (EtOAc: n-$C_6H_{14}$ = 3:5) | ν (liquid film) 3270, 2920, 2850, 1730, 1650, 1580, 1560 |
| 2 (o) | 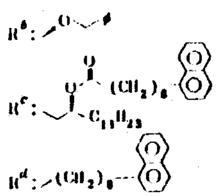 | benzyl 2-deoxy-2-[3S-[9-(1-naphthyl)nonanoyloxy]tetradecanoyl]amino-3-O-[9-(1-naphthyl)nonanoyl]-4,6-O-isopropylidene-β-D-glucopyranoside | Reference Example 1 (a) | Rf 0.79 (EtOAc: n-$C_6H_{14}$ = 3:5) | ν (liquid film) 3280, 2910, 2850, 1730, 1650 |
| 2 (p) | 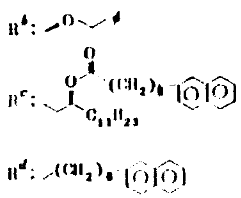 | benzyl 2-deoxy-2-[3S-[9-(2-naphthyl)nonanoyloxy]tetradecanoyl]amino-3-O-[9-(2-naphthyl)nonanoyl]-4,6-O-isopropylidene-β-D-glucopyranoside | Reference Example 1 (a) | | |

| No. | $R^b$, $R^c$, $R^d$ | Name | Starting Material | TLC | IR ($cm^{-1}$) |
|---|---|---|---|---|---|
| 2 (q) | 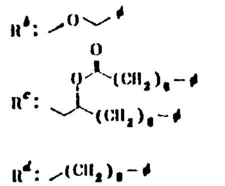 | benzyl 2-deoxy-2-[3R-(9-phenylnonanoyloxy)-9-phenylnonanoyl]amino-3-O-(9-phenylnonanoyl)-4,6-O-isopropylidene-β-D-glucopyranoside | Reference Example 1 (b) | Rf 0.78 (EtOAc: n-$C_6H_{14}$ = 1:1) | $\nu$ 3450, 3320, 2920, 2840, 1730, 1650, 1600 |
| 2 (r) | 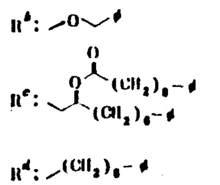 | benzyl 2-deoxy-2-[3S-(9-phenylnonanoyloxy)-9-phenylnonanoyl]amino-3-O-(9-phenylnonanoyl)-4,6-O-isopropylidene-β-D-glucopyranoside | Reference Example 1 (c) | Rf 0.74 (EtOAc: n-$C_6H_{14}$ = 1:1) | $\nu$ 3350, 2920, 2850, 1730, 1720, 1650, 1600 |
| 2 (s) | 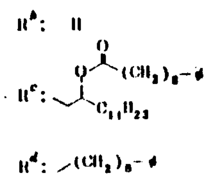 | 2-deoxy-2-[3R-(9-phenylnonanoyloxy)tetradecanoyl]amino-3-O-(9-phenylnonanoyl)-4,6-O-isopropylidene-1,5-anhydro-D-glucitol | Reference Example 1 (d) | Rf 0.25 (EtOAc: n-$C_6H_{14}$ = 1:2) | $\nu$ 3400, 2920, 2850, 1740, 1660, 1600, 1510, 1460, 1370 |
| 2 (t) | 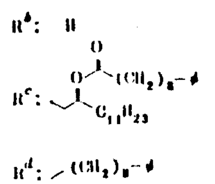 | 2-deoxy-2-[3S-(9-phenylnonanoyloxy)tetradecanoyl]amino-3-O-(9-phenylnonanoyl)-4,6-O-isopropylidene-1,5-anhydro-D-glucitol | Reference Example 1 (e) | Rf 0.25 (EtOAc: n-$C_6H_{14}$ = 1:2) | $\nu$ 3400, 2920, 2850, 1740, 1660, 1600, 1510, 1460, 1370 |

| No. | $R^b$, $R^c$, $R^d$ | Name | Starting Material | TLC | IR ($cm^{-1}$) |
|---|---|---|---|---|---|
| 2 (u) | 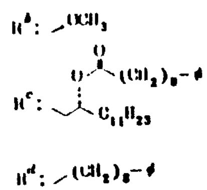 | methyl 2-deoxy-2-[3R-(9-phenylnonanoyloxy)tetradecanoyl]amino-3-O-(9-phenylnonanoyl)-4,6-isopropylidene-β-D-glucopyranoside | Reference Example 1 (f) | Rf 0.52 (EtOAc: n-$C_6H_{14}$ = 1:1) | |

| No. | $R^b$, $R^c$, $R^d$ | Name | Starting Material | TLC | IR ($cm^{-1}$) |
|---|---|---|---|---|---|
| 2 (v) | $R^b$ : -H<br>$R^c$ : 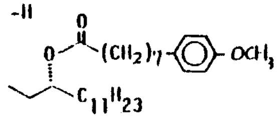<br>$R^d$ : 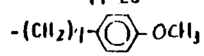 | 2-deoxy-2-[3R-[8-(4-methoxyphenyl)octanoyloxy]tetradecanoyl]amino-3-O-[8-(4-methoxyphenyl)octanoyl]-4,6-O-isopropylidene-1,5-anhydro-D-glucitol | Reference Example 1 (d) | Rf 0.78 (EtOAc: n-$C_6H_{14}$ = 1:1) | ν 1730, 1710, 1650, 1600 |
| 2 (w) | $R^b$ : -H<br>$R^c$ : 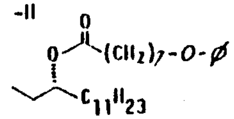<br>$R^d$ : $-(CH_2)_7-O-\phi$ | 2-deoxy-2-[3R-(8-phenoxyoctanoyloxy)tetradecanoyl]amino-3-O-(8-phenoxyoctanoyl)-4,6-O-isopropylidene-1,5-anhydro-D-glucitol | Reference Example 1 (d) | Rf 0.80 (EtOAc: n-$C_6H_{14}$ = 1:1) | ν 1730, 1660, 1600, 1540 |
| 2 (x) | $R^b$ : -H<br>$R^c$ : 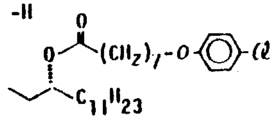<br>$R^d$ : 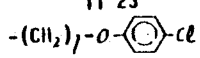 | 2-deoxy-2-[3R-[8-(4-chlorophenoxy)octanoyloxy]tetradecanoyl]amino-3-O-[8-(4-chlorophenoxy)octanoyl]-4,6-O-isopropylidene-1,5-anhydro-D-glucitol | Reference Example 1 (d) | Rf 0.81 (n-$C_6H_{14}$: EtOAc = 1:1) | ν 1730, 1660, 1590 |
| 2 (y) | $R^b$ : -H<br>$R^c$ : 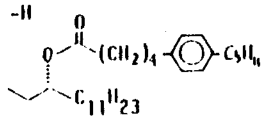<br>$R^d$ : 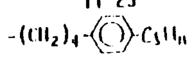 | 2-deoxy-2-[3R-[5-(4-pentylphenyl)pentanoyloxy]tetradecanoyl]amino-3-O-[5-(4-pentylphdnyl)pentanoyl]-4,6-O-isopropylidene-1,5-anhydro-D-glucitol | Reference Example 1 (d) | Rf 0.81 (n-$C_6H_{14}$: EtOAc = 3:5) | ν ($CHCl_3$ solution) 1730, 1650, 1520 |

| No. | $R^b$, $R^c$, $R^d$ | Name | Starting Material | TLC | IR ($cm^{-1}$) |
|---|---|---|---|---|---|
| 2 (z) | $R^b$ : -H<br>$R^c$ :<br>$R^d$ : 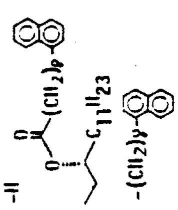 | 2-deoxy-2-[3R-[9-(1-naphthyl)octanoyloxy] tetradecanoyl]amino-3-O-[9-(1-naphthyl) nonanoyl]-4,6-O-isopropylidene-1,5-anhydro-D-glucitol | Reference Example 1 (d) | Rf 0.24 ($n-C_6H_{14}$: EtOAc = 2:1) | ($CHCl_3$ solution) ν 3270, 1730, 1652, 1543, 860, 780 |

<u>Reference</u> <u>example</u> <u>3</u>

Synthesis of benzyl 2-deoxy-2-[3R-(4-phenylbutyryloxy) tetradecanoyl]amino-3-0-(4-phenylbutyryl)-β-D-glucopyranoside

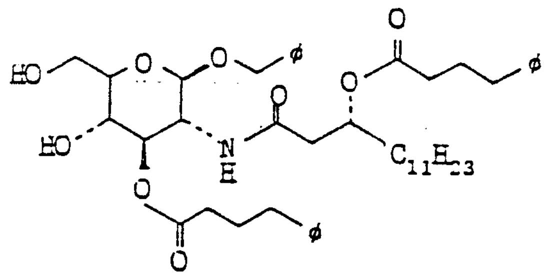

Water (3 mℓ) and acetic acid (9 mℓ) were added to a solution of the compound (764 mg) prepared in reference example 2 dissolved into THF (9 mℓ). The mixture was refluxed for 5.5 hrs. The solution was concentrated and toluene was added to the residue. The solution was concentrated to give the title compound having the following physical data.

TLC: Rf 0.2 ($CH_2C\ell_2$:$CH_3OH$ = 98:2);

IR : $\nu$ 3300, 2940, 2860, 1725, 1660, 1540, 1450, 1370, 1250, 1170, 1080, 1040, 730, 690 $cm^{-1}$

Reference example 3(a) - 3(z)

By the same procedure as reference example 3, using with each starting materials, following compounds having the physical data shown in the Table V were given.

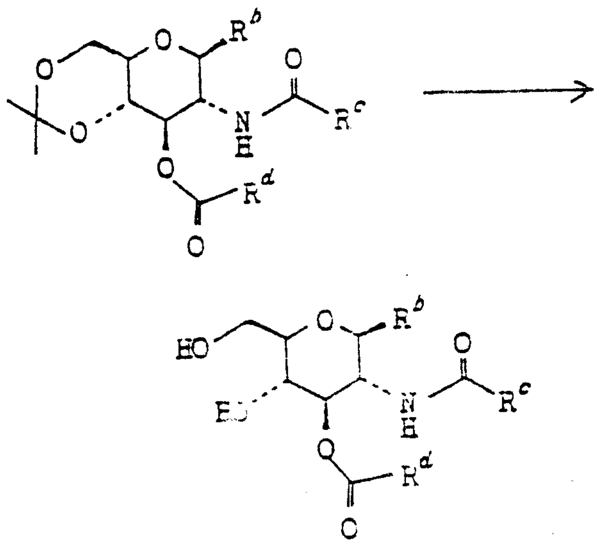

Table V

| No. | $R^b$, $R^c$, $R^d$ | Name | Starting Material | TLC | IR ($cm^{-1}$) |
|---|---|---|---|---|---|
| 3 (a) | 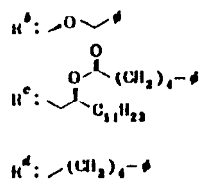 | benzyl 2-deoxy-2-[3S-(5-phenylpentanoyloxy)tetradecanoyl]amino-3-O-(5-phenylpentanoyl)-β-D-glucopyranoside | Reference Example 2 (a) | | |
| 3 (b) | 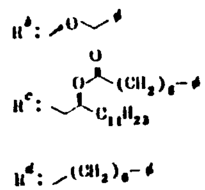 | benzyl 2-deoxy-2-[3S-(7-phenylheptanoyloxy)tetradecanoyl]amino-3-O-(7-phenylheptanoyl)-β-D-glucopyranoside | Reference Example 2 (b) | | |
| 3 (c) | 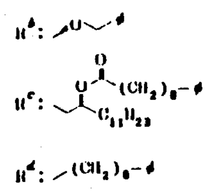 | benzyl 2-deoxy-2-[3S-(9-phenylnonanoyloxy)tetradecanoyl]amino-3-O-(9-phenylnonanoyl)-β-D-glucopyranoside | Reference Example 2 (c) | | |
| 3 (d) | 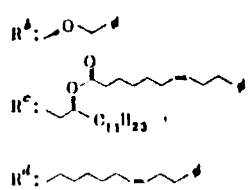 | benzyl 2-deoxy-2-[3S-(10-phenyl-7Z-decenoyloxy)tetradecanoyl]amino-3-O-(10-phenyl-7Z-decenoyl)-β-D-glucopyranoside | Reference Example 2 (d) | | |

| No. | $R^b$, $R^c$, $R^d$ | Name | Starting Material | TLC | IR ($cm^{-1}$) |
|---|---|---|---|---|---|
| 3 (e) | 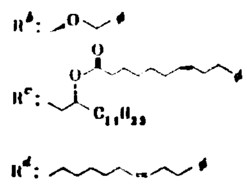 | benzyl 2-deoxy-2-[3R-(10-phenyl-7Z-decenoyloxy)tetradecanoyl]amino-3-O-(10-phenyl-7Z-decenoyl)-β-D-glucopyranoside | Reference Example 2 (e) | | |
| 3 (f) | 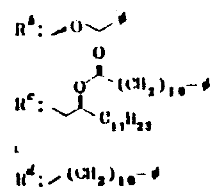 | benzyl 2-deoxy-2-[3S-(11-phenylundecanoyloxy)tetradecanoyl]amino-3-O-(11-phenylundecanoyl)-β-D-glucopyranoside | Reference Example 2 (f) | | |
| 3 (g) | 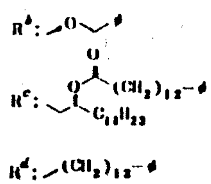 | benzyl 2-deoxy-2-[3S-(13-phenyltridecanoyloxy)tetradecanoyl]amino-3-O-(13-phenyltridecanoyl)-β-D-glucopyranoside | Reference Example 2 (g) | | |
| 3 (h) | 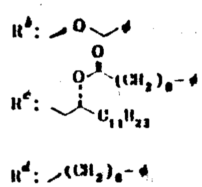 | benzyl 2-deoxy-2-[3R-(9-phenylnonanoyloxy)tetradecanoyl]amino-3-O-(9-phenylnonanoyl)-β-D-glucopyranoside | Reference Example 2 (h) | Rf 0.18 (EtOAc: n-$C_6H_{14}$ = 3:5) | ν 3450, 3270, 2920, 2850, 1730, 1650, 1550 |

| No. | $R^b$, $R^c$, $R^d$ | Name | Starting Material | TLC | IR ($cm^{-1}$) |
|---|---|---|---|---|---|
| 3 (i) | 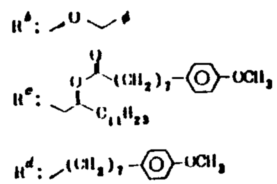 | benzyl 2-deoxy-2-[3S-[8-(4-methoxyphenyl)octanoyloxy]tetradecanoyl]amino-3-O-[8-(4-methoxyphenyl)octanoyl]-β-D-glucopyranoside | Reference Example 2 (i) | Rf 0.07 (EtOAc: n-$C_6H_{14}$ = 1:1) | |
| 3 (j) | 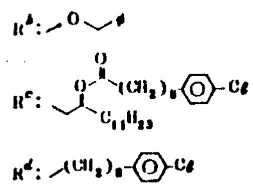 | benzyl 2-deoxy-2-[3S-[9-(4-chlorophenyl)nonanoyloxy]tetradecanoyl]amino-3-O-[9-(4-chlorophenyl)nonanoyl]-β-D-glucopyranoside | Reference Example 2 (j) | Rf 0.06 (EtOAc: n-$C_6H_{14}$ = 3:5) | |
| 3 (k) | 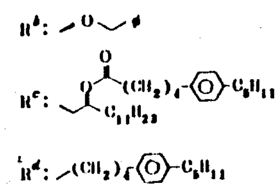 | benzyl 2-deoxy-2-[3S-[5-(4-pentylphenyl)pentanoyloxy]tetradecanoyl]amino-3-O-[5-(4-pentylphenyl)pentanoyl]-β-D-glucopyranoside | Reference Example 2 (k) | Rf 0.04 (EtOAc: n-$C_6H_{14}$ = 3:5) | |
| 3 (l) | 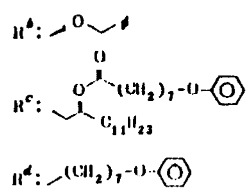 | benzyl 2-deoxy-2-[3S-(8-phenoxyoctanoyloxy)tetradecanoyl]amino-3-O-(8-phenoxyoctanoyl)-β-D-glucopyranoside | Reference Example 2 (l) | Rf 0.06 (EtOAc: n-$C_6H_{14}$ = 3:5) | |

| No. | $R^b$, $R^c$, $R^d$ | Name | Starting Material | TLC | IR ($cm^{-1}$) |
|---|---|---|---|---|---|
| 3 (m) | 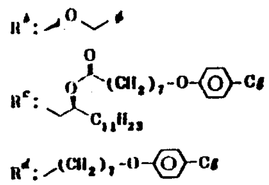 | benzyl 2-deoxy-2-[3S-[8-(4-chlorophenoxy)octanoyloxy]tetradecanoyl]amino-3-O-[8-(4-chlorophenoxy)octanoyl]-β-D-glucopyranoside | Reference Example 2 (m) | Rf 0.03 (EtOAc: n-$C_6H_{14}$ = 3:5) | |
| 3 (n) | 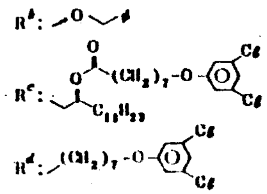 | benzyl 2-deoxy-2-[3S-[8-(3,5-dichlorophenoxy)octanoyloxy]tetradecanoyl]amino-3-O-[8-(3,5-dichlorophenoxy)octanoyl]-β-D-glucopyranoside | Reference Example 2 (n) | Rf 0.05 (EtOAc: n-$C_6H_{14}$ = 3:5) | |
| 3 (o) | 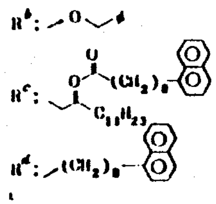 | benzyl 2-deoxy-2-[3S-[9-(1-naphthyl)nonanoyloxy]tetradecanoyl]amino-3-O-[9-(1-naphthyl)nonanoyl]-β-D-glucopyranoside | Reference Example 2 (o) | Rf 0.03 (EtOAc: n-$C_6H_{14}$ = 3:5) | |
| 3 (p) | 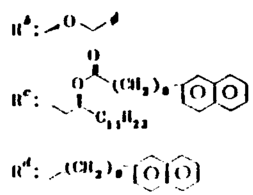 | benzyl 2-deoxy-2-[3S-[9-(2-naphthyl)nonanoyloxy]tetradecanoyl]amino-3-O-[9-(2-naphthyl)nonanoyl]-β-D-glucopyranoside | Reference Example 2 (p) | Rf 0.06 (EtOAc: n-$C_6H_{14}$ = 3:5) | |

| No. | $R^b$, $R^c$, $R^d$ | Name | Starting Material | TLC | IR ($cm^{-1}$) |
|---|---|---|---|---|---|
| 3 (q) | 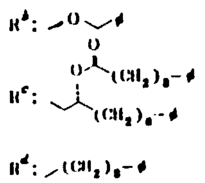 | benzyl 2-deoxy-2-[3R-(9-phenylnonanoyloxy)-9-phenylnonanoyl]amino-3-O-(9-phenylnonanoyl)-β-D-glucopyranoside | Reference Example 2 (q) | Rf 0.13 (EtOAc: n-$C_6H_{14}$ = 1:2) | |
| 3 (r) | 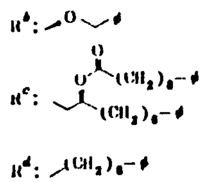 | benzyl 2-deoxy-2-[3S-(9-phenylnonanoyloxy)-9-phenylnonanoyl]amino-3-O-(9-phenylnonanoyl)-β-D-glucopyranoside | Reference Example 2 (r) | Rf 0.15 (EtOAc: n-$C_6H_{14}$ = 1:2) | |
| 3 (s) | 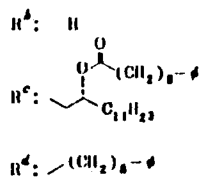 | 2-deoxy-2-[3R-(9-phenylnonanoyloxy)tetradecanoyl]amino-3-O-(9-phenylnonanoyl)-1,5-anhydro-D-glucitol | Reference Example 2 (s) | Rf 0.38 ($CH_2C\ell_2$: $CH_3OH$ = 9:1) | |
| 3 (t) | 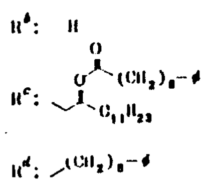 | 2-deoxy-2-[3S-(9-phenylnonanoyloxy)tetradecanoyl]amino-3-O-(9-phenylnonanoyl)-1,5-anhydro-D-glucitol | Reference Example 2 (t) | Rf 0.38 ($CH_2C\ell_2$: $CH_3OH$ = 9:1) | |

| No. | $R^b$, $R^c$, $R^d$ | Name | Starting Material | TLC | IR ($cm^{-1}$) |
|---|---|---|---|---|---|
| 3 (u) | 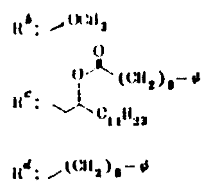 | methyl 2-deoxy-2-[3R-(9-phenylnonanoyloxy) tetradecanoyl]amino-3-O-(9-phenylnonanoyl)-β-D-glucopyranoside | Reference Example 2 (u) | Rf 0.55 ($CH_2Cl_2$: $CH_3OH$ = 9:1) | |

| No. | $R^b$, $R^c$, $R^d$ | Name | Starting Material | TLC | IR ($cm^{-1}$) |
|---|---|---|---|---|---|
| 3 (v) | $R^b$ : -H<br>$R^c$ : 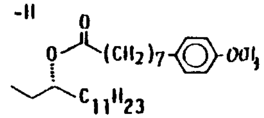<br>$R^d$ : 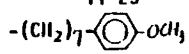 | 2-deoxy-2-[3R-[8-(4-methoxyphenyl)octanoyloxy] tetradecanoyl]amino-3-O-[8-(4-methoxyphenyl) octanoyl]-1,5-anhydro-D-glucitol | Reference Example 2 (v) | Rf 0.06 (EtOAc: n-$C_6H_{14}$ = 1:1) | |
| 3 (w) | $R^b$ : -H<br>$R^c$ : 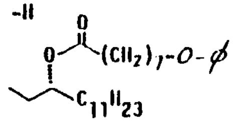<br>$R^d$ : $-(CH_2)_7-O-\phi$ | 2-deoxy-2-[3R-(8-phenoxyoctanoyloxy) tetradecanoyl]amino-3-O-(8-phenoxyoctanoyl)-1,5-anhydro-D-glucitol | Reference Example 2 (w) | Rf 0.07 (EtOAc: n-$C_6H_{14}$ = 1:1) | |
| 3 (x) | $R^b$ : -H<br>$R^c$ : 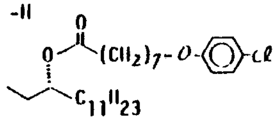<br>$R^d$ : 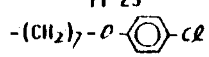 | 2-deoxy-2-[3R-[8-(4-chlorophenoxy)octanoyloxy] tetradecanoyl]amino-3-O-[8-(4-chlorophenoxy) octanoyl]-1,5-anhydro-D-glucitol | Reference Example 2 (x) | | |
| 3 (y) | $R^b$ : -H<br>$R^c$ : 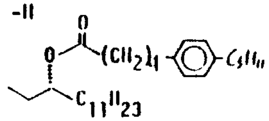<br>$R^d$ : 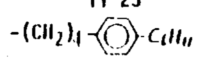 | 2-deoxy-2-[3R-[5-(4-pentylphenyl)pentanoyloxy] tetradecanoyl]amino-3-O-[5-(4-pentylphenyl) pentanoyl]-1,5-anhydro-D-glucitol | Reference Example 2 (y) | | |

| No. | $R^b$, $R^c$, $R^d$ | Name | Starting Material | TLC | IR ($cm^{-1}$) |
|---|---|---|---|---|---|
| 3 (z) | $R^b$ : -H<br>$R^c$ : 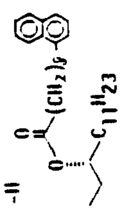<br>$R^d$ : $-(CH_2)_9-$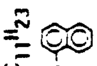 | 2-deoxy-2-[3R-[9-(1-naphthyl)nonanoyloxy] tetradecanoyl]amino-3-O-[9-(1-naphthyl)nonanoyl] -1,5-anhydro-D-glucitol | Reference Example 2 (z) | Rf 0.55 ($n$-$C_6H_{14}$: EtOAc = 3:1) | |

Reference example 4

Synthesis of benzyl 2-deoxy-2-[3R-(4-phenylbutyryloxy) tetradecanoyl]amino-3-0-(4-phenylbutyryl)-6-0-t-butyldimethylsilylglucopyranoside

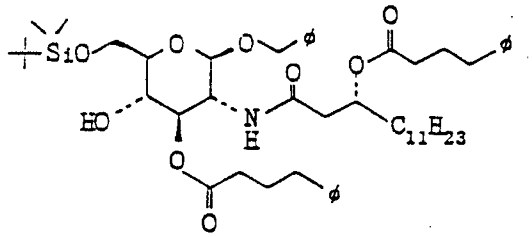

t-Butyldimethylsilyl chloride (338 mg) and 4-(N,N-dimethylamino)pyridine (11 mg) were added to a solution of the compound (691 mg) prepared in reference example 3 dissolved into dry pyridine (15 mℓ). The mixture was stirred for 7.5 hrs in an atmosphere of nitrogen at room temperature. The reaction solution was evaporated. The residue was purified by column chromatography on silica gel ($CH_2C\ell_2$:$CH_3OH$ = 98:2) to give the title compound (936 mg) having the following physical data.

TLC: Rf 0.85 ($CH_2C\ell_2$:$CH_3OH$ = 97:3);

IR : ν 3350, 2940, 2860, 1720, 1680, 1490, 1450, 1360, 1250, 1130, 1070, 830, 690 $cm^{-1}$

Reference example 4(a) - 4(z)

By the same procedure as reference example 4, using with each starting materials, following compounds having the physical data shown in the Table VI were given.

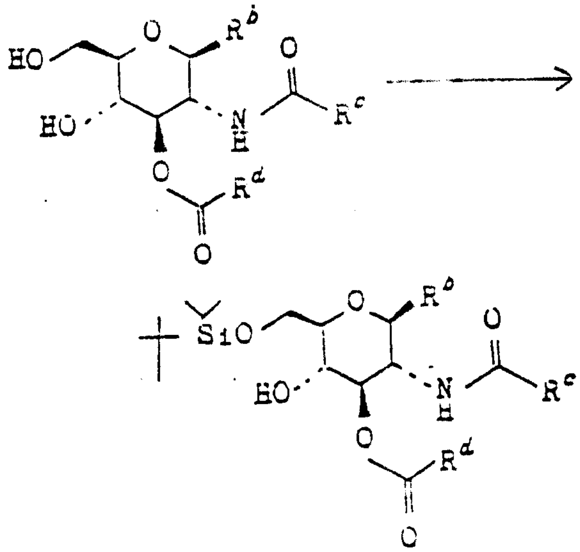

Table VI

| No. | $R^b$, $R^c$, $R^d$ | Name | Starting Material | TLC | IR ($cm^{-1}$) |
|---|---|---|---|---|---|
| 4 (a) | $R^b$: 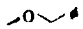<br>$R^c$: 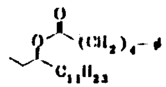<br>$R^d$: 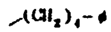 | benzyl 2-deoxy-2-[3S-(5-phenylpentanoyloxy) tetradecanoyl]amino-3-O-(5-phenylpentanoyl)-6-O-t-butyldimethylsilyl-β-D-glucopyranoside | Reference Example 3 (a) | Rf 0.75 ($CH_2Cl_2$: $CH_3OH$ = 97:3) | |
| 4 (b) | $R^b$: 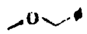<br>$R^c$: 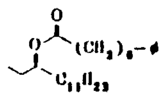<br>$R^d$: 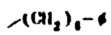 | benzyl 2-deoxy-2-[3S-(7-phenylheptanoyloxy) tetradecanoyl]amino-3-O-(7-phenylheptanoyl)-6-O-t-butyldimethylsilyl-β-D-glucopyranoside | Reference Example 3 (b) | Rf 0.80 ($CH_2Cl_2$: $CH_3OH$ = 97:3) | |
| 4 (c) | $R^b$: 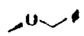<br>$R^c$: 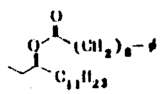<br>$R^d$: 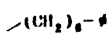 | benzyl 2-deoxy-2-[3S-(9-phenylnonanoyloxy) tetradecanoyl]amino-3-O-(9-phenylnonanoyl)-6-O-t-butyldimethylsilyl-β-D-glucopyranoside | Reference Example 3 (c) | Rf 0.85 (EtOAc n-$C_6H_{14}$ = 1:1) | |
| 4 (d) | $R^b$: 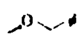<br>$R^c$: 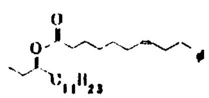<br>$R^d$: 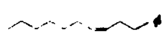 | benzyl 2-deoxy-2-[3S-(10-phenyl-7Z-decenoyloxy) tetradecanoyl]amino-3-O-(10-phenyl-7Z-decenoyl)-6-O-t-butyldimethylsilyl-β-D-glucopyranoside | Reference Example 3 (d) | Rf 0.94 ($CH_2Cl_2$: EtOAc = 97:3) | |

| No. | $R^b$, $R^c$, $R^d$ | Name | Starting Material | TLC | IR ($cm^{-1}$) |
|---|---|---|---|---|---|
| 4 (e) | 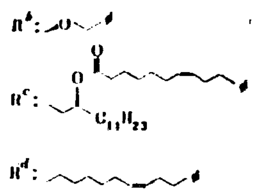 | benzyl 2-deoxy-2-[3R-(10-phenyl-7Z-decenoyloxy)tetradecanoyl]amino-3-O-(10-phenyl-7Z-decenoyl)-6-O-t-butyldimethylsilyl-β-D-glucopyranoside | Reference Example 3 (e) | | ν 3460, 2940, 2860, 1730, 1690, 1510, 1460, 1370, 1320, 1260, 1160, 1130, 1080, 840, 700 |
| 4 (f) | 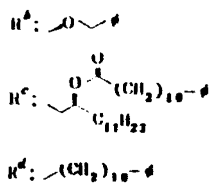 | benzyl 2-deoxy-2-[3S-(11-phenylundecanoyloxy)tetradecanoyl]amino-3-O-(11-phenylundecanoyl)-6-O-t-butyldimethylsilyl-β-D-glucopyranoside | Reference Example 3 (f) | | |
| 4 (g) | 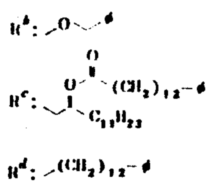 | benzyl 2-deoxy-2-[3S-(13-phenyltridecanoyloxy)tetradecanoyl]amino-3-O-(13-phenyltridecanoyl)-6-O-t-butyldimethylsilyl-β-D-glucopyranoside | Reference Example 3 (g) | Rf 0.84 ($CH_2Cl_2$: n-$C_6H_{14}$ = 1:1) | |
| 4 (h) | 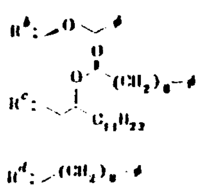 | benzyl 2-deoxy-2-[3R-(9-phenylnonanoyloxy)tetradecanoyl]amino-3-O-(9-phenylnonanoyl)-6-O-t-butyldimethylsilyl-β-D-glucopyranoside | Reference Example 3 (h) | | ν 3470, 3280, 2920, 2850, 1730, 1650, 1550 |

| No. | $R^b$, $R^c$, $R^d$ | Name | Starting Material | TLC | IR ($cm^{-1}$) |
|---|---|---|---|---|---|
| 4 (i) | $R^b$: 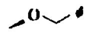<br>$R^c$: 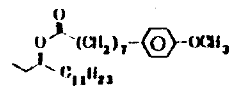<br>$R^d$: 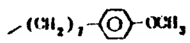 | benzyl 2-deoxy-2-[3S-[8-(4-methoxyphenyl) octanoyloxy]tetradecanoyl]amino-3-O-[8-(4-methoxyphenyl)octanoyl]-6-O-t-butyldimethylsilyl-β-D-glucopyranoside | Reference Example 3 (i) | Rf 0.74 (EtOAc: n-$C_6H_{14}$ = 3:5) | ν 3500, 3290, 2920, 2850, 1730, 1650, 1610 |
| 4 (j) | $R^b$: 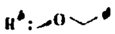<br>$R^c$: 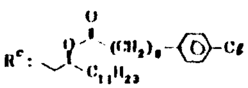<br>$R^d$: 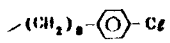 | benzyl 2-deoxy-2-[3S-[9-(4-chlorophenyl) nonanoyloxy]tetradecanoyl]amino-3-O-[9-(4-chlorophenyl)nonanoyl]-6-O-t-butyldimethylsilyl-β-D-glucopyranoside | Reference Example 3 (j) | Rf 0.70 (EtOAc: n-$C_6H_{14}$ = 3:5) | ν (liquid film) 3500, 3270, 2910, 2840, 1730, 1650, 1540 |
| 4 (k) | $R^b$: 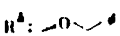<br>$R^c$: 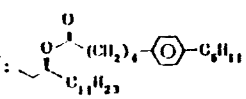<br>$R^d$: 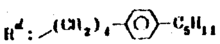 | benzyl 2-deoxy-2-[3S-[5-(4-pentylphenyl) pentanoyloxy]tetradecanoyl]amino-3-O-[5-(4-pentylphenyl)pentanoyl]-6-O-t-butyldimethylsilyl-β-D-glucopyranoside | Reference Example 3 (k) | Rf 0.81 (EtOAc: n-$C_6H_{14}$ = 3:5) | |
| 4 (l) | $R^b$: 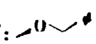<br>$R^c$: 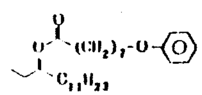<br>$R^d$: 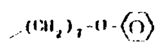 | benzyl 2-deoxy-2-[3S-(8-phenoxyoctanoyloxy) tetradecanoyl]amino-3-O-(8-phenoxyoctanoyl)-6-O-t-butyldimethylsilyl-β-D-glucopyranoside | Reference Example 3 (l) | Rf 0.72 (EtOAc: n-$C_6H_{14}$ = 3:5) | ν (liquid film) 3450, 3270, 2910, 2850, 1730, 1650, 1600 |

| No. | $R^b$, $R^c$, $R^d$ | Name | Starting Material | TLC | IR ($cm^{-1}$) |
|---|---|---|---|---|---|
| 4 (m) | 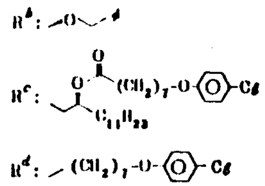 | benzyl 2-deoxy-2-[3S-[8-(4-chlorophenoxy)octanoyloxy]tetradecanoyl]amino-3-O-[8-(4-chlorophenoxy)octanoyl]-6-O-t-butyldimethylsilyl-β-D-glucopyranoside | Reference Example 3 (m) | Rf 0.75 (EtOAc: n-$C_6H_{14}$ = 3:5) | ν (liquid film) 3300, 2920, 2850, 1730, 1650, 1540, 1490, 1460 |
| 4 (n) | 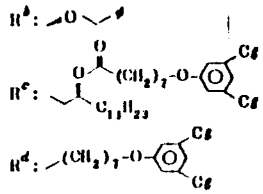 | benzyl 2-deoxy-2-[3S-[8-(3,5-dichlorophenoxy)octanoyloxy]tetradecanoyl]amino-3-O-[8-(3,5-dichlorophenoxy)octanoyl]-6-O-t-butyldimethylsilyl-β-D-glucopyranoside | Reference Example 3 (n) | Rf 0.59 (EtOAc: n-$C_6H_{14}$ = 3:5) | ν (liquid film) 3300, 2920, 2860, 1730, 1650, 1590, 1570 |
| 4 (o) | 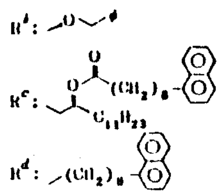 | benzyl 2-deoxy-2-[3S-[9-(1-naphthyl)nonanoyloxy]tetradecanoyl]amino-3-O-[9-(1-naphthyl)nonanoyl]-6-O-t-butyldimethylsilyl-β-D-glucopyranoside | Reference Example 3 (o) | Rf 0.62 (EtOAc: n-$C_6H_{14}$ = 3:5) | ν (liquid film) 3450, 3280, 2910, 2850, 1730, 1660 |
| 4 (p) | 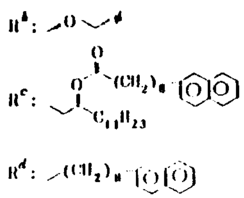 | benzyl 2-deoxy-2-[3S-[9-(2-naphthyl)nonanoyloxy]tetradecanoyl]amino-3-O-[9-(2-naphthyl)nonanoyl]-6-O-t-butyldimethylsilyl-β-D-glucopyranoside | Reference Example 3 (p) | | ν (liquid film) 3300, 2910, 2820, 1730, 1650, 1540 |

| No. | $R^b$, $R^c$, $R^d$ | Name | Starting Material | TLC | IR ($cm^{-1}$) |
|---|---|---|---|---|---|
| 4 (q) | 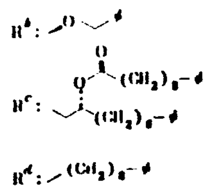 | benzyl 2-deoxy-2-[3R-(9-phenylnonanoyloxy)-9-phenylnonanoyl]amino-3-O-(9-phenylnonanoyl)-6-O-t-butyldimethylsilyl-β-D-glucopyranoside | Reference Example 3 (q) | Rf 0.68 (EtOAc: n-$C_6H_{14}$ = 1:2) | ν (liquid film) 3500, 3280, 2920, 2850, 1730, 1650, 1640 |
| 4 (r) | 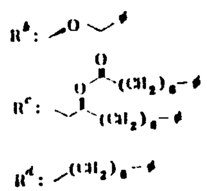 | benzyl 2-deoxy-2-[3S-(9-phenylnonanoyloxy)-9-phenylnonanoyl]amino-3-O-(9-phenylnonanoyl)-6-O-t-butyldimethylsilyl-β-D-glucopyranoside | Reference Example 3 (r) | Rf 0.75 (EtOAc: n-$C_6H_{14}$ = 1:2) | ν (liquid film) 3500, 3400, 2910, 1720, 1650, 1600 |
| 4 (s) | 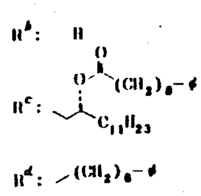 | 2-deoxy-2-[3R-(9-phenylnonanoyloxy)tetradecanoyl]amino-3-O-(9-phenylnonanoyl)-6-O-t-butyldimethylsilyl-1,5-anhydro-D-glucitol | Reference Example 3 (s) | Rf 0.17 (n-$C_6H_{14}$: EtOAc = 3:1) | |
| 4 (t) | 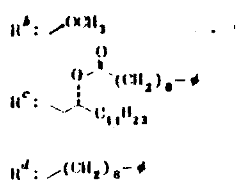 | methyl 2-deoxy-2-[3R-(9-phenylnonanoyloxy)tetradecanoyl]amino-3-O-(9-phenylnonanoyl)-6-O-t-butyldimethylsilyl-β-D-glucopyranoside | Reference Example 3 (u) | Rf 0.41 (n-$C_6H_{14}$: EtOAc = 2:1) | |

| No. | $R^b$, $R^c$, $R^d$ | Name | Starting Material | TLC | IR ($cm^{-1}$) |
|---|---|---|---|---|---|
| 4 (u) | $R^b$ : -H<br>$R^c$ : 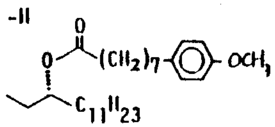<br>$R^d$ : 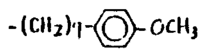 | 2-deoxy-2-[3R-[8-(4-methoxyphenyl)octanoyloxy]tetradecanoyl]amino-3-O-[8-(4-methoxyphenyl)octanoyl]-6-O-t-butyldimethylsilyl-1,5-anhydro-D-glucitol | Reference Example 3 (v) | Rf 0.19 ($n-C_6H_{14}$: EtOAc = 3:1) | ν 3500, 1730, 1650, 1610 |
| 4 (v) | $R^b$ : -H<br>$R^c$ : 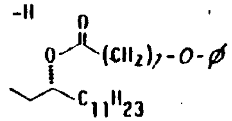<br>$R^d$ : $-(CH_2)_7-O-\phi$ | 2-deoxy-2-[3R-(8-phenoxyoctanoyloxy)tetradecanoyl]amino-3-O-(8-phenoxyoctanoyl)-6-O-t-butyldimethylsilyl-1,5-anhydro-D-glucitol | Reference Example 3 (w) | Rf 0.20 ($n-C_6H_{14}$: EtOAc = 3:1) | ν 3430, 1728, 1650, 1600 |
| 4 (w) | $R^b$ : -H<br>$R^c$ : 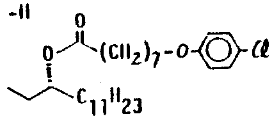<br>$R^d$ : 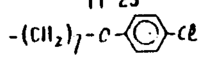 | 2-deoxy-2-[3R-[8-(4-chlorophenoxy)octanoyloxy]tetradecanoyl]amino-3-O-[8-(4-chlorophenoxy)octanoyl] 6-O-t-butyldimethylsilyl-1,5-anhydro-D-glucitol | Reference Example 3 (x) | Rf 0.21 ($n-C_6H_{14}$: EtOAc = 3:1) | ν ($CHCl_3$ solution) 3300, 1730, 1650, 1540 |
| 4 (x) | $R^b$ : -H<br>$R^c$ : 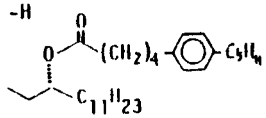<br>$R^d$ : 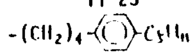 | 2-deoxy-2-[3R-[5-(4-pentylphenyl)pentanoyloxy]tetradecanoyl]amino-3-O-[5-(4-pentylphenyl)pentanoyl]-6-O-t-butyldimethylsilyl-1,5-anhydro-D-glucitol | Reference Example 3 (y) | Rf 0.22 ($n-C_6H_{14}$: EtOAc = 3:1) | ν ($CHCl_3$ solution) 3430, 1730, 1652, 1550 |

| No. | $R^b$, $R^c$, $R^d$ | Name | Starting Material | TLC | IR ($cm^{-1}$) |
|---|---|---|---|---|---|
| 4 (z) | $R^b$ : -H<br>$R^c$ :<br>$R^d$ : 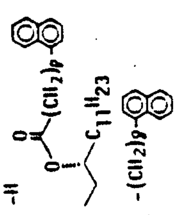 | 2-deoxy-2-[3R-[9-(1-naphthyl)nonanoyloxy]tetradecanoyl]amino-3-O-[9-(1-naphthyl)nonanoyl]-6-O-t-butyldimethylsilyl-1,5-anhydro-D-glucitol | Reference Example 3 (z) | Rf 0.19 ($n-C_6H_{14}$: EtOAc = 3:1) | ν ($CHCl_3$ solution) 3432, 1732, 1652, 1543, 840, 785 |

Reference example 5

Synthesis of 2-deoxy-2-[3R-(4-phenylbutyryloxy)tetradecanoyl] amino-3-0-(4-phenylbutyryl)-6-0-t-butyldimethylsilyl-D-glucopyranose

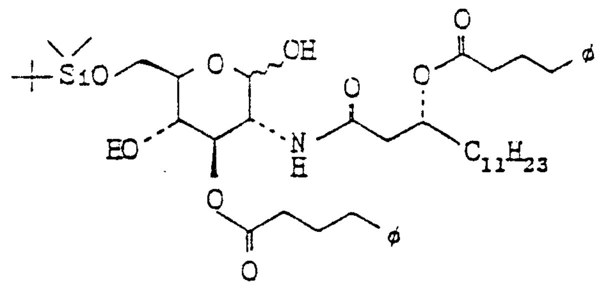

To a stirring solution of the compound (736 mg) prepared in reference example 4 dissolved into THF, palladium-carbon (content 10%; 368 mg) was added with small portions. In an atmosphere of hydrogen, the mixture was stirred vigorously for 24 hrs at 50 ° C. After reaction, the reaction mixture was filtered. And the filtrate was evaporated to give the title compound (653 mg) having the following physical data.

TLC: Rf 0.64 ($CH_2C\ell_2$:$CH_3OH$ = 9:1);

IR : $\nu$ 3350, 2920, 2840, 1720, 1640, 1540, 1440, 1250, 1130, 1070, 1050, 800, 740, 690 $cm^{-1}$

Reference example 5(a) - 5(p)

By the same procedure as reference example 5, using with each starting materials, following compounds having the physical data shown in the Table VII were given.

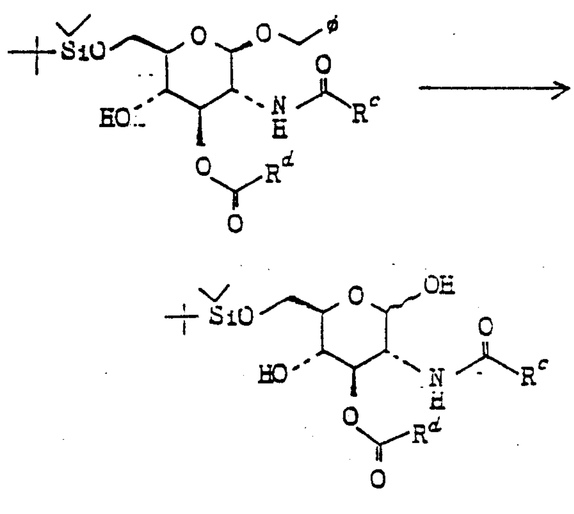

Table VII

| No. | $R^c$, $R^d$ | Name | Starting Material | TLC | IR ($cm^{-1}$) |
|---|---|---|---|---|---|
| 5 (a) | 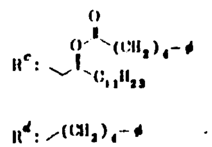 | 2-deoxy-2-[3S-(5-phenylpentanoyloxy)tetradecanoyl]amino-3-O-(5-phenylpentanoyl)-6-O-t-butyldimethylsilyl-D-glucopyranose | Reference Example 4 (a) | Rf 0.36 ($CH_2Cl_2$: $CH_3OH$ = 97:3) | |
| 5 (b) | 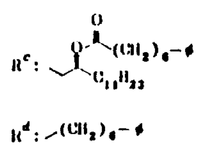 | 2-deoxy-2-[3S-(7-phenylheptanoyloxy)tetradecanoyl]amino-3-O-(7-phenylheptanoyl)-6-O-t-butyldimethylsilyl-D-glucopyranose | Reference Example 4 (b) | Rf 0.34 ($CH_2Cl_2$: $CH_3OH$ = 97:3) | |
| 5 (c) | 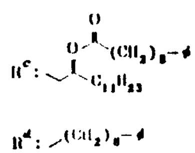 | 2-deoxy-2-[3S-(9-phenylnonanoyloxy)tetradecanoyl]amino-3-O-(9-phenylnonanoyl)-6-O-t-butyldimethylsilyl-D-glucopyranose | Reference Example 4 (c) | Rf 0.46 ($CH_2Cl_2$: EtOAc = 9:1) | |
| 5 (d) | 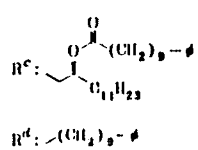 | 2-deoxy-2-[3S-(10-phenyldecanoyloxy)tetradecanoyl]amino-3-O-(10-phenyldecanoyl)-6-O-t-butyldimethylsilyl-D-glucopyranose | Reference Example 4 (d) | Rf 0.42 ($CH_2Cl_2$: $CH_3OH$ = 97:3) | |

| No. | $R^c$, $R^d$ | Name | Starting Material | TLC | IR ($cm^{-1}$) |
|---|---|---|---|---|---|
| 5 (e) | 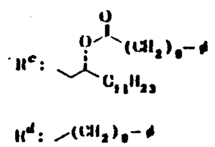 | 2-deoxy-2-[3R-(10-phenyldecanoyloxy)tetradecanoyl]amino-3-O-(10-phenyldecanoyl)-6-O-t-butyldimethylsilyl-D-glucopyranose | Reference Example 4 (e) | | ν 3460, 2930, 2860, 1720, 1670, 1500, 1450, 1260, 1090, 840 |
| 5 (f) | 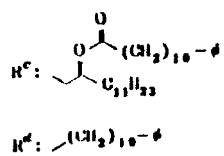 | 2-deoxy-2-[3S-(11-phenylundecanoyloxy)tetradecanoyl]amino-3-O-(11-phenylundecanoyl)-6-O-t-butyldimethylsilyl-D-glucopyranose | Reference Example 4 (f) | Rf 0.36 ($CH_2Cl_2$: EtOAc = 9:1) | |
| 5 (g) | 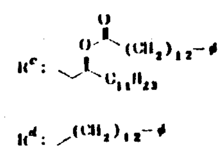 | 2-deoxy-2-[3S-(13-phenyltridecanoyloxy)tetradecanoyl]amino-3-O-(13-phenyltridecanoyl)-6-O-t-butyldimethylsilyl-D-glucopyranose | Reference Example 4 (g) | Rf 0.43 ($CH_2Cl_2$: $CH_3OH$ = 9:1) | |
| 5 (h) | 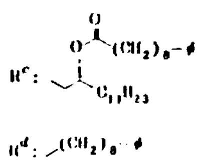 | 2-deoxy-2-[3R-(9-phenylnonanoyloxy)tetradecanoyl]amino-3-O-(9-phenylnonanoyl)-6-O-t-butyldimethylsilyl-D-glucopyranose | Reference Example 4 (h) | Rf 0.56 (EtOAc: n-$C_6H_{14}$ = 3:5) | |

| No. | $R^c$, $R^d$ | Name | Starting Material | TLC | IR ($cm^{-1}$) |
|---|---|---|---|---|---|
| 5 (i) | $R^c$: 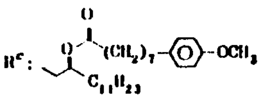 $R^d$: 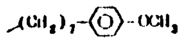 | 2-deoxy-2-[3S-[8-(4-methoxyphenyl)octanoyloxy]tetradecanoyl]amino-3-O-[8-(4-methoxyphenyl)octanoyl]-6-O-t-butyldimethylsilyl-D-glucopyranose | Reference Example 4 (i) | Rf 0.53 (EtOAc: n-$C_6H_{14}$ = 3:5) | |
| 5 (j) | $R^c$: 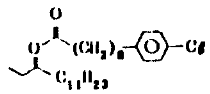 $R^d$: 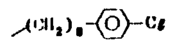 | 2-deoxy-2-[3S-[9-(4-chlorophenyl)nonanoyloxy]tetradecanoyl]amino-3-O-[9-(4-chlorophenyl)nonanoyl]-6-O-t-butyldimethylsilyl-D-glucopyranose | Reference Example 4 (j) | | |
| 5 (k) | $R^c$: 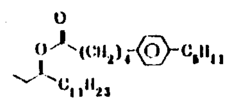 $R^d$: 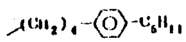 | 2-deoxy-2-[3S-[5-(4-pentylphenyl)pentanoyloxy]tetradecanoyl]amino-3-O-[5-(4-pentylphenyl)pentanoyl]-6-O-t-butyldimethylsilyl-β-D-glucopyranose | Reference Example 4 (k) | Rf 0.67 ($CH_2Cl_2$: $CH_3OH$ = 6:1) | |
| 5 (l) | $R^c$: 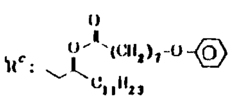 $R^d$: 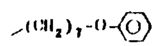 | 2-deoxy-2-[3S-(8-phenoxyoctanoyloxy)tetradecanoyl]amino-3-O-(8-phenoxyoctanoyl)-6-O-t-butyldimethylsilyl-D-glucopyranose | Reference Example 4 (l) | Rf 0.53 (EtOAc: n-$C_6H_{14}$ = 3:5) | |

| No. | $R^c$, $R^d$ | Name | Starting Material | TLC | IR ($cm^{-1}$) |
|---|---|---|---|---|---|
| 5 (m) | 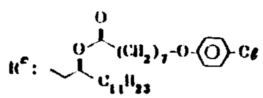 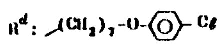 | 2-deoxy-2-[3S-[8-(4-chlorophenoxy)octanoyloxy]tetradecanoyl]amino-3-O-[8-(4-chlorophenoxy)octanoyl]-6-O-t-butyldimethylsilyl-D-glucopyranose | Reference Example 4 (m) | | |
| 5 (n) | 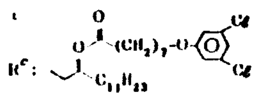 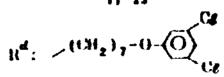 | 2-deoxy-2-[3S-[8-(3,5-dichlorophenoxy)octanoyloxy]tetradecanoyl]amino-3-O-[8-(3,5-dichlorophenoxy)octanoyl]-6-O-t-butyldimethylsilyl-D-glucopyranose | Reference Example 4 (n) | | |
| 5 (o) | 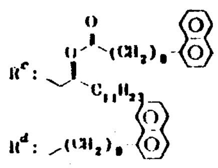 | 2-deoxy-2-[3S-[9-(1-naphthyl)nonanoyloxy]tetradecanoyl]amino-3-O-[9-(1-naphthyl)nonanoyl]-6-O-t-butyldimethylsilyl-D-glucopyranose | Reference Example 4 (o) | Rf 0.50 (EtOAc: n-$C_6H_{14}$ = 3:5) | |
| 5 (p) | 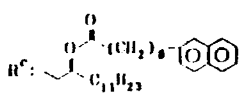 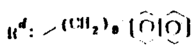 | 2-deoxy-2-[3S-[9-(2-naphthyl)nonanoyloxy]tetradecanoyl]amino-3-O-[9-(2-naphthyl)nonanoyl]-6-O-t-butyldimethylsilyl-D-glucopyranose | Reference Example 4 (p) | | |

| No. | $R^c$, $R^d$ | Name | Starting Material | TLC | IR ($cm^{-1}$) |
|---|---|---|---|---|---|
| 5 (q) | 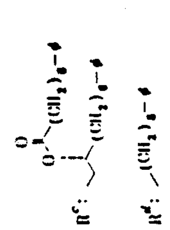 | 2-deoxy-2-[3R-(9-phenylnonanoyloxy)-9-phenylnonanoyl]amino-3-0-(9-phenylnonanoyl)-6-0-t-butyldimethylsilyl-D-gluxopyranose | Reference Example 4 (q) | Rf 0.57 (EtOAc: n-$C_6H_{14}$ = 1:2) | ν (liquid film) 3450, 3350, 2910, 2850, 1720, 1650, 1600 |
| 5 (r) | 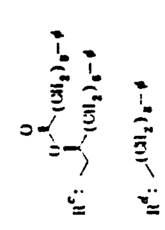 | 2-deoxy-2-[3S-(9-phenylnonanoyloxy)-9-phenylnonanoyl]amino-3-0-(9-phenylnonanoyl)-6-0-t-butyldimethylsilyl-D-glucopyranose | Reference Example 4 (r) | Rf 0.40 (EtOAc: n-$C_6H_{14}$ = 1:2) | |

Reference example 6

Synthesis of 2-deoxy-2-[3R-(4-phenylbutyryloxy)tetradecanoyl] amino-3-0-(4-phenylbutyryl)-4-0-sulfo-6-0-t-butyldimethylsilyl-D-glucopyranose

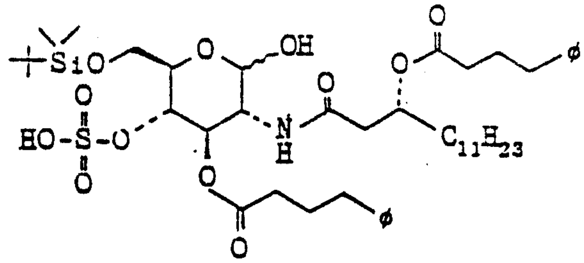

In an atmosphere of nitrogen, sulfur trioxide-pyridine complex (230 mg) was added to a solution of the compound (653 mg) prepared in reference example 5 dissolved into dry pyridine. The mixture was stirred for 4 hrs at room temperature, and concentrated. To the residue, toluene was added and the solution was evaporated to give the title compounds.

Reference example 6(a) - 6(p)

By the same procedure as reference example 6, using with each starting materials, following compounds having the physical data shown in the Table VIII were given.

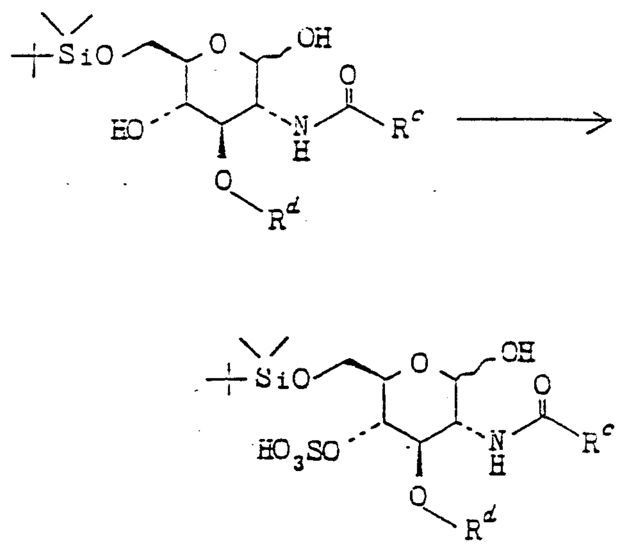

Table VIII

| No. | $R^c$, $R^d$ | Name | Starting Material | TLC |
|---|---|---|---|---|
| 6 (a) | $R^c$: 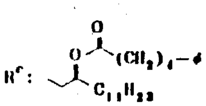 $R^d$: 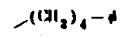 | 2-deoxy-2-[3S-(5-phenylpentanoyloxy) tetradecanoyl]amino-3-O-(5-phenylpentanoyl)-4-O-sulfo-6-O-t-butyldimethylsilyl-D-glucopyranose | Reference Example 5 (a) | |
| 6 (b) | $R^c$: 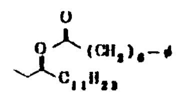 $R^d$: 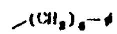 | 2-deoxy-2-[3S-(7-phenylheptanoyloxy) tetradecanoyl]amino-3-O-(7-phenylheptanoyl)-4-O-sulfo-6-O-t-butyldimethylsilyl-D-glucopyranose | Reference Example 5 (b) | |
| 6 (c) | $R^c$: 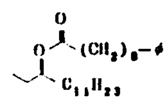 $R^d$: 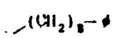 | 2-deoxy-2-[3S-(9-phenylnonanoyloxy) tetradecanoyl]amino-3-O-(9-phenylnonanoyl)-4-O-sulfo-6-O-t-butyldimethylsilyl-D-glucopyranose | Reference Example 5 (c) | |
| 6 (d) | $R^c$: 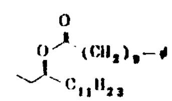 $R^d$: 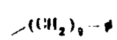 | 2-deoxy-2-[3S-(10-phenyldecanoyloxy) tetradecanoyl]amino-3-O-(10-phenyldecanoyl)-4-O-sulfo-6-O-t-butyldimethylsilyl-D-glucopyranose | Reference Example 5 (d) | |

| No. | $R^c$, $R^d$ | Name | Starting Material | TLC |
|---|---|---|---|---|
| 6 (e) | R^c : 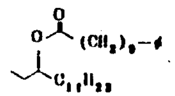<br>R^d : 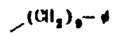 | 2-deoxy-2-[3R-(10-phenyldecanoyloxy)tetradecanoyl]amino-3-O-(10-phenyldecanoyl)-4-O-sulfo-6-O-t-butyldimethylsilyl-D-glucopyranose | Reference Example 5 (e) | |
| 6 (f) | R^c : 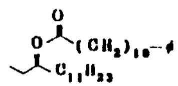<br>R^d : 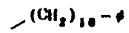 | 2-deoxy-2-[3S-(11-phenylundecanoyloxy)tetradecanoyl]amino-3-O-(11-phenylundecanoyl)-4-O-sulfo-6-O-t-butyldimethylsilyl-D-glucopyranose | Reference Example 5 (f) | |
| 6 (g) | R^c : 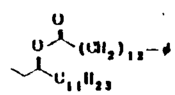<br>R^d : 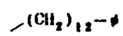 | 2-deoxy-2-[3S-(13-phenyltridecanoyloxy)tetradecanoyl]amino-3-O-(13-phenyltridecanoyl)-4-O-sulfo-6-O-t-butyldimethylsilyl-D-glucopyranose | Reference Example 5 (g) | |
| 6 (h) | R^c : 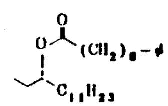<br>R^d : 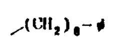 | 2-deoxy-2-[3R-(9-phenylnonanoyloxy)tetradecanoyl]amino-3-O-(9-phenylnonanoyl)-4-O-sulfo-6-O-t-butyldimethylsilyl-D-glucopyranose | Reference Example 5 (h) | |

| No. | $R^c$, $R^d$ | Name | Starting Material | TLC |
|---|---|---|---|---|
| 6 (i) | $R^c$ : 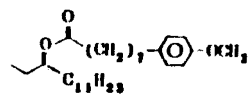 $R^d$ : 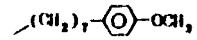 | 2-deoxy-2-[3S-[8-(4-methoxyphenyl)octanoyloxy]tetradecanoyl]amino-3-O-[8-(4-methoxyphenyl)octanoyl]-4-O-sulfo-6-O-t-butyldimethylsilyl-D-glucopyranose | Reference Example 5 (i) | Rf 0.41 (EtOAc: n-$C_6H_{14}$ = 1:6) |
| 6 (j) | $R^c$ : 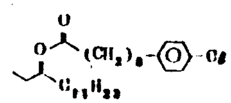 $R^d$ : 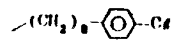 | 2-deoxy-2-[3S-[9-(4-chlorophenyl)nonanoyloxy]tetradecanoyl]amino-3-O-[9-(4-chlorophenyl)nonanoyl]-4-O-sulfo-6-O-t-butyldimethylsilyl-D-glucopyranose | Reference Example 5 (j) | |
| 6 (k) | $R^c$ : 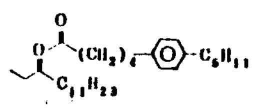 $R^d$ : 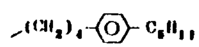 | 2-deoxy-2-[3S-[5-(4-pentylphenyl)pentanoyloxy]tetradecanoyl]amino-3-O-[5-(4-pentylphenyl)pentanoyl]-4-O-sulfo-6-O-t-butyldimethylsilyl-D-glucopyranose | Reference Example 5 (k) | Rf 0.30 ($CH_2Cl_2$: $CH_3OH$ = 6:1) |
| 6 (l) | $R^c$ : 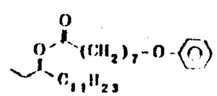 $R^d$ : 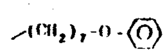 | 2-deoxy-2-[3S-(8-phenoxyoctanoyloxy)tetradecanoyl]amino-3-O-(8-phenoxyoctanoyl)-4-O-sulfo-6-O-t-butyldimethylsilyl-D-glucopyranose | Reference Example 5 (l) | Rf 0.61 ($CH_2Cl_2$: $CH_3OH$ = 6:1) |

| No. | $R^c$, $R^d$ | Name | Starting Material | TLC |
|---|---|---|---|---|
| 6 (m) | $R^c$: 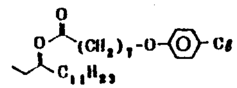 $R^d$: 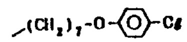 | 2-deoxy-2-[3S-[8-(4-chlorophenoxy)octanoyloxy]tetradecanoyl]amino-3-O-[8-(4-chlorophenoxy)octanoyl]-4-O-sulfo-6-O-t-butyldimethylsilyl-D-glucopyranose | Reference Example 5 (m) | |
| 6 (n) | $R^c$: $R^d$: 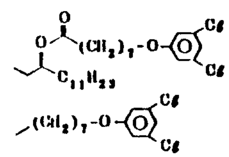 | 2-deoxy-2-[3S-[8-(3,5-dichlorophenoxy)octanoyloxy]tetradecanoyl]amino-3-O-[8-(3,5-dichlorophenoxy)octanoyl]-4-O-sulfo-6-O-t-butyldimethylsilyl-D-glucopyranose | Reference Example 5 (n) | |
| 6 (o) | $R^c$: 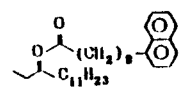 $R^d$: 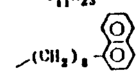 | 2-deoxy-2-[3S-[9-(1-naphthyl)nonanoyloxy]tetradecanoyl]amino-3-O-[9-(1-naphthyl)nonanoyl]-4-O-sulfo-6-O-t-butyldimethylsilyl-D-glucopyranose | Reference Example 5 (o) | Rf 0.69 ($CH_2Cl_2$: $CH_3OH$ = 9:2) |
| 6 (p) | $R^c$: 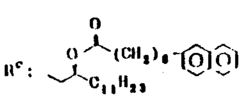 $R^d$: 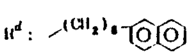 | 2-deoxy-2-[3S-[9-(2-naphthyl)nonanoyloxy]tetradecanoyl]amino-3-O-[9-(2-naphthyl)nonanoyl]-4-O-sulfo-6-O-t-butyldimethylsilyl-D-glucopyranose | Reference Example 5 (p) | |

| No. | $R^c$, $R^d$ | Name | Starting Material | TLC |
|---|---|---|---|---|
| 6 (q) | $R^c$: 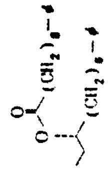 $R^d$: $-(CH_2)_8-\phi$ | 2-deoxy-2-[3R-(9-phenylnonanoyloxy)-9-phenylnonanoyl]amino-3-O-(9-phenylnonanoyl)-4-O-sulfo-6-O-t-butyldimethylsilyl-D-glucopyranose | Reference Example 5 (q) | |
| 6 (r) | $R^c$: 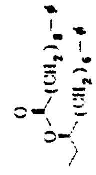 $R^d$: $-(CH_2)_8-\phi$ | 2-deoxy-2-[3S-(9-phenylnonanoyloxy)-9-phenylnonanoyl]amino-3-O-(9-phenylnonanoyl)-4-O-sulfo-6-O-t-butyldimethylsilyl-D-glucopyranose | Reference Example 5 (r) | Rf 0.43 ($CH_2Cl_2$ : $CH_3OH$ = 9:1) |

Reference example 6(s) - 6(y)

By the same procedure as reference example 6, using with each starting materials, following compounds having the physical data shown in the Table IX were given.

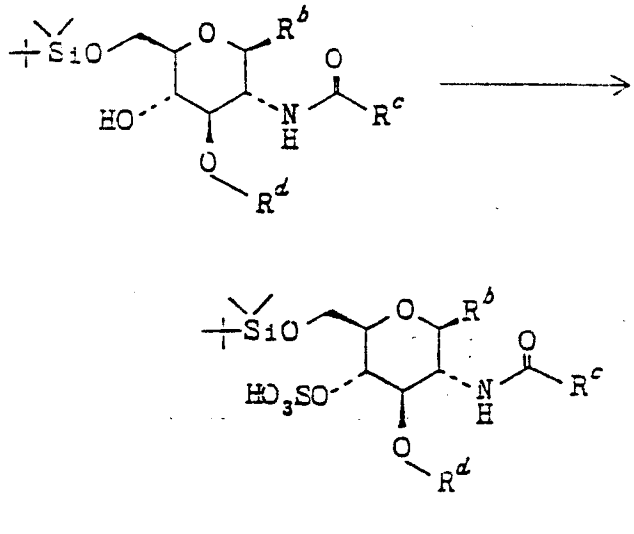

Table IX

| No. | $R^b$, $R^c$, $R^d$ | Name | Starting Material | TLC |
|---|---|---|---|---|
| 6 (s) | $R^b$: H<br>$R^c$: 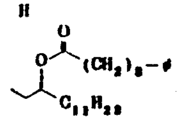<br>$R^d$: 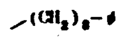 | 2-deoxy-2-[3R-(9-phenylnonanoyloxy)tetradecanoyl]amino-3-O-(9-phenylnonanoyl)-4-O-sulfo-6-O-t-butyldimethylsilyl-D-glucitol | Reference Example 4 (s) | Rf 0.22 ($CH_2Cl_2$: $CH_3OH$ = 9:1) |
| 6 (t) | $R^b$: 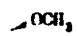<br>$R^c$: 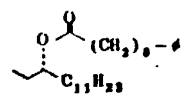<br>$R^d$: 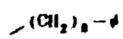 | methyl 2-deoxy-2-[3R-(9-phenylnonanoyloxy)tetradecanoyl]amino-3-O-(9-phenylnonanoyl)-4-O-sulfo-6-O-t-butyldimethylsilyl-β-D-glucopyranoside | Reference Example 4 (t) | Rf 0.20 ($CH_2Cl_2$: $CH_3OH$ = 9:1) |

| No. | $R^b$, $R^c$, $R^d$ | Name | Starting Material | TLC |
|---|---|---|---|---|
| 6 (u) | $R^b$ : -H<br>$R^c$ :<br>$R^d$ :<br>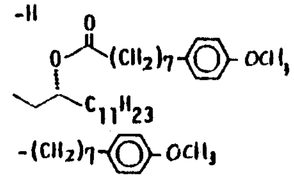 | 2-deoxy-2-[3R-[8-(4-methoxyphenyl)octanoyloxy]tetradecanoyl]amino-3-O-[8-(4-methoxyphenyl)octanoyl]-4-O-sulfo-6-O-t-butyldimethylsilyl-1,5-anhydro-D-glucitol | Reference Example 4 (u) | Rf 0.21 ($CH_2Cl_2$ : $CH_3OH$ = 9:1) |
| 6 (v) | $R^b$ : -H<br>$R^c$ :<br>$R^d$ :<br>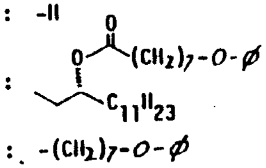 | 2-deoxy-2-[3R-(8-phenoxyoctanoyloxy)tetradecanoyl]amino-3-O-(8-phenoxyoctanoyl)-4-O-sulfo-6-O-t-butyldimethylsilyl-1,5-anhydro-D-glucitol | Reference Example 4 (v) | Rf 0.20 ($CH_2Cl_2$ : $CH_3OH$ = 9:1) |
| 6 (w) | $R^b$ : -H<br>$R^c$ :<br>$R^d$ :<br>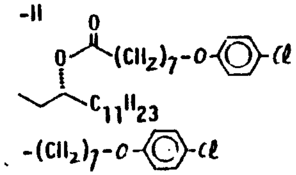 | 2-deoxy-2-[3R-[8-(4-chlorophenoxy)octanoyloxy]tetradecanoyl]amino-3-O-[8-(4-chlorophenoxy)octanoyl]-4-O-sulfo-6-O-t-butyldimethylsilyl-1,5-anhydro-D-glucitol | Reference Example 4 (w) | Rf 0.21 ($CH_2Cl_2$ : $CH_3OH$ = 9:1) |
| 6 (x) | $R^b$ : -H<br>$R^c$ :<br>$R^d$ :<br>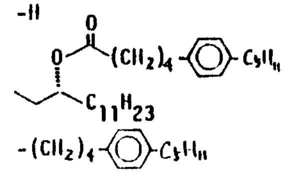 | 2-deoxy-2-[3R-[5-(4-pentylphenyl)pentanoyloxy]tetradecanoyl]amino-3-O-[5-(4-pentylphenyl)pentanoyl]-4-O-sulfo-6-O-t-butyldimethylsilyl-1,5-anhydro-D-glucitol | Reference Example 4 (x) | Rf 0.21 ($CH_2Cl_2$ : $CH_3OH$ = 9:1) |

| No. | $R^b$, $R^c$, $R^d$ | Name | Starting Material | TLC |
|---|---|---|---|---|
| 6 (y) | $R^b$ : -H<br>$R^c$ :<br>$R^d$ :<br>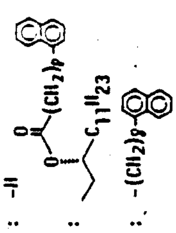 | 2-deoxy-2-[3R-[9-(1-naphthyl)nonanoyloxy]tetradecanoyl]amino-3-0-[9-(1-naphthyl)nonanoyl]-4-0-sulfo-6-0-t-butyldimethylsilyl-1,5-anhydro-D-glucitol | Reference Example 4 (y) | Rf 0.21 ($CH_2Cl_2$:$CH_3OH$ = 9:1) |

Reference example 7

Synthesis of 2-deoxy-2[3S-(9-phenylnonanoyloxy)tetradecanoyl] amino-3-0-(9-phenylnonanoyl)-6-tosyl-1,5-anhydro-D-glucitol

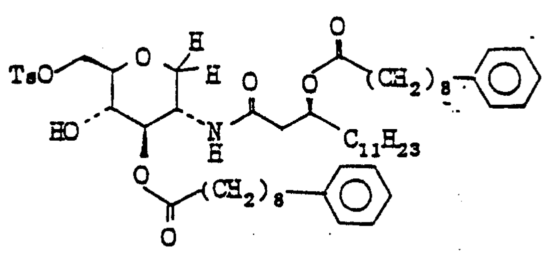

A mixture of the compound (597 mg) prepared in reference example 3(t), tosyl chloride (146 mg) and pyridine (3 mℓ) was stirred overnight at room temperature. After reaction, toluene was added to the reaction mixture, and the mixture was azeotropically evaporated. To the residue, water and methylene chloride were added. The organic layer separated was washed with an aqueous saturated solution of copper sulfate, water and brine, successively, dried and evaporated. The residue was purified by column chromatography on silica gel (n-$C_6H_{14}$:EtOAc = 2:1) to give the title compound (461 mg) having the following physical data.

TLC: Rf 0.49 (n-$C_6H_{14}$:EtOAc = 1:1)

Reference example 8

Synthesis of 2-deoxy-2-[3S-(9-phenylnonanoyloxy)tetradecanoyl] amino-3-0-(9-phenylnonanoyl)-6-deoxy-6-iodo-1,5-anhydro-D-glucitol

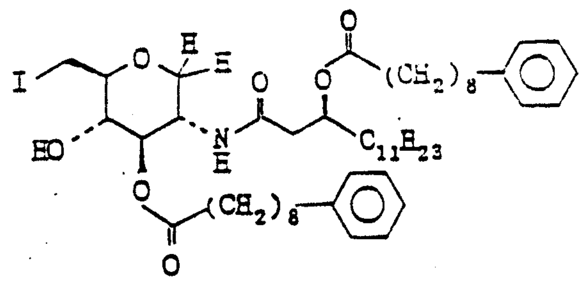

A mixture of the compound (461 mg) prepared in reference example 7, sodium iodide (1.42 g) and acetone (7 mℓ) was refluxed for 4 hrs. After reaction, to the reaction mixture, water and methylene chloride were added. The organic layer separated was washed, dried and evaporated. The residue was purified by column chromatography on silica gel (n-$C_6H_{14}$:EtOAc = 4:1) to give the title compound (232 mg) having the following physical data.

TLC: Rf 0.35 (n-$C_6H_{14}$:EtOAc = 2:1);

IR (liquid film): $\nu$ 3600-3150, 2920, 2850, 1730, 1650, 1530, 1450, 1370 $cm^{-1}$

Reference example 9

Synthesis of 2,6-dideoxy-2-[3S-(9-phenylnonanoyloxy)tetradecanoyl] amino-3-0-(9-phenylnonanoyl)-1,5-anhydro-D-glucitol

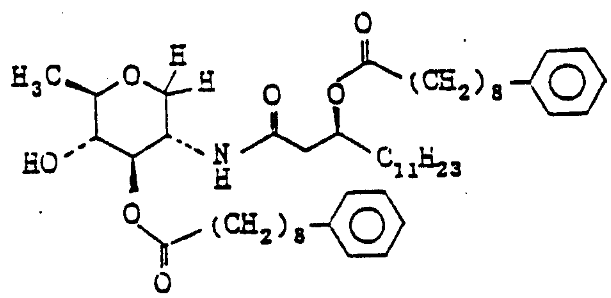

A mixture of the compound (230 mg) prepared in reference example 8, tri-n-butylstanane (133 μℓ), α,α'-arobisisobutyronitrile (AIBN; a small amount) and toluene (4 mℓ) was irradiated by high-voltage mercury light for 1.5 hrs with ice-cooling. The reaction mixture was purified by column chromatography on silica gel (n-$C_6H_{14}$:EtOAc = 2:1) to give the title compound (215 mg) having the following physical data.

TLC: Rf 0.39 (n-$C_6H_{14}$:EtOAc = 6:4)

Reference example 10

Synthesis of benzyl 2-deoxy-2-[9-(1-naphthyl)nonanoyl]amino-3-0-[9-(1-naphthyl)nonanoyl]-4,6-0-isopropylidene-β-D-glucopyranoside

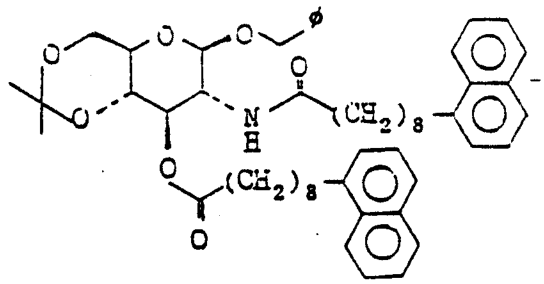

A mixture of benzyl 2-deoxy-2-amino-4,6-0-isopropylidene-β-D-glucopyranoside (248 mg), 9-(1-naphthyl)nonanoic acid (550 mg), 2-chloro-1-methylpyridinium iodide (450 mg), triethylamine (0.34 mℓ), 4-(N,N-dimethylamino)pyridine (98 mg) and methylene chloride (20 mℓ) was stirred for 2 hrs at room temperature. To the reaction mixture, brine and ethyl acetate were added. The organic layer separated was dried and purified by column chromatography on silica gel (n-$C_6H_{14}$:EtOAc = 4:1) to give the title compound (611 mg) having the following physical data.

TLC: Rf 0.59 (EtOAc:n-$C_6H_{14}$ = 3:5);

IR (liquid film): ν 3480, 3380, 2920, 2850, 1730, 1650, 1590 $cm^{-1}$

Reference example 10(a)

Synthesis of benzyl 2-deoxy-2-[9-(2-naphthyl)nonanoyl]amino-3-0-[9-(2-naphthyl)nonanoyl]-4,6-0-isopropylidene-β-D-glucopyranoside

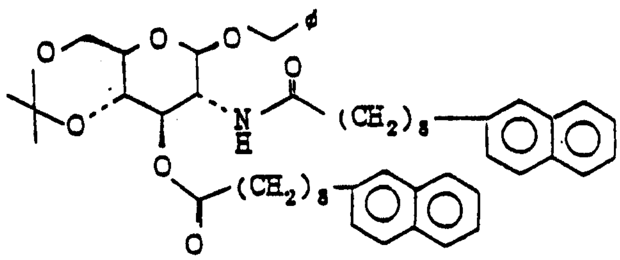

By the same procedure as reference example 10, using with 9-(2-naphthyl)nonanoic acid instead of 9-(1-naphthyl)nonanoic acid, the title compound having the following physical data was given.

TLC: Rf 0.80 (EtOAc:n-$C_6H_{14}$ = 3:5);

IR : ν 3350, 2920, 2840, 1730, 1650, 1520 $cm^{-1}$

Reference example 11

Synthesis of benzyl 2-deoxy-2-[9-(1-naphthyl)nonanoyl]amino-3-0-[9-(1-naphthyl)nonanoyl]-β-D-glucopyranoside

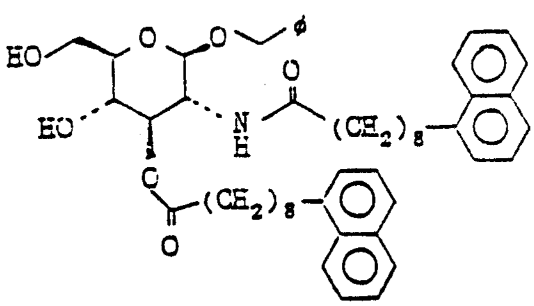

By the same procedure as reference example 3, using with the compound (611 mg) prepared in reference example 10, the title compound having the following physical data was given.

TLC: Rf 0.05 (EtOAc:n-$C_6H_{14}$ = 3:5)

Reference example 11(a)

Synthesis of 2-deoxy-2-[9-(2-naphthyl)nonanoyl]amino-3-0-[9-(2-naphthyl)nonanoyl]-β-D-glucopyranoside

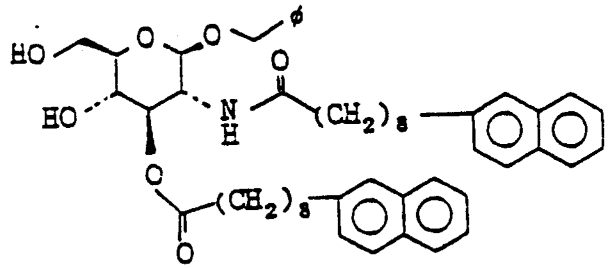

By the same procedure as reference example 11, using with the compound prepared in reference example 10(a), the title compound was given.

Reference example 12

Synthesis of benzyl 2-deoxy-2-[9-(1-naphthyl)nonanoyl]amino-3-0-[9-(1-naphthyl)nonanoyl]-6-0-t-butyldimethylsilyl-β-D-glucopyranoside

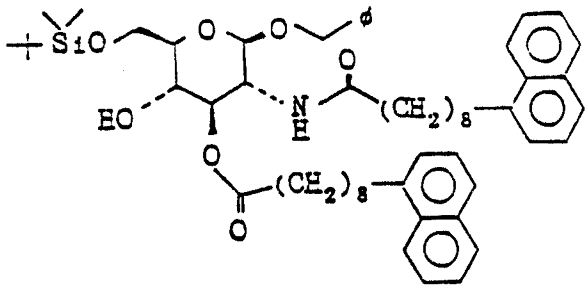

By the same procedure as reference example 4, using the compound prepared in the reference example 11, the title compound (493 mg) having the following physical data was given.

TLC: Rf 0.55 (EtOAc:n-$C_6H_{14}$ = 3:5);

IR : ν 3430, 3250, 3050, 2920, 2850, 1730, 1710, 1640, 1540 $cm^{-1}$

Reference example 12(a)

Synthesis of benzyl 2-deoxy-2-[9-(2-naphthyl)nonanoyl]amino-3-0-[9-(2-naphthyl)nonanoyl]-6-0-t-butyldimethylsilyl-β-D-glucopyranoside

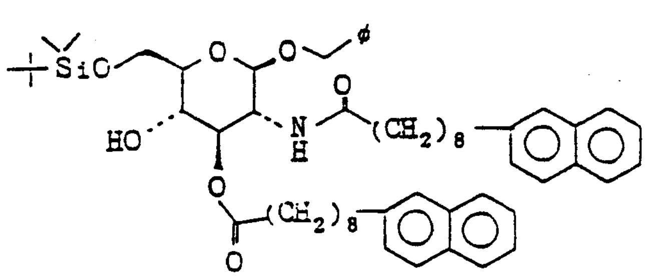

By the same procedure as reference example 12, using the compound prepared in reference example 11(a), the title compound having the following physical data was given.

IR : ν 3450, 2920, 2850, 1730, 1710, 1640, 1540 $cm^{-1}$

Reference example 13

Synthesis of 2-deoxy-2-[9-(1-naphthyl)nonanoyl]amino-3-0-[9-(1-naphthyl)nonanoyl]-6-0-t-butyldimethylsilyl-D-glucopyranose

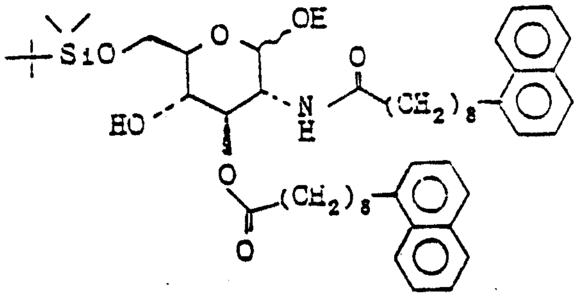

By the same procedure as reference example 5, using with the compound (493 mg) prepared in reference example 12, the title compound having the following physical data was given.

TLC: Rf 0.33 (EtOAc:n-$C_6H_{14}$ = 3:5)

Reference example 13(a)

Synthesis of 2-deoxy-2-[9-(2-naphthyl)nonanoyl]amino-3-0-[9-(2-naphthyl)nonanoyl]-6-0-t-butyldimethylsilyl-D-glucopyranose

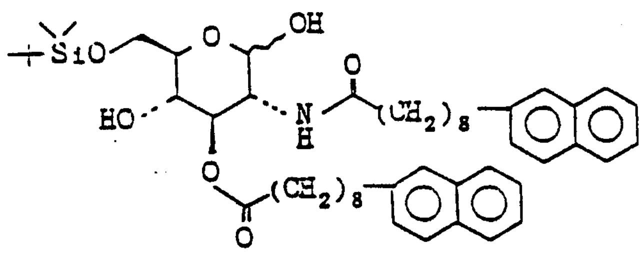

By the same procedure as reference example 13, using with the compound prepared in reference example 12(a), the title compound having the following physical data.

TLC: Rf 0.59 (EtOAc:n-$C_6H_{14}$ = 3:5)

Reference example 14

Synthesis of 2-deoxy-2-[9-(1-naphthyl)nonanoyl]amino-3-0-[9-(1-naphthyl)nonanoyl]-4-0-sulfo-6-0-t-butyldimethylsilyl-D-glucopyranose

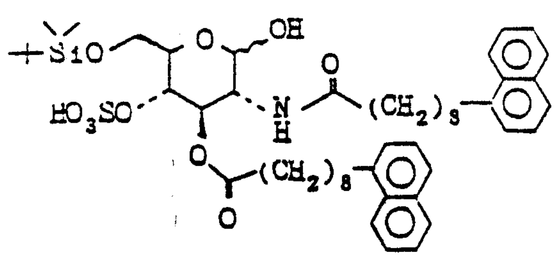

By the same procedure as reference example 6, using with the compound prepared in reference example 13, the title compound having the following physical data was given.

Reference example 14(a)

Synthesis of 2-deoxy-2-[9-(2-naphthyl)nonanoyl]amino-3-0-[9-(2-naphthyl)nonanoyl]-4-0-sulfo-6-0-t-butyldimethylsilyl-D-glucopyranose

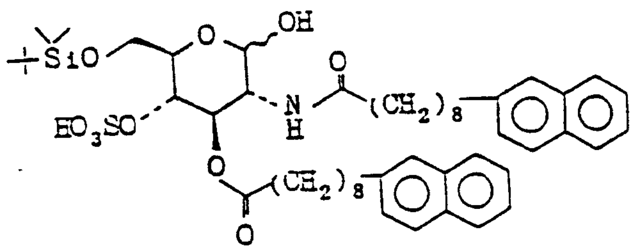

By the same procedure as reference example 14, using with the compound prepared in reference example 13(a), the title compound having the following physical data.

TLC: Rf 0.73 ($CH_3OH$:$CH_2C\ell_2$ = 2:9)

Example 1

Synthesis off 2-deoxy-2-[3R-(4-phenylbutyryloxy)tetradecanoyl] amino-3-0-(4-phenylbutyryl)-4-0-sulfo-D-glucopyranose

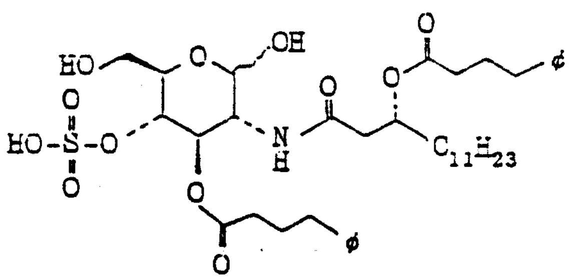

A solution of the compound (550 mg) prepared in reference example 6 dissolved in a mixed solvent of methanol (4 m$\ell$) and acetic acid (3 m$\ell$) was stirred for 9 hrs at room temperature. The reaction solution was evaporated, and toluene was added to the residue. The solution was evaporated. The residue was purified by column chromatography on silica gel ($CH_2C\ell_2$:$CH_3OH$ = 88:12). To the compound obtained, dry dioxan was added, and the solution was freeze-dried to give the title compound (514 mg) having the following physical data.

TLC: Rf 0.37 ($CH_2C\ell_2$:$CH_3OH$ = 9:1);

IR : $\nu$ 3350, 2920, 2840, 1720, 1640, 1530, 1450, 1240, 1130, 1080, 1000, 820, 740, 700, 580 $cm^{-1}$

Example 1(a) - 1(aa)

By the same procedure as example 1, using each starting materials, following compounds having the physical data shown in the Table X were given.

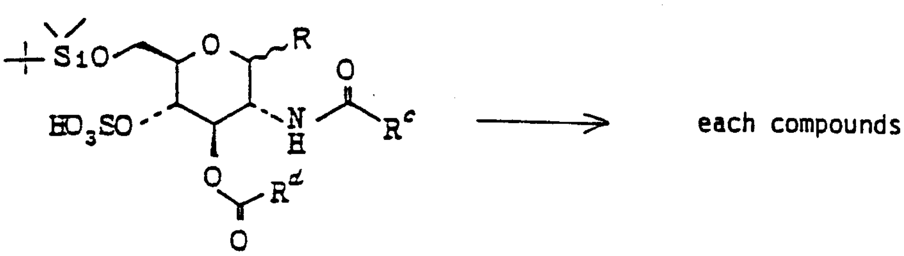

Table X

| No. | Structural Formula | Name | Starting Material | TLC | IR ($cm^{-1}$) |
|---|---|---|---|---|---|
| 1 (a) | 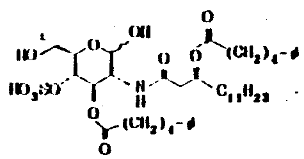 | 2-deoxy-2-[3S-(5-phenylpentanoyloxy)tetradecanoyl]amino-3-O-(5-phenylpentanoyl)-4-O-sulfo-D-glucopyranose | Reference Example 6 (a) | Rf 0.27 ($CH_2Cl_2$: $CH_3OH$ = 85:15) | ν 3400, 2920, 2850, 1720, 1650, 1520, 1450, 1360, 1240, 1120, 1050, 990, 960, 820, 760, 740, 690, 580 |
| 1 (b) | 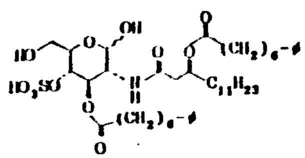 | 2-deoxy-2-[3S-(7-phenylheptanoyloxy)tetradecanoyl]amino-3-O-(7-phenylheptanoyl)-4-O-sulfo-D-glucopyranose | Reference Example 6 (b) | Rf 0.27 ($CH_2Cl_2$: $CH_3OH$ = 85:15) | ν 3400, 2920, 2850, 1720, 1650, 1520, 1450, 1360, 1240, 1120, 1050, 990, 960, 820, 760, 740, 690, 580 |
| 1 (c) | 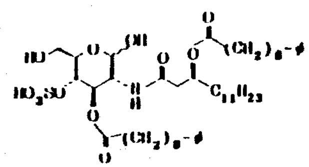 | 2-deoxy-2-[3S-(9-phenylnonanoyloxy)tetradecanoyl]amino-3-O-(9-phenylnonanoyl)-4-O-sulfo-D-glucopyranose | Reference Example 6 (c) | Rf 0.35 ($CH_2Cl_2$: $CH_3OH$ = 85:15) | ν 3500-3300, 2940, 2855, 1730, 1670, 1460, 1265, 1170, 1120, 1045, 1000 |
| 1 (d) | 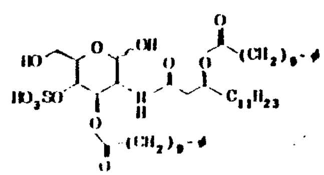 | 2-deoxy-2-[3S-(10-phenyldecanoyloxy)tetradecanoyl]amino-3-O-(10-phenyldecanoyl)-4-O-sulfo-D-glucopyranose | Reference Example 6 (d) | Rf 0.17 ($CH_2Cl_2$: $CH_3OH$ = 85:15) | ν 3400, 2930, 2850, 1720, 1660, 1540, 1480, 1270, 1170, 1120, 1040, 1000, 820, 690, 590 |

| No. | Structural Formula | Name | Starting Material | TLC | IR ($cm^{-1}$) |
|---|---|---|---|---|---|
| 1 (e) | 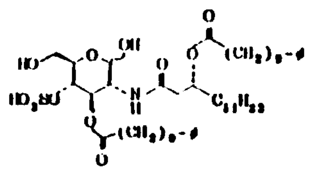 | 2-deoxy-2-[3R-(10-phenyldecanoyloxy) tetradecanoyl]amino-3-O-(10-phenyldecanoyl)-4-O-sulfo-D-glucopyranose | Reference Example 6 (e) | Rf 0.17 ($CH_2Cl_2$: $CH_3OH$ = 85:15) | $\nu$ 3400, 2930, 1720, 1660, 1540, 1480, 1270, 1170, 1120, 1040, 1000, 820, 590 |
| 1 (f) | 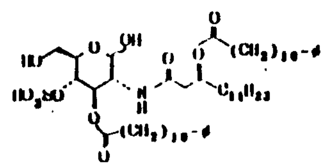 | 2-deoxy-2-[3S-(11-phenylundecanoyloxy) tetradecanoyl]amino-3-O-(11-phenylundecanoyl)-4-O-sulfo-D-glucopyranose | Reference Example 6 (f) | Rf 0.35 ($CH_2Cl_2$: $CH_3OH$ = 85:15) | $\nu$ 3400, 2930, 2850, 1720, 1640, 1530, 1450, 1270, 1220, 1110, 1030, 990, 820, 740, 690 |
| 1 (g) | 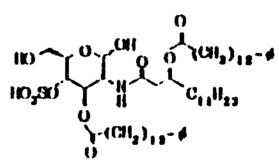 | 2-deoxy-2-[3S-(13-phenyltridecanoyloxy) tetradecanoyl]amino-3-O-(13-phenyltridecanoyl)-4-O-sulfo-D-glucopyranose | Reference Example 6 (g) | Rf 0.35 ($CH_2Cl_2$: $CH_3OH$ = 85:15) | $\nu$ 3400, 2940, 2850, 1720, 1650, 1460, 1270, 1120, 1050, 1000, 820, 700 |
| 1 (h) | 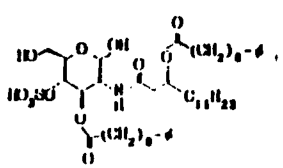 | 2-deoxy-2-[3R-(9-phenylnonanoyloxy) tetradecanoyl]amino-3-O-(9-phenylnonanoyl)-4-O-sulfo-D-glucopyranose | Reference Example 6 (h) | Rf 0.45 ($CH_2Cl_2$: $CH_3OH$ = 6:1) | $\nu$ 3400, 2920, 2850, 1720, 1640, 1540, 1450, 1370, 1250, 1120, 1040, 990 |

| No. | Structural Formula | Name | Starting Material | TLC | IR ($cm^{-1}$) |
|---|---|---|---|---|---|
| 1 (m) | 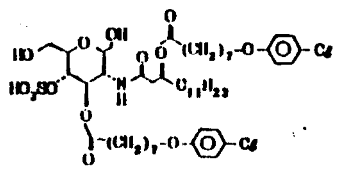 | 2-deoxy-2-[3S-[8-(4-chlorophenoxy)octanoyloxy]tetradecanoyl]amino-3-O-[8-(4-chlorophenoxy)octanoyl]-4-O-sulfo-D-glucopyranose | Reference Example 6 (m) | Rf 0.40 ($CH_2Cl_2$: $CH_3OH$ = 6:1) | ν 3400, 2920, 2850, 1730, 1650, 1600, 1580, 1530, 1490, 1470, 1390, 1240, 1170, 1110, 1050, 820, 750 |
| 1 (n) | 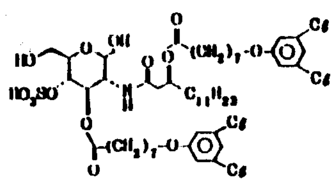 | 2-deoxy-2-[3S-[8-(3,5-dichlorophenoxy)octanoyloxy]tetradecanoyl]amino-3-O-[8-(3,5-dichlorophenoxy)octanoyl]-4-O-sulfo-D-glucopyranose | Reference Example 6 (n) | Rf 0.28 ($CH_2Cl_2$: $CH_3OH$ = 10:1) | ν 3400, 2920, 2850, 1730, 1650, 1590, 1570, 1440, 1420, 1380, 1260, 1220, 1100, 1030, 990, 830, 800 |
| 1 (o) | 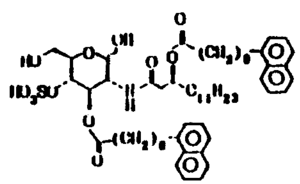 | 2-deoxy-2-[3S-[9-(1-naphthyl)nonanoyloxy]tetradecanoyl]amino-3-O-[9-(1-naphthyl)nonanoyl]-4-O-sulfo-D-glucopyranose | Reference Example 6 (o) | Rf 0.20 ($CH_2Cl_2$: $CH_3OH$ = 9:2) | ν 3400, 2910, 2840, 1720, 1650, 1530, 1450, 1370, 1230, 1120, 1040, 1000, 920, 770 |
| 1 (p) | 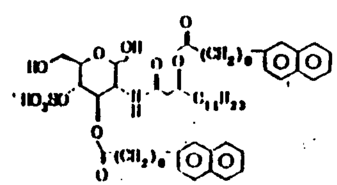 | 2-deoxy-2-[3S-[9-(2-naphthyl)nonanoyloxy]tetradecanoyl]amino-3-O-[9-(2-naphthyl)nonanoyl]-4-O-sulfo-D-glucopyranose | Reference Example 6 (p) | Rf 0.31 ($CH_2Cl_2$: $CH_3OH$ = 5:1) | ν 3400, 2920, 2850, 1720, 1640, 1540, 1450, 1230, 1110 |

| No. | Structural Formula | Name | Starting Material | TLC | IR ($cm^{-1}$) |
| --- | --- | --- | --- | --- | --- |
| 1 (m) | 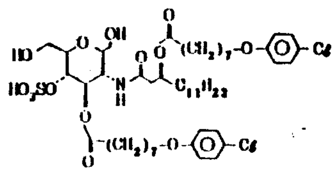 | 2-deoxy-2-[3S-[8-(4-chlorophenoxy)octanoyloxy]tetradecanoyl]amino-3-O-[8-(4-chlorophenoxy)octanoyl]-4-O-sulfo-D-glucopyranose | Reference Example 6 (m) | Rf 0.40 ($CH_2Cl_2$: $CH_3OH$ = 6:1) | ν 3400, 2920, 2850, 1730, 1650, 1600, 1580, 1530, 1490, 1470, 1390, 1240, 1170, 1110, 1050, 820, 750 |
| 1 (n) | 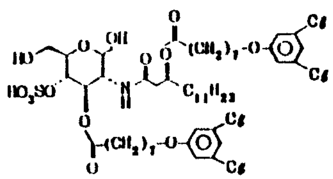 | 2-deoxy-2-[3S-[8-(3,5-dichlorophenoxy)octanoyloxy]tetradecanoyl]amino-3-O-[8-(3,5-dichlorophenoxy)octanoyl]-4-O-sulfo-D-glucopyranose | Reference Example 6 (n) | Rf 0.28 ($CH_2Cl_2$: $CH_3OH$ = 10:1) | ν 3400, 2920, 2850, 1730, 1650, 1590, 1570, 1440, 1420, 1380, 1260, 1220, 1100, 1030, 990, 830, 800 |
| 1 (o) | 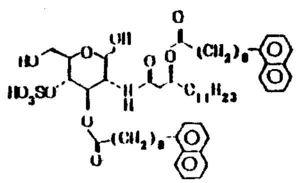 | 2-deoxy-2-[3S-[9-(1-naphthyl)nonanoyloxy]tetradecanoyl]amino-3-O-[9-(1-naphthyl)nonanoyl]-4-O-sulfo-D-glucopyranose | Reference Example 6 (o) | Rf 0.20 ($CH_2Cl_2$: $CH_3OH$ = 9:2) | ν 3400, 2910, 2840, 1720, 1650, 1530, 1450, 1370, 1230, 1120, 1040, 1000, 920, 770 |
| 1 (p) | 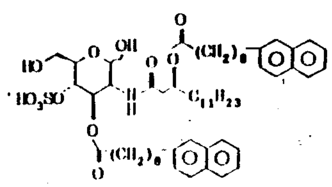 | 2-deoxy-2-[3S-[9-(2-naphthyl)nonanoyloxy]tetradecanoyl]amino-3-O-[9-(2-naphthyl)nonanoyl]-4-O-sulfo-D-glucopyranose | Reference Example 6 (p) | Rf 0.31 ($CH_2Cl_2$: $CH_3OH$ = 5:1) | ν 3400, 2920, 2850, 1720, 1640, 1540, 1450, 1230, 1110 |

| No. | Structural Formula | Name | Starting Material | TLC | IR ($cm^{-1}$) |
|---|---|---|---|---|---|
| 1 (q) | 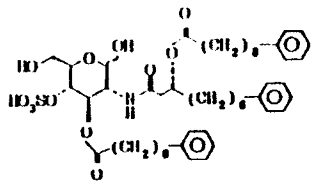 | 2-deoxy-2-[3R-(9-phenylnonanoyloxy)-9-phenylnonanoyl]amino-3-O-(9-phenylnonanoyl)-4-O-sulfo-D-glucopyranose | Reference Example 6 (q) | Rf 0.10 ($CH_2C\ell_2$: $CH_3OH$ = 9:1) | ν 3400, 2920, 2840, 1720, 1650, 1530, 1450 |
| 1 (r) | 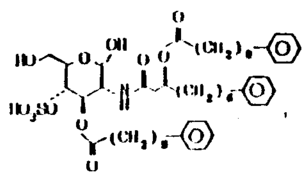 | 2-deoxy-2-[3S-(9-phenylnonanoyloxy)-9-phenylnonanoyl]amino-3-O-(9-phenylnonanoyl)-4-O-sulfo-D-glucopyranose | Reference Example 6 (r) | Rf 0.10 ($CH_2C\ell_2$: $CH_3OH$ = 9:1) | ν 3400, 3340, 2920, 2850, 1720, 1650, 1540, 1440, 1210, 1140, 980, 690 |
| 1 (s) | 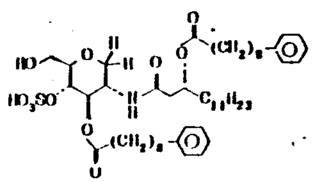 | 2-deoxy-2-[3R-(9-phenylnonanoyloxy) tetradecanoyl]amino-3-O-(9-phenylnonanoyl)-4-O-sulfo-1,5-anhydro-D-glucitol | Reference Example 6 (s) | Rf 0.35 ($CH_2C\ell_2$: $CH_3OH$ = 5:1) | ν 3430, 2930, 2850, 1730, 1660, 1555, 1460, 1245 |
| 1 (t) | 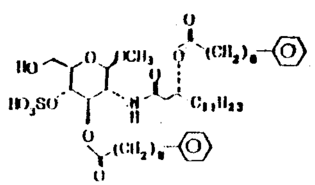 | methyl 2-deoxy-2-[3R-(9-phenylnonanoyloxy) tetradecanoyl]amino-3-O-(9-phenylnonanoyl)-4-O-sulfo-β-D-glucopyranoside | Reference Example 6 (t) | Rf 0.30 ($CH_2C\ell_2$: $CH_3OH$ = 6:1) | ν 3450, 2925, 2850, 1735, 1650, 1540, 1460, 1380, 1240, 1040, 995 |

| No. | Structural Formula | Name | Starting Material | TLC | IR ($cm^{-1}$) |
|---|---|---|---|---|---|
| 1 (u) | 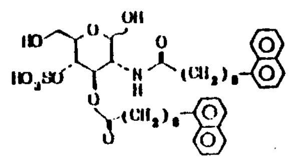 | 2-deoxy-2-[9-(1-naphthyl)nonanoyl]amino-3-O-[9-(1-naphthyl)nonanoyl]-4-O-sulfo-D-glucopyranose | Reference Example 14 | Rf 0.19 ($CH_3OH$: $CH_2Cl_2$ = 2:9) | ν 3400, 2940, 2860, 1730, 1660, 1530, 1260, 1010 |
| 1 (v) | 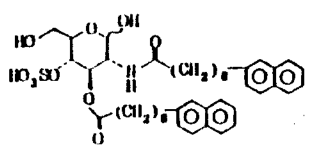 | 2-deoxy-2-[9-(2-naphthyl)nonanoyl]amino-3-O-[9-(2-naphthyl)nonanoyl]-4-O-sulfo-D-glucopyranose | Reference Example 14 (a) | Rf 0.34 ($CH_3OH$: $CH_2Cl_2$ = 1:10) | ν 3450, 3350, 2910, 2850, 1720, 1650, 1630, 1540, 1450 |

| No. | Structural Formula | Name | Starting Material | TLC | IR ($cm^{-1}$) |
|---|---|---|---|---|---|
| 1 (w) | 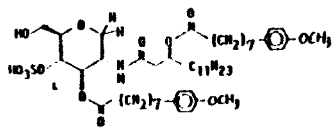 | 2-deoxy-2-[3R-[8-(4-methoxyphenyl)octanoyloxy]tetradecanoyl]amino-3-O-[8-(4-methoxyphenyl)octanoyl]-4-O-sulfo-1,5-anhydro-D-glucitol | Reference Example 6 (u) | Rf 0.34 ($CH_2Cl_2$: $CH_3OH$ = 5:1) | ν 3430, 1730, 1660, 1550 |
| 1 (x) | 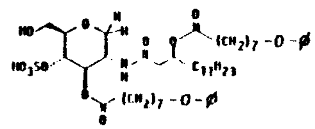 | 2-deoxy-2-[3R-(8-phenoxyoctanoyloxy)tetradecanoyl]amino-3-O-(8-phenoxyoctanoyl)-4-O-sulfo-1,5-anhydro-D-glucitol | Reference Example 6 (v) | Rf 0.30 ($CH_2Cl_2$: $CH_3OH$ = 5:1) | ν 3510, 1728, 1650, 1600, 1538 |
| 1 (y) | 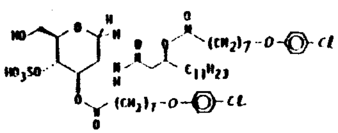 | 2-deoxy-2-[3R-[8-(4-chlorophenoxy)octanoyloxy]tetradecanoyl]amino-3-O-[8-(4-chlorophenoxy)octanoyl]-4-O-sulfo-1,5-anhydro-D-glucitol | Reference Example 6 (w) | Rf 0.29 ($CH_2Cl_2$: $CH_3OH$ = 5:1) | ν 3510, 1730, 1651, 1600, 1540 |
| 1 (z) | 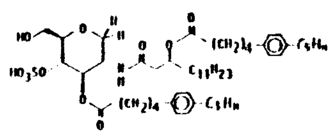 | 2-deoxy-2-[3R-[5-(4-pentylphenyl)pentanoyloxy]tetradecanoyl]amino-3-O-[5-(4-pentylphenyl)pentanoyl]-4-O-sulfo-1,5-anhydro-D-glucitol | Reference Example 6 (x) | Rf 0.30 ($CH_2Cl_2$: $CH_3OH$ = 5:1) | ν 3520, 1730, 1648, 1600, 1540 |

| No. | Structural Formula | Name | Starting Material | TLC | IR ($cm^{-1}$) |
|---|---|---|---|---|---|
| 1 (aa) | 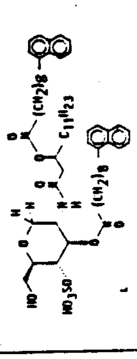 | 2-deoxy-2-[3R-[9-(1-naphthyl)nonanoyloxy] tetradecanoyl]amino-3-O-[9-(1-naphthyl) nonanoyl]-4-O-sulfo-1,5-anhydro-D-glucitol | Reference Example 6 (y) | Rf 0.29 ($CH_2Cl_2$: $CH_3OH$ = 5:1) | ν 3380, 1725, 1653, 1539, 780 |

Example 2

Synthesis of 2,6-deoxy-2-[3S-(9-phenylnonanoyloxy)tetradecanoyl] amino-3-0-(9-phenylnonanoyl)-4-0-sulfo-1,5-anhydro-D-glucitol

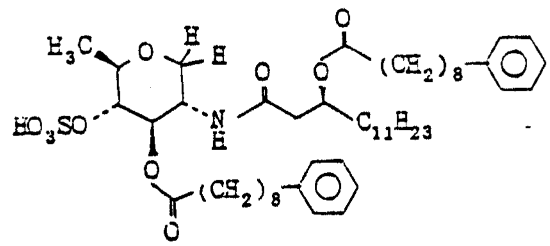

A mixture of the compound (213 mg) prepared in reference example 9, sulfur trioxide-pyridine complex (126 mg) and pyridine (2 mℓ) was stirred for 2.5 hrs at room temperature. After reaction, toluene and ethanol were added to the reaction mixture and the mixture was evaporated azeotropically. The solution was evaporated. The residue was purified by column chromatography on silica gel ($CH_2C\ell_2$:$CH_3OH$ = 15:1). To the compound obtained, dry dioxan was added, and the solution was freeze-dried to give the title compound (180 mg) having the following physical data.

TLC: Rf 0.25 ($CH_2C\ell_2$:$CH_3OH$ = 7:1);

IR : $\nu$ 3430, 2930, 2850, 1710, 1650, 1565, 1450, 1265-1225, 1065, 995 $cm^{-1}$

## Claims

**Claims for the following Contracting States: BE, DE, FR, GB, IT, LU, NL, CH, LI, SE**

**1.** A glucopyranose derivative of general formula:

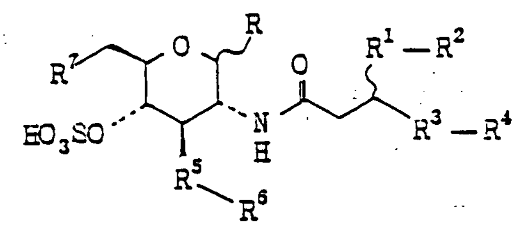

(I)

[wherein, R represents a hydrogen atom, a hydroxy group or an alkoxy group of from 1 to 4 carbon atom(s),

$R^1$ represents a single bond or an oxycarbonylalkyl group of from 2 to 20 carbon atoms,

$R^2$ and $R^6$, independently, represent a hydrogen atom or a general formula:

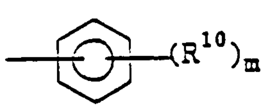

,

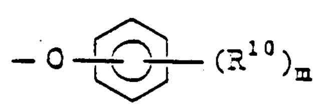

,

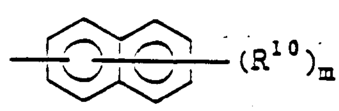

or

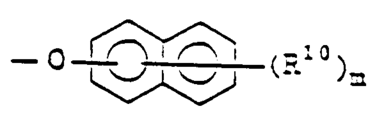

(wherein, $R^{10}$ represents a hydrogen atom, an alkyl or alkoxy group of from 1 to 7 carbon atom(s) or a halogen atom, and m represents 1, 2 or 3 ), respectively,

$R^3$ represents an alkylene group of from 1 to 20 carbon atom(s),

$R^4$ represents a hydrogen atom or a general formula:

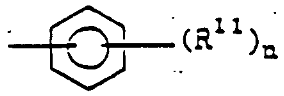 , 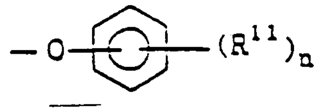 ,

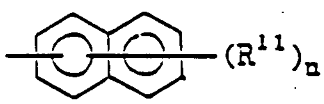 or 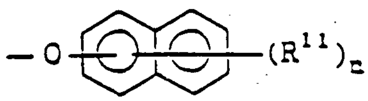

(wherein, $R^{11}$ represents a hydrogen atom, an alkyl or alkoxy group of from 1 to 7 carbon atom(s) or a halogen atom, and n represents 1, 2 or 3 ),

$R^5$ represents an oxycarbonyl group of from 2 to 20 carbon atoms,

$R^7$ represents a hydrogen atom or a hydroxy group, with

the proviso that, $R^2$, $R^4$ and $R^6$ do not represent hydrogen atoms at the same time ]; or a non-toxic salt thereof.

**2.** A compound according to claim 1, wherein $R^7$ is a hydroxy group.

**3.** A compound according to claim 2, wherein R is a hydroxy group.

**4.** A compound according to claim 3, wherein $R^2$ is a phenyl or phenoxy group, and $R^4$ is a hydrogen atom.

**5.** A compound according to claim 4, which is selected from the group consisting of
2-deoxy-2-[3R-(4-phenylbutyryloxy)tetradecanoyl]amino-3-O-(4-phenylbutyryl)-4-O-sulfo-D-glucopyranose,
2-deoxy-2-[3S-(5-phenylpentanoyloxy)tetradecanoyl]amino-3-O-(5-phenylpentanoyl)-4-O-sulfo-D-glucopyranose,
2-deoxy-2-[3S-(7-phenylheptanoyloxy)tetradecanoyl]amino-3-O-(7-phenylheptanoyl)-4-O-sulfo-D-glucopyranose,
2-deoxy-2-[3S-(9-phenylnonanoyloxy)tetradecanoyl]amino-3-O-(9-phenylnonanoyl)-4-O-sulfo-D-glucopyranose,
2-deoxy-2-[3S-(10-phenyldecanoyloxy)tetradecanoyl]amino-3-O-(10-phenyldecanoyl)-4-O-sulfo-D-glucopyranose,
2-deoxy-2-[3R-(10-phenyldecanoyloxy)tetradecanoyl]amino-3-O-(10-phenyldecanoyl)-4-O-sulfo-D-glucopyranose,
2-deoxy-2-[3S-(11-phenylundecanoyloxy)tetradecanoyl]amino-3-O-(11-phenylundecanoyl)-4-O-sulfo-D-glucopyranose,
2-deoxy-2-[3S-(13-phenyltridecanoyloxy)tetradecanoyl]amino-3-O-(13-phenyltridecanoyl)-4-O-sulfo-D-glucopyranose,
2-deoxy-2-[3R-(9-phenylnonanoyloxy)tetradecanoyl]amino-3-O-(9-phenylnonanoyl)-4-O-sulfo-D-glucopyranose and
2-deoxy-2-[3S-(8-phenoxyoctanoyloxy)tetradecanoyl]amino-3-O-(8-phenoxyoctanoyl)-4-O-sulfo-D-glucopyranose.

**6.** A compound according to claim 3, wherein both of $R^2$ and $R^4$ are phenyl groups.

**7.** A compound according to claim 6, which is selected from the group consisting of
2-deoxy-2-[3R-(9-phenylnonanoyloxy)-9-phenylnonanoyl]amino-3-O-(9-phenylnonanoyl)-4-O-sulfo-D-glucopyranose and
2-deoxy-2-[3S-(9-phenylnonanoyloxy)-9-phenylnonanoyl]amino-3-O-(9-phenylnonanoyl)-4-O-sulfo-D-glucopyranose.

**8.** A compound according to claim 3, wherein $R^2$ is a substituted phenyl or phenoxy group.

**9.** A compound according to claim 8, which is selected from the group consisting of
2-deoxy-2-[3S-[8-(4-methoxyphenyl)octanoyloxy]tetradecanoyl]amino-3-O-[8-(4-methoxyphenyl)-octanoyl]-4-O-sulfo-D-glucopyranose,

2-deoxy-2-[3S-[9-(4-chlorophenyl)nonanoyloxy]tetradecanoyl]amino-3-O-[9-(4-chlorophenyl)nonanoyl]-4-O-sulfo-D-glucopyranose,
2-deoxy-2-[3S-[5-(4-pentylphenyl)pentanoyloxy]tetradecanoyl]amino-3-O-[5-(4-pentylphenyl)pentanoyl]-4-O-sulfo-D-glucopyranose,
2-deoxy-2-[3S-[8-(4-chlorophenoxy)octanoyloxy]tetradecanoyl]amino-3-O-[8-(4-chlorophenoxy)octanoyl]-4-O-sulfo-D-glucopyranose and
2-deoxy-2-[3S-[8-(3,5-dichlorophenoxy)octanoyloxy]tetradecanoyl] amino-3-O-[8-(3,5-dichlorophenoxy)-octanoyl]-4-O-sulfo-D-glucopyranose.

**10.** A compound according to claim 3, wherein $R^2$ is a naphthyl or naphthyloxy group.

**11.** A compound according to claim 10, which is selected from the group consisting of
2-deoxy-2-[3S-[9-(1-naphthyl)nonanoyloxy]tetradecanoyl]amino-3-O-[9-(1-naphthyl)nonanoyl]-4-O-sulfo-D-glucopyranose and
2-deoxy-2-[3S-[9-(2-naphthyl)nonanoyloxy]tetradecanoyl]amino-3-O-[9-(2-naphthyl)nonanoyl]-4-O-sulfo-D-glucopyranose.

**12.** A compound according to claim 3, wherein $R^1$ is a single bond, $R^2$ is a hydrogen atom and $R^4$ is a naphthyl or naphthyloxy group.

**13.** A compound according to claim 12, which is selected from the group consisting of
2-deoxy-2-[9-(1-naphthyl)nonanoyl]amino-3-O-[9-(1-naphthyl) nonanoyl]-4-O-sulfo-D-glucopyranose and
2-deoxy-2-[9-(2-naphthyl)nonanoyl]amino-3-O-[9-(2-naphthyl) nonanoyl]-4-O-sulfo-D-glucopyranose.

**14.** A compound according to claim 2, wherein R is a hydrogen atom.

**15.** A compound according to claim 14, wherein $R^2$ is a phenyl or phenoxy group, and $R^4$ is a hydrogen atom.

**16.** A compound according to claim 15, which is selected from the group consisting of
2-deoxy-2-[3R-(9-phenylnonanoyloxy)tetradecanoyl]amino-3-O-(9-phenylnonanoyl)-4-O-sulfo-1,5-anhydro-D-glucitol and
2-deoxy-2-[3R-(8-phenoxyoctanoyloxy)tetradecanoyl]amino-3-O-(8-phenoxyoctanoyl)-4-O-sulfo-1,5-anhydro-D-glucitol.

**17.** A compound according to claim 14, wherein $R^2$ is a substituted phenyl or phenoxy group.

**18.** A compound according to claim 17, which is selected from the group consisting of
2-deoxy-2-[3R-[8-(4-methoxyphenyl)octanoyloxy)tetradecanoyl]amino-3-O-[8-(4-methoxyphenyl)-octanoyl]-4-O-sulfo-1,5-anhydro-D-glucitol,
2-deoxy-2-[3R-[8-(4-chlorophenoxy)octanoyloxy)tetradecanoyl]amino-3-O-[8-(4-chlorophenoxy)octanoyl]-4-O-sulfo-1,5-anhydro-D-glucitol and
2-deoxy-2-[3R-[5-(4-pentylphenyl)pentanoyloxy]tetradecanoyl]amino-3-O-[5-(4-pentylphenyl)pentanoyl]-4-O-sulfo-1,5-anhydro-D-glucitol.

**19.** A compound according to claim 14, wherein $R^2$ is a naphthyl or naphthyloxy group.

**20.** 2-Deoxy-2-[3R-[9-(1-naphthyl)nonanoyloxy]tetradecanoyl]amino-3-O-[9-(1-naphthyl)octanoyl]-4-O-sulfo-1,5-anhydro-D-glucitol.

**21.** A compound according to claim 2, wherein R is a methoxy group.

**22.** Methyl 2-deoxy-2-[3R-(9-phenylnonanoyloxy)tetradecanoyl]amino-3-O-(9-phenylnonanoyl)-4-O-sulfo-$\beta$-D-glucopyranoside.

**23.** A compound according to claim 1, wherein both of $R^7$ and R are hydrogen atoms.

**24.** 2,6-Deoxy-2-[3S-(9-phenylnonanoyloxy)tetradecanoyl]amino-3-O-(9-phenylnonanoyl)-4-O-sulfo-1,5-anhydro-D-glucitol.

**25.** A process for the preparation of the compound of the general formula:

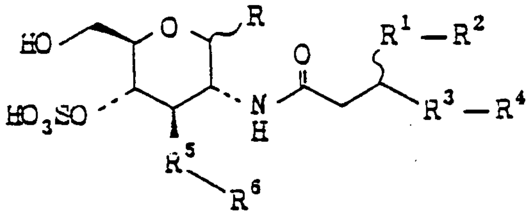
(Ia)

[wherein, R represents a hydrogen atom, a hydroxy group or an alkoxy group of from 1 to 4 carbon atom(s),
$R^1$ represents a single bond or an oxycarbonylalkyl group of from 2 to 20 carbon atoms,
$R^2$ and $R^6$, independently, represent a hydrogen atom or a general formula:

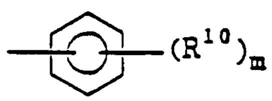 , 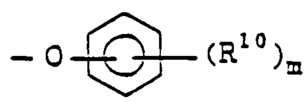 ,

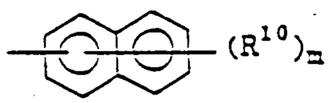 or 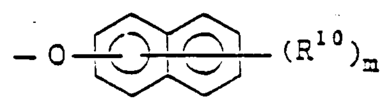

(wherein, $R^{10}$ represents a hydrogen atom, an alkyl or alkoxy group of from 1 to 7 carbon atom(s) or a halogen atom, and m represents 1, 2 or 3 ), respectively,
$R^3$ represents an alkylene group of from 1 to 20 carbon atom(s),
$R^4$ represents a hydrogen atom or a general formula:

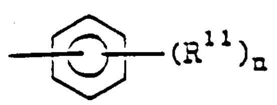 , 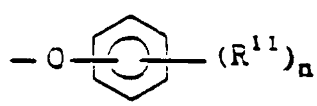 ,

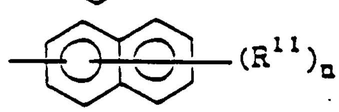 or 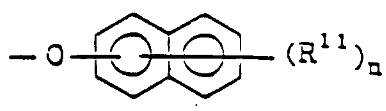

(wherein, $R^{11}$ represents a hydrogen atom, an alkyl or alkoxy group of from 1 to 7 carbon atom(s) or a halogen atom, and n represents 1, 2 or 3 ),
$R^5$ represents an oxycarbonyl group of from 2 to 20 carbon atoms, ith the proviso that, $R^2$, $R^4$ and $R^6$ do not represent hydrogen atoms at the same time ],
which is characterized by hydrol zing a compound of the general formula:

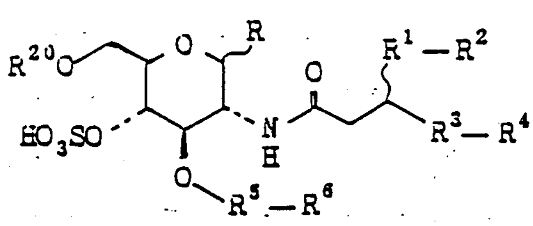
(II)

[wherein $R^{20}$ represents a trialkylsilyl group, and the other symbols are as hereinbefore defined ] in an acidic condition.

**26.** A process for the preparation of the compound of the general formula:

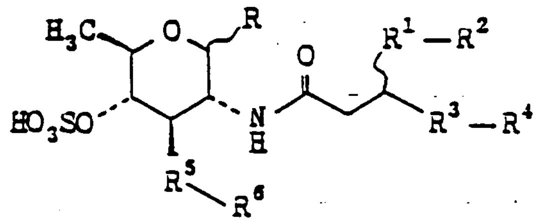

(Ib)

[wherein all of the symbols are as defined in claim 25 ], which is characterized by introducing a sulfo group into the hydroxy group on the 4th position of a compound of the general formula:

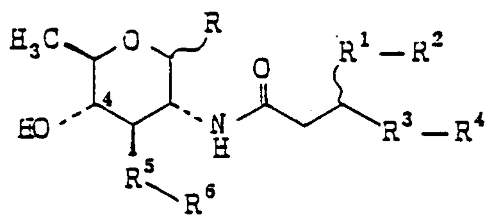

(III)

[wherein all of the symbols are as defined in claim 25].

**27.** A pharmaceutical composition for use in the enhancing immunity characterized by comprising as active ingredient, a compound of the general formula (I), together with a pharmaceutical carrier and/or coating.

**28.** A pharmaceutical composition for use in the treatment of tumor characterized by comprising as active ingredient, a compound of the general formula (I), together with a pharmaceutical carrier and/or coating.

**29.** A compound as claimed in any one of claims 1 to 24 for use as a medicament for enhancing immunity.

**30.** A compound as claimed in any one of claims 1 to 24 for use as a medicament for treating a tumor.

**Claims for the following Contracting States: AT, ES, GR**

**1.** A process for the preparation of a glucopyranose derivative of general formula:

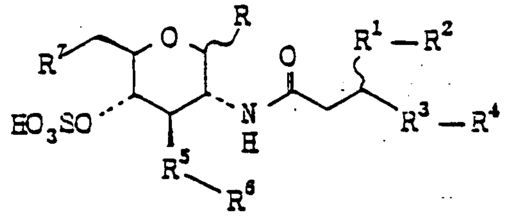

(I)

[wherein, R represents a hydrogen atom, a hydroxy group or an alkoxy group of from 1 to 4 carbon atom(s),
$R^1$ represents a single bond or an oxycarbonylalkyl group of from 2 to 20 carbon atoms,
$R^2$ and $R^6$, independently, represent a hydrogen atom or a general formula:

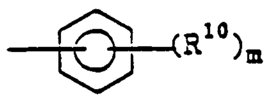 , 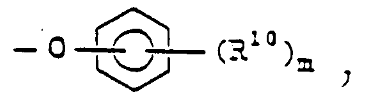 ,

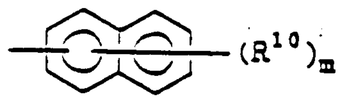 or 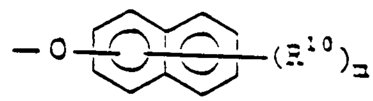

(wherein, $R^{10}$ represents a hydrogen atom, an alkyl or alkoxy group of from 1 to 7 carbon atom(s) or a halogen atom, and m represents 1, 2 or 3.), respectively,
$R^3$ represents an alkylene group of from 1 to 20 carbon atom(s),
$R^4$ represents a hydrogen atom or a general formula:

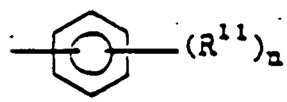 , 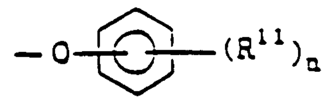 ,

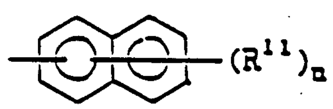 or 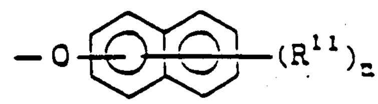

(wherein, $R^{11}$ represents a hydrogen atom, an alkyl or alkoxy group of from 1 to 7 carbon atom(s) or a halogen atom, and n represents 1, 2 or 3 ),
$R^5$ represents an oxycarbonyl group of from 2 to 20 carbon atoms,
$R^7$ represents a hydrogen atom or a hydroxy group, with
the proviso that, $R^2$, $R^4$ and $R^6$ do not represent hydrogen atoms at the same time ]; or a non-toxic salt thereof;
said process being characterized by
(a) when $R^7$ is a hydroxy group, hydrolyzing a compound of the general formula:

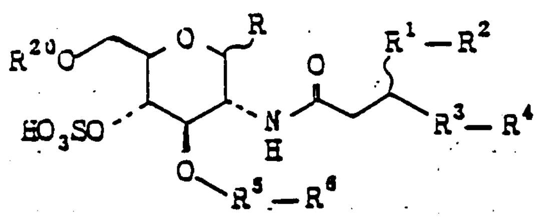 (II)

[wherein $R^{20}$ represents a trialkylsilyl group, and the other symbols are as defined above ]
in an acidic condition; or
(b) when $R^7$ is a hydrogen atom, introducing a sulfo group into the hydroxy group on the 4th position of a compound of the general formula:

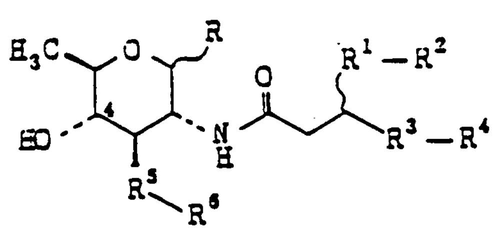 (III)

[wherein all of the symbols are as defined above].

**2.** A process according to claim 1, wherein $R^7$ is a hydroxy group.

**3.** A process according to claim 2, wherein R is a hydroxy group.

**4.** A process according to claim 3, wherein $R^2$ is a phenyl or phenoxy group, and $R^4$ is a hydrogen atom.

**5.** A process according to claim 4, wherein the compound is selected from the group consisting of 2-deoxy-2-[3R-(4-phenylbutyryloxy)tetradecanoyl]amino-3-O-(4-phenylbutyryl)-4-O-sulfo-D-glucopyranose, 2-deoxy-2-[3S-(5-phenylpentanoyloxy)tetradecanoyl]amino-3-O-(5-phenylpentanoyl)-4-O-sulfo-D-glucopyranose, 2-deoxy-2-[3S-(7-phenylheptanoyloxy)tetradecanoyl]amino-3-O-(7-phenylheptanoyl)-4-O-sulfo-D-glucopyranose, 2-deoxy-2-[3S-(9-phenylnonanoyloxy)tetradecanoyl]amino-3-O-(9-phenylnonanoyl)-4-O-sulfo-D-glucopyranose, 2-deoxy-2-[3S-(10-phenyldecanoyloxy)tetradecanoyl]amino-3-O-(10-phenyldecanoyl)-4-O-sulfo-D-glucopyranose, 2-deoxy-2-[3R-(10-phenyldecanoyloxy)tetradecanoyl]amino-3-O-(10-phenyldecanoyl)-4-O-sulfo-D-glucopyranose, 2-deoxy-2-[3S-(11-phenylundecanoyloxy)tetradecanoyl]amino-3-O-(11-phenylundecanoyl)-4-O-sulfo-D-glucopyranose, 2-deoxy-2-[3S-(13-phenyltridecanoyloxy)tetradecanoyl]amino-3-O-(13-phenyltridecanoyl)-4-O-sulfo-D-glucopyranose, 2-deoxy-2-[3R-(9-phenylnonanoyloxy)tetradecanoyl]amino-3-O-(9-phenylnonanoyl)-4-O-sulfo-D-glucopyranose and 2-deoxy-2-[3S-(8-phenoxyoctanoyloxy)tetradecanoyl]amino-3-O-(8-phenoxyoctanoyl)-4-O-sulfo-D-glucopyranose.

**6.** A process according to claim 3, wherein both of $R^2$ and $R^4$ are phenyl groups.

**7.** A process according to claim 6, wherein the compound is selected from the group consisting of 2-deoxy-2-[3R-(9-phenylnonanoyloxy)-9-phenylnonanoyl]amino-3-O-(9-phenylnonanoyl)-4-O-sulfo-D-glucopyranose and 2-deoxy-2-[3S-(9-phenylnonanoyloxy)-9-phenylnonanoyl]amino-3-O-(9-phenylnonanoyl)-4-O-sulfo-D-glucopyranose.

**8.** A process according to claim 3, wherein $R^2$ is a substituted phenyl or phenoxy group.

**9.** A process according to claim 8, wherein the compound is selected from the group consisting of 2-deoxy-2-[3S-[8-(4-methoxyphenyl)octanoyloxy]tetradecanoyl]amino-3-O-[8-(4-methoxyphenyl)-octanoyl]-4-O-sulfo-D-glucopyranose, 2-deoxy-2-[3S-[9-(4-chlorophenyl)nonanoyloxy]tetradecanoyl]amino-3-O-[9-(4-chlorophenyl)nonanoyl]-4-O-sulfo-D-glucopyranose, 2-deoxy-2-[3S-[5-(4-pentylphenyl)pentanoyloxy]tetradecanoyl]amino-3-O-[5-(4-pentylphenyl)pentanoyl]-4-O-sulfo-D-glucopyranose, 2-deoxy-2-[3S-[8-(4-chlorophenoxy)octanoyloxy]tetradecanoyl]amino-3-O-[8-(4-chlorophenoxy)octanoyl]-4-O-sulfo-D-glucopyranose and 2-deoxy-2-[3S-[8-(3,5-dichlorophenoxy)octanoyloxy]tetradecanoyl] amino-3-O-[8-(3,5-dichlorophenoxy)-octanoyl]-4-O-sulfo-D-glucopyranose.

**10.** A process according to claim 3, wherein $R^2$ is a naphthyl or naphthyloxy group.

**11.** A process according to claim 10, wherein the compound is selected from the group consisting of 2-deoxy-2-[3S-[9-(1-naphthyl)nonanoyloxy]tetradecanoyl]amino-3-O-[9-(1-naphthyl)nonanoyl]-4-O-sulfo-D-glucopyranose and 2-deoxy-2-[3S-[9-(2-naphthyl)nonanoyloxy]tetradecanoyl]amino-3-O-[9-(2-naphthyl)nonanoyl]-4-O-sulfo-D-glucopyranose.

**12.** A process according to claim 3, wherein $R^1$ is a single bond, $R^2$ is a hydrogen atom and $R^4$ is a naphthyl or naphthyloxy group.

**13.** A process according to claim 12, wherein the compound is selected from the group consisting of
2-deoxy-2-[9-(1-naphthyl)nonanoyl]amino-3-O-[9-(1-naphthyl) nonanoyl]-4-O-sulfo-D-glucopyranose and
2-deoxy-2-[9-(2-naphthyl)nonanoyl]amino-3-O-[9-(2-naphthyl) nonanoyl]-4-O-sulfo-D-glucopyranose.

**14.** A process according to claim 2, wherein R is a hydrogen atom.

**15.** A process according to claim 14, wherein $R^2$ is a phenyl or phenoxy group, and $R^4$ is a hydrogen atom.

**16.** A process according to claim 15, wherein the compound is selected from the group consisting of
2-deoxy-2-[3R-(9-phenylnonanoyloxy)tetradecanoyl]amino-3-O-(9-phenylnonanoyl)-4-O-sulfo-1,5-anhydro-D-glucitol and
2-deoxy-2-[3R-(8-phenoxyoctanoyloxy)tetradecanoyl]amino-3-O-(8-phenoxyoctanoyl)-4-O-sulfo-1,5-anhydro-D-glucitol.

**17.** A process according to claim 14, wherein $R^2$ is a substituted phenyl or phenoxy group.

**18.** A process according to claim 17, wherein the compound is selected from the group consisting of
2-deoxy-2-[3R-[8-(4-methoxyphenyl)octanoyloxy)tetradecanoyl]amino-3-O-[8-(4-methoxyphenyl)-octanoyl]-4-O-sulfo-1,5-anhydro-D-glucitol,
2-deoxy-2-[3R-[8-(4-chlorophenoxy)octanoyloxy]tetradecanoyl]amino-3-O-[8-(4-chlorophenoxy)octanoyl]-4-O-sulfo-1,5-anhydro-D-glucitol and
2-deoxy-2-[3R-[5-(4-pentylphenyl)pentanoyloxy]tetradecanoyl]amino-3-O-[5-(4-pentylphenyl)pentanoyl]-4-O-sulfo-1,5-anhydro-D-glucitol.

**19.** A process according to claim 14, wherein $R^2$ is a naphthyl or naphthyloxy group.

**20.** A process according to claim 19, wherein the compound is
2-deoxy-2-[3R-[9-(1-naphthyl)nonanoyloxy]tetradecanoyl]amino-3-O-[9-(1-naphthyl)octanoyl]-4-O-sulfo-1,5-anhydro-D-glucitol.

**21.** A process according to claim 2, wherein R is a methoxy group.

**22.** A process according to claim 16, wherein the compound is
methyl 2-deoxy-2-[3R-(9-phenylnonanoyloxy)tetradecanoyl]amino-3-O-(9-phenylnonanoyl)-4-O-sulfo-$\beta$-D-glucopyranoside.

**23.** A process according to claim 1, wherein both of $R^7$ and R are hydrogen atoms.

**24.** A process according to claim 23, wherein the compound is
2,6-deoxy-2-[3S-(9-phenylnonanoyloxy)tetradecanoyl]amino-3-O-(9-phenylnonanoyl)-4-O-sulfo-1,5-anhydro-D-glucitol.

**25.** A process for preparing a pharmaceutical composition comprising provided a compound of general formula:-

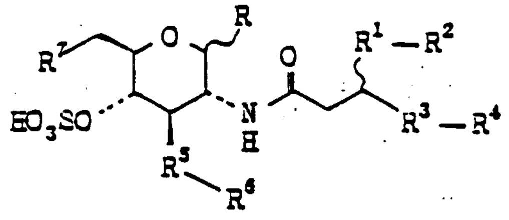

(I)

wherein all the symbols are as defined in claim 1, with a pharmaceutical carrier and/or a coating.

## Revendications

## Revendications pour les Etats contractants suivants: BE, DE, FR, GB, IT, LU, NL, CH, LI, SE

**1.** Un dérivé du glucopyrannose de formule générale :

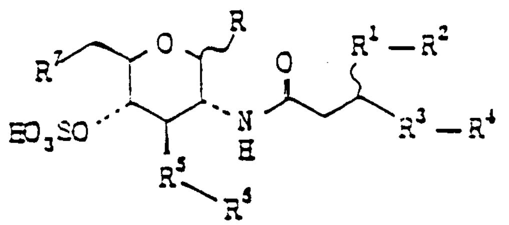

(I)

[dans laquelle R représente un atome d'hydrogène, un groupe hydroxy ou un groupe alcoxy de 1 à 4 atomes de carbone,
$R^1$ représente une liaison simple ou un groupe oxycarbonylalkyle de 2 à 20 atomes de carbone,
$R^2$ et $R^6$ représentent indépendamment un atome d'hydrogène ou une formule générale :

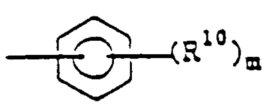 , 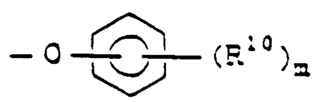 ,

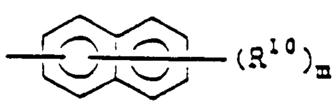 ou 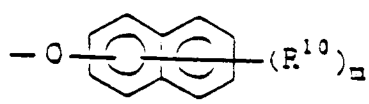

(dans laquelle $R^{10}$ représente un atome d'hydrogène, un groupe alkyle ou alcoxy de 1 à 7 atomes de carbone ou un atome d'halogène et m représente 1, 2 ou 3), respectivement,
$R^3$ représente un groupe alkylène de 1 à 20 atomes de carbone,
$R^4$ représente un atome d'hydrogène ou une formule générale :

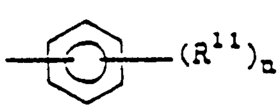 , 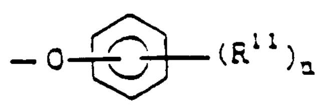 ,

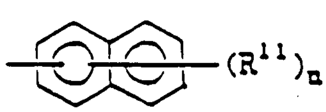 ou 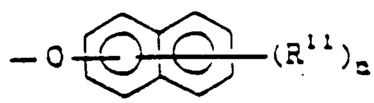

(dans laquelle $R^{11}$ représente un atome d'hydrogène, un groupe alkyle ou alcoxy de 1 à 7 atomes de carbone ou un atome d'halogène, et n représente 1, 2 ou 3),
$R^5$ représente un groupe oxycarbonyle de 2 à 20 atomes de carbone,
$R^7$ représente un atome d'hydrogène ou un groupe hydroxy à la condition que $R^2$, $R^4$ et $R^6$ ne représentent pas des atomes d'hydrogène en même temps] ; ou bien un sel non toxique dudit dérivé.

**2.** Un composé selon la revendication 1, selon lequel $R^7$ un groupe hydroxy.

**3.** Un composé selon la revendication 2, selon lequel R est un groupe hydroxy.

**4.** Un composé selon la revendication 3, selon lequel $R^2$ est un groupe phényle ou phénoxy et $R^4$ est un atome d'hydrogène.

**5.** Un composé selon la revendication 4, qui est sélectionné dans le groupe comprenant les suivants :

2--désoxy--2--[3R--(4--phénylbutyryloxy)tétradécanoyl]amino-3-O-(4-phénylbutyryl)-4-O-sulfo-D-glucopyrannose,
2--désoxy--2--[3S-(5-phénylpentanoyloxy)tétradécanoyl]amino-3-O-(5-phénylpentanoyl)-4-O-sulfo-D-glucopyrannose,
2-désoxy-2-[3S-(7-phénylheptanoyloxy)tétradécanoyl]amino--3--O--(7-phénylheptanoyl)-4-O-sulfo-D-glucopyrannose,
2-désoxy-2--[3S--(-9--phénylnonanoyloxy)tétradécanoyl]amino-3-O-(9-phénylnonanoyl)-4-O-sulfo-D-glucopyrannose,
2-désoxy-2--[3S--(10-phényldécanoyloxy)tétradécanoyl]amino-3-O-(10-phényldécanoyl)-4-O-sulfo-D-glucopyrannose,
2-désoxy-2-[3R-(10-phényldécanoyloxy)tétradécanoyl]amino--3--O-(10-phényldécanoyl)-4-O-sulfo-D-glucopyrannose,
2-désoxy-2-[3S-(11-phénylundécanoyloxy)tétradécanoyl]amino-3-O-(11-phénylundécanoyl)-4-O-sulfo-D-glucopyrannose,
2--désoxy--2--[3S-(13-phényltridécanoyloxy)tétradécanoyl]amino-3-O-(13-phényltridécanoyl)-4-O-sulfo-D-glucopyrannose,
2-désoxy-2-[3R-(9-phénylnonanoyloxy)--tétradécanoyl]amino--3--O-(9-phénylnonanoyl)-4-O-sulfo-D-glucopyrannose et
2-désoxy-2-[3S-(8-phénoxyoctanoyloxy)tétradécanoyl]amino-3-O-(8-phénoxyoctanoyl)-4-O-sulfo-D-glucopyrannose.

**6.** Un composé selon la revendication 3, selon lequel $R^2$ et $R^4$ sont tous les deux des groupes phényle.

**7.** Un composé selon la revendication 6, qui est sélectionné dans le groupe contenant les suivants :
2-désoxy-2-[3R-(9-phénylnonanoyloxy)-9-phénylnonanoyl]amino-3-O-(9-phénylnonanoyl)-4-O-sulfo-D-glucopyrannose et
2-désoxy-2-[3S-(9-phénylnonanoyloxy)-9-phénylnonanoyl]amino-3-O-(9-phénylnonanoyl)-4-O-sulfo-D-glucopyrannose.

**8.** Un composé selon la revendication 3, selon lequel $R^2$ est un groupe phényle ou phénoxy substitué.

**9.** Un composé selon la revendication 8, qui est choisi dans le groupe comprenant les suivants :
2-désoxy-2-[3S-[8-(4-méthoxyphényl)octanoyloxy]tétradécanoyl]amino-3-O-[8-(4-méthoxyphényl)-octanoyl]-4-O-sulfo-D-glucopyrannose,
2--désoxy-2-[3S-[9-(4-chlorophényl)nonanoyloxy]tétradécanoyl]amino-3-O-[9-4-chlorophényl)nonanoyl]-4-O-sulfo-D-glucopyrannose,
2-désoxy-2-[3S-[5-(4-pentylphényl)pentanoyloxy]tétradécanoyl]amino-3-O-[5-(4-pentylphényl)pentanoyl]-4-O-sulfo-D-glucopyrannose,
2-désoxy-2-[3S-[8-(4-chorophénoxy)octanoyloxy]tétradécanoyl]amino-3-O-[8-(4-chlorophénoxy)-octanoyl]-4-O-sulfo-D-glucopyrannose et
2-désoxy--2-[3S-[8-(3,5-dichlorophénoxy)octanoyloxy]tétradécanoyl]amino-3-O-[8-(3,5-dichlorophénoxy)-octanoyl]-4-O-sulfo-D-glucopyrannose.

**10.** Un composé selon la revendication 3, selon lequel $R^2$ est un groupe naphtyle ou naphtyloxy.

**11.** Un composé selon la revendication 10, qui est sélectionné dans le groupe comprenant les suivants :
2-désoxy-2-[3S-[9-(1-naphtyl)nonanoyloxy]tétradécanoyl]amino--3--O-[9-(1-naphtyl)nonanoyl]-4-O-sulfo-D-glucopyrannose et
2-désoxy-2-[3S-[9--(2--naphtyl)nonanoyloxy]tétradécanoyl]amino-3-O-[9-(2-naphtyl)nonanoyl]-4-O-sulfo-glucopyrannose.

**12.** Un composé selon la revendication 3, selon lequel $R^1$ est une liaison simple, $R^2$ est un atome d'hydrogène et $R^4$ est un groupe naphtyle ou naphtyloxy.

**13.** Un composé selon la revendication 12, qui est choisi dans le groupe comprenant les suivants :
2-désoxy-2-[9-(1-naphtyl)nonanoyl]amino--3--O--[9--(1-naphtyl)nonanoyl]-4-O-sulfo-D-glucopyrannose et
2-désoxy-2-[9-(2-naphtyl)nonanoyl]amino-3-O-[9-(2-naphtyl)nonanoyl]-4-O-sulfo-D-glucopyrannose.

**14.** Un composé selon la revendication 2, selon lequel R est un atome d'hydrogène.

**15.** Un composé selon la revendication 14, selon lequel $R^2$ est un groupe phényle ou phénoxy et $R^4$ est un atome d'hydrogène.

**16.** Un composé selon la revendication 15, qui est choisi dans le groupe comprenant les suivants :
2-désoxy-2-[3R-(9--phénylnonanoyloxy)tétradécanoyl]amino--3--O--(9-phénylnonanoyl)-4-O-sulfo-1,5-anhydro-D-glucitol et
2-désoxy-2-[3R-(8-phénoxyoctanoyloxy)tétradécanoyl]amino--3--O--(8-phénoxyoctanoyl)-4-O-sulfo-1,5-anhydro-D-glucitol.

**17.** Un composé selon la revendication 14, selon lequel $R^2$ est un groupe phényle ou phénoxy substitué.

**18.** Un composé selon la revendication 17, qui est choisi dans le groupe comprenant les suivants :
2-désoxy-2-[3R-[8-(4-méthoxyphényl)octanoyloxy)tétradécanoyl]amino-3-O-[8-(4-méthoxyphényl)-octanoyl]-4-O-sulfo-1,5-anhydro-D-glucitol,
2-désoxy-2-[3R-[8-(4-chlorophénoxy)octanoyloxy]tétradécanoyl]amino-3-O-[8-(4-chlorophénoxy)-octanoyl]-4-O-sulfo-1,5-anhydro-D-glucitol et
2-désoxy-2-[3R-[5-(4-pentylphényl)pentanoyloxy]tétradécanoyl]amino-3-O-[5-(4-pentylphényl)-pentanoyl]-4-O-sulfo-1,5-anhydro-D-glucitol.

**19.** Un compose selon la revendication 14, selon lequel $R^2$ est un groupe naphtyle ou naphtyloxy.

**20.** 2--désoxy-2-[3R-[9-(1-naphtyl)nonanoyloxy]tétradécanoyl]amino--3--O--[9-(1-naphtyl)octanoyl]-4-O-sulfo-1,5-anhydro-D--glucitol.

**21.** Un composé selon la revendication 2, selon lequel R est un groupe méthoxy.

**22.** Méthyl-2-désoxy-2-[3R-(9-phénylnonanoyloxy)tétradécanoyl]amino-3-O-(9-phénylnonanoyl)-4-O-sulfo-$\beta$-D-glucopyrannoside.

**23.** Un composé selon la revendication 1, selon lequel $R^7$ et R sont tous les deux des atomes d'hydrogène.

**24.** 2,6-désoxy-2-[3S-(9-phénylnonanoyloxy)tétradécanoyl]amino-3-O-(9-phénylnonanoyl)-4-O-sulfo-1,5-anhydro-D-glucitol.

**25.** Un procédé de préparation du composé de formule générale :

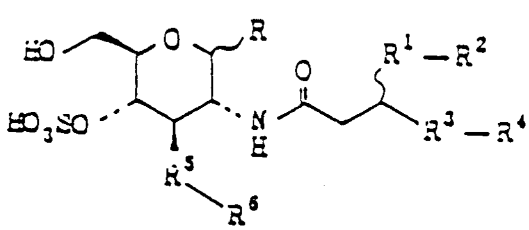

(Ia)

[dans laquelle R représente un atome d'hydrogène, un groupe hydroxy ou un groupe alcoxy de 1 à 4 atomes de carbone,
$R^1$ représente une liaison simple ou un groupe oxycarbonylalkyle de 2 à 20 atomes de carbone,
$R^2$ et $R^6$ représentent indépendamment un atome d'hydrogène ou une formule générale :

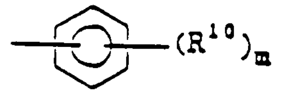,
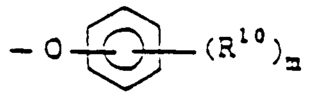,

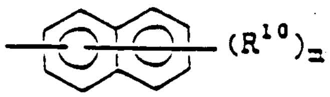
ou
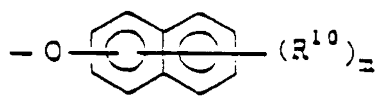

(dans laquelle $R^{10}$ représente un atome d'hydrogène, un groupe alkyle ou alcoxy de 1 à 7 atomes de carbone ou un atome d'halogène, et m représente 1, 2 ou 3), respectivement,
$R^3$ représente un groupe alkylène de 1 à 20 atomes de carbone,
$R^4$ représente un atome d'hydrogène ou une formule générale :

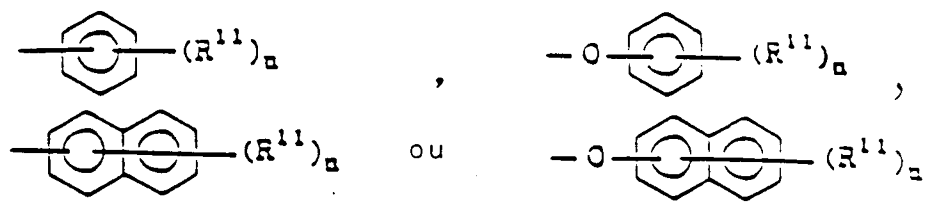
, ou ,

(dans laquelle $R^{11}$ représente un atome d'hydrogène, un groupe alkyle ou alcoxy de 1 à 7 atomes de carbone ou un atome d'halogène, et n représente 1, 2 ou 3),
$R^5$ représente un groupe oxycarbonyle de 2 à 20 atomes de carbone à la condition que $R^2$, $R^4$ et $R^6$ ne représentent pas des atomes d'hydrogène en même temps],
qui est caractérisé par l'hydrolyse d'un composé de formule générale :

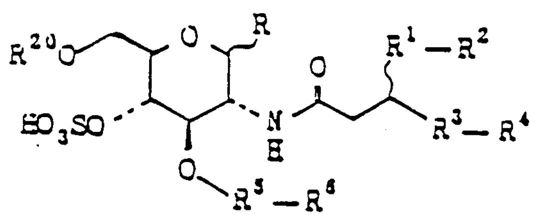
(II)

[dans laquelle $R^{20}$ représente un groupe trialkylsilyle et les autres symboles sont comme défini précédemment] dans des conditions acides.

**26.** Un procédé de préparation du composé de formule générale :

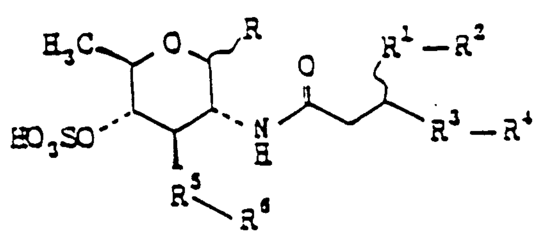
(Ib)

[dans laquelle tous les symboles sont comme défini dans la revendication 25], qui est caractérisé par l'introduction d'un groupe sulfo dans le groupe hydroxy dans la position 4 d'un composé de formule générale :

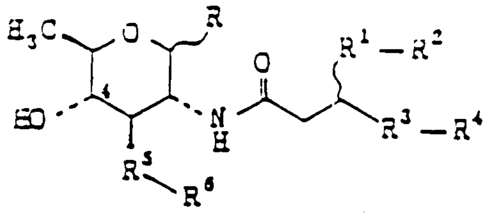

(III)

[dans laquelle tous les symboles sont comme défini dans la revendication 25].

**27.** Une composition pharmaceutique à utiliser pour améliorer l'immunité caractérisée en ce qu'elle comprend comme ingrédient actif, un composé de formule générale (I), ensemble avec un support pharmaceutique et/ou un revêtement pharmaceutique.

**28.** Une composition pharmaceutique à utiliser dans le traitement des tumeurs caractérisée en ce qu'elle comprend comme ingrédient actif un composé de formule générale (I), ensemble avec un support pharmaceutique et/ou un revêtement pharmaceutique.

**29.** Un composé selon l'une quelconque des revendications 1 à 24, à utiliser comme médicament pour améliorer l'immunité.

**30.** Un composé selon l'une quelconque des revendications 1 à 24, à utiliser comme médicament pour le traitement d'une tumeur.

**Revendications pour les Etats contractants suivants: AT, ES, GR**

**1.** Un procédé de préparation d'un dérivé de glucopyrannose de formule générale :

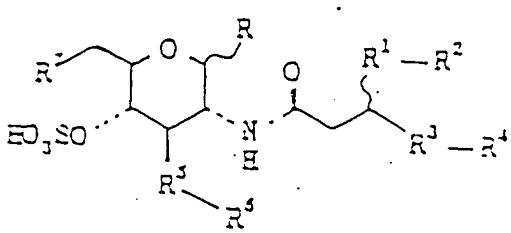

(I)

[dans laquelle R représente un atome d'hydrogène, un groupe hydroxy ou un groupe alcoxy de 1 à 4 atomes de carbone,
$R^1$ représente une liaison simple ou un groupe oxycarbonylalkyle de 2 à 20 atomes de carbone,
$R^2$ et $R^6$ représentent indépendamment un atome d'hydrogène ou une formule générale :

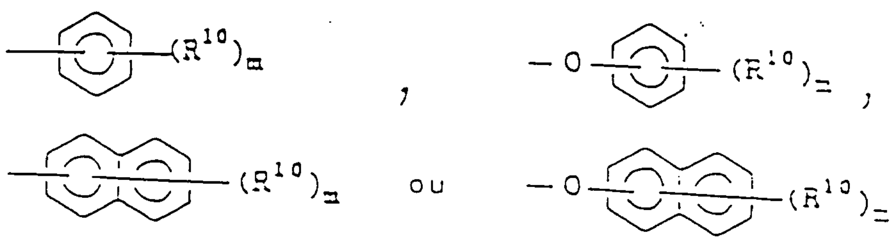

(dans laquelle $R^{10}$ représente un atome d'hydrogène, un groupe alkyle ou alcoxy de 1 à 7 atomes de carbone ou un atome d'halogène et m représente 1, 2 ou 3), respectivement,
$R^3$ représente un groupe alkylène de 1 à 20 atomes de carbone,
$R^4$ représente un atome d'hydrogène ou une formule générale :

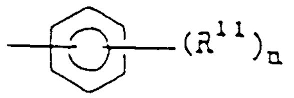 , 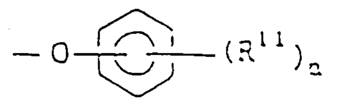 ,

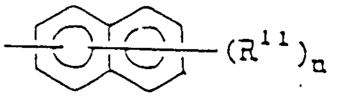 ou 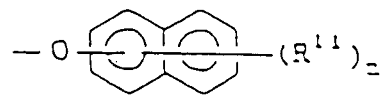

(dans laquelle $R^{11}$ représente un atome d'hydrogène, ou un groupe alkyle ou alcoxy de 1 à 7 atomes de carbone ou un atome d'halogène et n représente 1, 2 ou 3),
$R^5$ représente un groupe oxycarbonyle de 2 à 20 atomes de carbone,
$R^7$ représente un atome d'hydrogène ou un groupe hydroxy, à la condition que $R^2$, $R^4$ et $R^6$ ne représentent pas des atomes d'hydrogène en même temps] ; ou d'un sel non toxique dudit dérivé ;

ledit procédé étant caractérisé par :

(a) lorsque $R^7$ est un groupe hydroxy, l'hydrolyse d'un composé de formule générale :

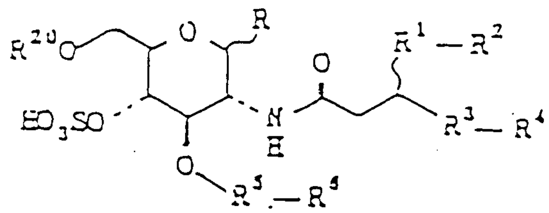

(II)

[dans laquelle $R^{20}$ représente un groupe trialkylsilyle et les autres symboles sont comme défini précédemment] dans des conditions acides ; ou

(b) lorsque $R^7$ est un atome d'hydrogène, l'introduction d'un groupe sulfo dans le groupe hydroxy dans la position 4 d'un composé de formule générale :

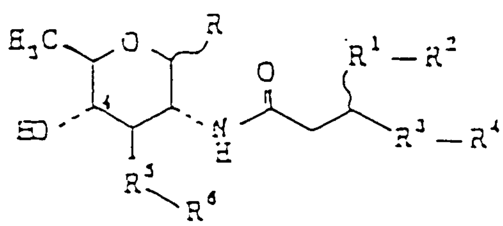

(III)

[dans laquelle tous les symboles sont comme défini précédemment].

2. Un procédé selon la revendication 1, selon lequel $R^7$ est un groupe hydroxy.

3. Un procédé selon la revendication 2, selon lequel R est un groupe hydroxy.

4. Un procédé selon la revendication 3, selon lequel $R^2$ est un groupe phényle ou phénoxy et $R^4$ est un atome d'hydrogène.

5. Un procédé selon la revendication 4, selon lequel le composé est choisi dans le groupe comprenant les suivants :
2--désoxy--2--[3R--(4--phénylbutyryloxy)tétradécanoyl]amino-3-O-(4-phénylbutyryl)-4-O-sulfo-D-glucopyrannose,
2--désoxy--2--[3S-(5-phénylpentanoyloxy)tétradécanoyl]amino-3-O-(5-phénylpentanoyl)-4-O-sulfo-D-glucopyrannose,
2-désoxy-2-[3S-(7-phénylheptanoyloxy)tétradécanoyl]amino--3--O--(7-phénylheptanoyl)-4-O-sulfo-D-glucopyrannose,

2-désoxy-2--[3S--(-9--phénylnonanoyloxy)tétradécanoyl]amino-3-O-(9-phénylnonanoyl)-4-O-sulfo-D-glucopyrannose,
2-désoxy-2--[3S--(10-phényldécanoyloxy)tétradécanoyl]amino-3-O-(10-phényldécanoyl)-4-O-sulfo-D-glucopyrannose,
2-désoxy-2-[3R-(10-phényldécanoyloxy)tétradécanoyl]amino--3--O-(10-phényldécanoyl)-4-O-sulfo-D-glucopyrannose,
2-désoxy-2-[3S-(11-phénylundécanoyloxy)tétradécanoyl]-3-O-(11-phénylundécanoyl)-4-O-sulfo-D-glucopyrannose,
2--désoxy--2--[3S-(13-phényltridécanoyloxy)tétradécanoyl]amino-3-O-(13-phényltridécanoyl)-4-O-sulfo-D-glucopyrannose,
2-désoxy-2-[3R-(9-phénylnonanoyloxy)--tétradécanoyl]amino--3--O-(9-phénylnonanoyl)-4-O-sulfo-D-glucopyrannose et
2-désoxy-2-[3S-(8-phénoxyoctanoyloxy)tétradécanoyl]amino--3--O--(8-phénoxyoctanoyl)-4-O-sulfo-D-glucopyrannose.

**6.** Un procédé selon la revendication 3, selon lequel $R^2$ et $R^4$ sont tous les deux des groupes phényle.

**7.** Un procédé selon la revendication 6, selon lequel le composé est choisi dans le groupe comprenant les suivants :
2-désoxy-2-[3R-(9-phénylnonanoyloxy)-9-phénylnonanoyl]amino-3-O-(9-phénylnonanoyl)-4-O-sulfo-D-glucopyrannose et
2-désoxy-2-[3S-(9-phénylnonanoyloxy)-9-phénylnonanoyl]amino-3-O-(9-phénylnonanoyl)-4-O-sulfo-D-glucopyrannose.

**8.** Un procédé selon la revendication 3, selon lequel $R^2$ est un groupe phényle ou phénoxy substitué.

**9.** Un procédé selon la revendication 8, selon lequel le composé est choisi dans le groupe comprenant les suivants :
2-désoxy-2-[3S-[8-(4-méthoxyphényl)octanoyloxy]tétradécanoyl]amino-3-O-[8-(4-méthoxyphényl)-cotanoyl]-4-O-sulfo-D-glucopyrannose,
2-désoxy-2-[3S-[9-(4-chlorophényl)nonanoyloxy]tétradécanoyl]amino-3-O-[9-(4-chlorophényl)nonanoyl]-4-O-sulfo-D-glucopyrannose,
2-désoxy-2-[3S-[5-(4-pentylphényl)pentanoyloxy]tétradécanoyl]amino-3-O-[5-(4-pentylphényl)pentanoyl]-4-O-sulfo-D-glucopyrannose,
2-désoxy-2-[3S-[8-(4-chlorophénoxy)octanoyloxy]tétradécanoyl]amino-3-O-[8-(4-chlorophénoxy)-octanoyl]-4-O-sulfo-D-glucopyrannose et
2-désoxy--2-[3S-[8-(3,5-dichlorophénoxy)octanoyloxy]tétradécanoyl]amino-3-O-[8-(3,5-dichlorophénoxy)-octanoyl]-4-O-sulfo-D-glucopyrannose.

**10.** Un procédé selon la revendication 3, selon lequel $R^2$ est un groupe naphtyle ou naphtyloxy.

**11.** Un procédé selon la revendication 10, selon lequel le composé est choisi dans le groupe comprenant les suivants :
2-désoxy-2-[3S-[9-(1-naphtyl)nonanoyloxy]tétradécanoyl]amino--3--O-[9-(1-naphtyl)nonanoyl]-4-O-sulfo-D-glucopyrannose et
2-désoxy-2-[3S-[9--(2--naphtyl)nonanoyloxy]tétradécanoyl]amino-3-O-[9-(2-naphtyl)nonanoyl]-4-O-sulfo-D-glucopyrannose.

**12.** Un procédé selon la revendication 3, selon lequel $R^1$ est une liaison simple, $R^2$ est un atome d'hydrogène et $R^4$ est un groupe naphtyle ou naphtyloxy.

**13.** Un procédé selon la revendication 12, selon lequel le composé est choisi dans le groupe comprenant les suivants :
2-désoxy-2-[9-(1-naphtyl)nonanoyl]amino--3--O--[9--(1-naphtyl)nonanoyl]-4-O-sulfo-D-glucopyrannose et
2-désoxy-2-[9-(2-naphtyl)nonanoyl]amino-3-O-[9-(2-naphtyl)nonanoyl]-4-O-sulfo-D-glucopyrannose.

**14.** Un procédé selon la revendication 2, selon lequel R est un atome d'hydrogène.

15. Un procédé selon la revendication 14, selon lequel $R^2$ est un groupe phényle ou phénoxy et $R^4$ est un atome d'hydrogène.

16. Un procédé selon la revendication 15, selon lequel le composé est choisi dans le groupe comprenant les suivants :
2-désoxy-2-[3R-(9--phénylnonanoyloxy)tétradécanoyl]amino--3--O--(9-phénylnonanoyl)-4-O-sulfo-1,5-anhydro-D-glucitol et
2-désoxy-2-[3R-(8-phénoxyoctanoyloxy)tétradécanoyl]amino--3--O--(8-phénoxyoctanoyl)-4-O-sulfo-1,5-anhydro-D-glucitol.

17. Un procédé selon la revendication 14, selon lequel $R^2$ est un groupe phényle ou phénoxy substitué.

18. Un procédé selon la revendication 17, selon lequel le composé est choisi dans le groupe comprenant les suivants :
2-désoxy-2-[3R-[8-(4-méthoxyphényl)octanoyloxy)tétradécanoyl]amino-3-O-[8-(4-méthoxyphényl)-octanoyl]-4-O-sulfo-1,5-anhydro-D-glucitol,
2-désoxy-2-[3R-[8-(4-chlorophénoxy)octanoyloxy]tétradécanoyl]amino-3-O-[8-(4-chlorophénoxy)-octanoyl]-4-O-sulfo-1,5-anhydro-D-glucitol et
2-désoxy-2-[3R-[5-(4-pentylphényl)pentanoyloxy]tétradécanoyl]amino-3-O-[5-(4-pentylphényl)-pentanoyl]-4-O-sulfo-1,5-anhydro-D-glucitol.

19. Un procédé selon la revendication 14, selon lequel $R^2$ est un groupe naphtyle ou naphtyloxy.

20. Un procédé selon la revendication 19, selon lequel le composé est le 2-désoxy-2-[3R-[9-(1-naphtyl)-nonanoyloxy]tétradécanoyl]amino--3--O--[9-(1-naphtyl)octanoyl]-4-O-sulfo-1,5-anhydro-D--glucitol.

21. Un procédé selon la revendication 2, selon lequel R est un groupe méthoxy.

22. Un procédé selon la revendication 16, selon lequel le composé est le méthyl-2-désoxy-2-[3R-(9--phénylnonanoyloxy)tétradécanoyl]amino-3-O-(9-phénylnonanoyl)-4-O-sulfo-$\beta$-D-glucopyrannoside.

23. Un procédé selon la revendication 1, selon lequel $R^7$ et R sont tous les deux des atomes d'hydrogène.

24. Un procédé selon la revendication 23, selon lequel le composé est le 2,6-désoxy-2-[3S-(9-phénylnonanoyloxy)tétradécanoyl]amino-3-O-(9-phénylnonanoyl)-4-O-sulfo-1,5-anhydro-D-glucitol.

25. Un procédé de préparation d'une composition pharmaceutique consistant à fournir un composé de formule générale :

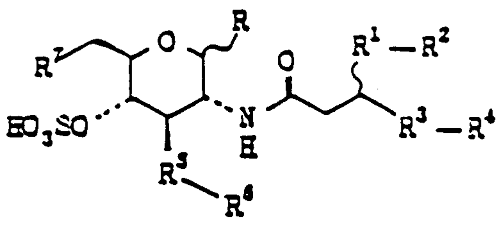

(I)

dans laquelle tous les symboles sont comme défini ci-dessus dans la revendication 1, avec un support pharmaceutique et/ou un revêtement pharmaceutique.

**Patentansprüche**
**Patentansprüche für folgende Vertragsstaaten: BE, CH, DE, FR, GB, IT, LI, LU, NL, SE**

1. Glucopyranosederivat der allgemeinen Formel

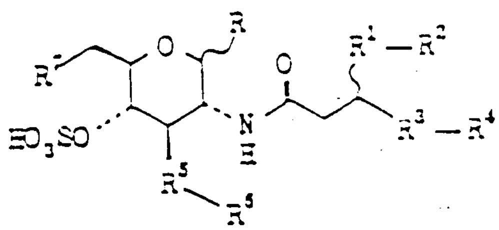

(I)

[wobei R ein Wasserstoffatom, eine Hydroxygruppe oder eine Alkoxygruppe mit 1 bis 4 Kohlenstoffatomen darstellt; $R^1$ eine Einfachbindung oder eine Oxycarbonylalkylgruppe mit 2 bis 20 Kohlenstoffatomen bedeutet; $R^2$ und $R^6$ unabhängig voneinander ein Wasserstoffatom oder eine Gruppe der allgemeinen Formeln

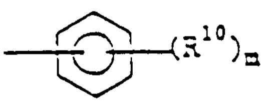 , 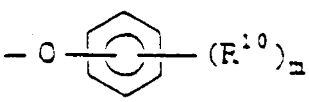 ,

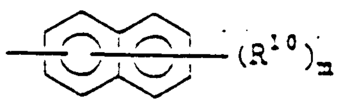 oder 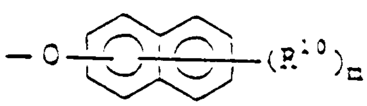

darstellen (wobei $R^{10}$ ein Wasserstoffatom, eine Alkyl- oder Alkoxygruppe mit 1 bis 7 Kohlenstoffatomen oder ein Halogenatom bedeutet, und m 1, 2 oder 3 darstellt),; $R^3$ eine Alkylengruppe mit 1 bis 20 Kohlenstoffatomen ist; $R^4$ ein Wasserstoffatom oder eine Gruppe der allgemeinen Formeln

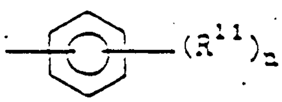 , 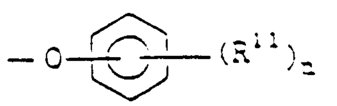 ,

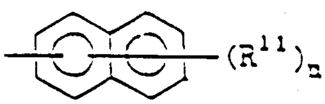 oder 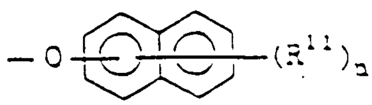

bedeutet (wobei $R^{11}$ ein Wasserstoffatom, eine Alkyl- oder Alkoxygruppe mit 1 bis 7 Kohlenstoffatomen oder ein Halogenatom darstellt, und n 1, 2 oder 3 ist); $R^5$ eine Oxycarbonylgruppe mit 2 bis 20 Kohlenstoffatomen darstellt; $R^7$ ein Wasserstoffatom oder eine Hydroxygruppe bedeutet, unter der Voraussetzung, dass $R^2$, $R^4$ und $R^6$ nicht gleichzeitig Wasserstoffatome darstellen]; oder ein nichttoxisches Salz hiervon.

**2.** Verbindung nach Anspruch 1, wobei $R^7$ eine Hydroxygruppe ist.

**3.** Verbindung nach Anspruch 2, wobei R eine Hydroxygruppe ist.

**4.** Verbindung nach Anspruch 3, wobei $R^2$ eine Phenyl- oder Phenoxygruppe ist und $R^4$ ein Wasserstoffatom ist.

**5.** Verbindung nach Anspruch 4, die aus der Reihe, bestehend aus

2-Deoxy-2-[3R-(4-phenylbutyryloxy)tetradecanoyl]amino-3-O-(4-phenylbutyryl)-4-O-sulfo-D-glucopyranose,
2-Deoxy-2-[3S-(5-phenylpentanoyloxy)tetradecanoyl]amino-3-O-(5-phenylpentanoyl)-4-O-sulfo-D-glucopyranose,
2-Deoxy-2-[3S-(7-phenylheptanoyloxy)tetradecanoyl]amino-3-O-(7-phenylheptanoyl)-4-O-sulfo-D-glucopyranose,
2-Deoxy-2-[3S-(9-phenylnonanyloxy)tetradecanoyl]amino-3-O-(9-phenylnonanyl)-4-O-sulfo-D-glucopyranose,
2-Deoxy-2-[3S-(10-phenyldecanoyloxy)tetradecanoyl]amino-3-O-(10-phenyldecanoyl)-4-O-sulfo-D-glucopyranose,
2-Deoxy-2-[3R-(10-phenyldecanoyloxy)tetradecanoyl]amino-3-O-(10-phenyldecanoyl)-4-O-sulfo-D-glucopyranose,
2-Deoxy-2-[3S-(11-phenylundecanoyloxy)tetradecanoyl]amino-3-O-(11-phenylundecanoyl)-4-O-sulfo-D-glucopyranose,
2-Deoxy-2-[3S-(13-phenyltridecanoyloxy)tetradecanoyl]amino-3-O-(13-phenyltridecanoyl)-4-O-sulfo-D-glucopyranose,
2-Deoxy-2-[3R-(9-phenylnonanoyloxy)tetradecanoyl]amino-3-O-(9-phenylnonanoyl)-4-O-sulfo-D-glucopyranose, und
2-Deoxy-2-[3S-(8-phenoxyoctanoyloxy)tetradecanoyl]amino-3-O-(8-phenoxyoctanoyl)-4-O-sulfo-D-glucopyranose,

ausgewählt ist.

**6.** Verbindung nach Anspruch 3, wobei sowohl $R^2$ als auch $R^4$ Phenylgruppen sind.

**7.** Verbindung nach Anspruch 6, die aus der Reihe

2-Deoxy-2-[3R-(9-phenylnonanoyloxy)-9-phenylnonanoyl]amino-3-O-(9-phenylnonanoyl)-4-O-sulfo-D-glucopyranose, und
2-Deoxy-2-[3S-(9-phenylnonanoyloxy)-9-phenylnonanoyl]amino-3-O-(9-phenylnonanoyl)-4-O-sulfo-D-glucopyranose

ausgewählt ist.

**8.** Verbindung nach Anspruch 3, wobei $R^2$ eine substituierte Phenyl- oder Phenoxygruppe ist.

**9.** Verbindung nach Anspruch 8, die aus der Reihe

2-Deoxy-2-(3S-[8-(4-methoxyphenyl)octanoyloxy]tetradecanoyl)amino-3-O-[8-(4-methoxyphenyl)-octanoyl]-4-O-sulfo-D-glucopyranose,
2-Deoxy-2-(3S-[9-(4-chlorophenyl)nonanoyloxy]tetradecanoyl)amino-3-O-[9-(4-chlorophenyl)nonanoyl]-4-O-sulfo-D-glucopyranose,
2-Deoxy-2-(3S-[5-(4-pentylphenyl)pentanoyloxy]tetradecanoyl)amino-3-O-[5-(4-pentylphenyl)pentanoyl]-4-O-sulfo-D-glucopyranose,
2-Deoxy-2-(3S-[8-(4-chlorophenoxy)octanoyloxy]tetradecanoyl)amino-3-O-[8-(4-chlorophenoxy)-octanoyl]-4-O-sulfo-D-glucopyranose und
2-Deoxy-2-(3S-[8-(3,5-dichlorophenoxy)octanoyloxy]tetradecanoyl)amino-3-O-[8-(3,5-dichlorophenoxy)-octanoyl]-4-O-sulfo-D-glucopyranose

ausgewählt ist.

**10.** Verbindung nach Anspruch 3, wobei $R^2$ eine Naphthyl- oder Naphthyloxygruppe ist.

**11.** Verbindung nach Anspruch 10, die aus der Reihe

2-Deoxy-2-(3S-[9-(1-naphthyl)nonanoyloxy]tetradecanoyl)amino-3-O-[9-(1-naphthyl)nonanoyl]-4-O-sulfo-D-glucopyranose und
2-Deoxy-2-(3S-[9-(2-naphthyl)nonanoyloxy]tetradecanoyl)amino-3-O-[9-(2-naphthyl)nonanoyl]-4-O-sulfo-D-glucopyranose

ausgewählt ist.

**12.** Verbindung nach Anspruch 3, wobei $R^1$ eine Einfachbindung ist, $R^2$ ein Wasserstoffatom bedeutet und

$R^4$ eine Naphthyl- oder Naphthyloxygruppe ist.

**13.** Verbindung nach Anspruch 12, die aus der Reihe

2-Deoxy-2-[9-(1-naphthyl)nonanoyl]amino-3-O-[9-(1-naphthyl)nonanoyl]-4-O-sulfo-D-glucopyranose und
2-Deoxy-2-[9-(2-naphthyl)nonanoyl]amino-3-O-[9-(2-naphthyl)nonanoyl]-4-O-sulfo-D-glucopyranose

ausgewählt ist.

**14.** Verbindung nach Anspruch 2, wobei R ein Wasserstoffatom ist.

**15.** Verbindung nach Anspruch 14, wobei $R^2$ eine Phenyl- oder Phenoxygruppe ist, und $R^4$ ein Wasserstoffatom ist.

**16.** Verbindung nach Anspruch 15, die aus der Reihe

2-Deoxy-2-[3R-(9-phenylnonanoyloxy)tetradecanoyl]amino-3-O-(9-phenylnonanoyl)-4-O-sulfo-1,5-anhydro-D-glucitol und
2-Deoxy-2-[3R-(8-phenoxyoctanoyloxy)tetradecanoyl]amino-3-O-(8-phenoxyoctanoyl)-4-O-sulfo-1,5-anhydro-D-glucitol

ausgewählt ist.

**17.** Verbindung nach Anspruch 14, wobei $R^2$ eine substituierte Phenyl- oder Phenoxygruppe ist.

**18.** Verbindung nach Anspruch 17, die aus der Reihe

2-Deoxy-2-(3R-[8-(4-methoxyphenyl)octanoyloxy]tetradecanoyl)amino-3-O-[8-(4-methoxyphenyl)-octanoyl]-4-O-sulfo-1,5-anhydro-D-glucitol,
2-Deoxy-2-(3R-[8-(4-chlorophenoxy)octanoyloxy]tetradecanoyl)amino-3-O-[8-(4-chlorophenoxy)-octanoyl]-4-O-sulfo-1,5-anhydro-D-glucitol und
2-Deoxy-2-(3R-[5-(4-pentylphenyl)pentanoyloxy]tetradecanoyl)amino-3-O-[5-(4-pentylphenyl)pentanoyl]-4-O-sulfo-1,5-anhydro-D-glucitol

ausgewählt ist.

**19.** Verbindung nach Anspruch 14, wobei $R^2$ eine Naphthyl- oder Naphthyloxygruppe ist.

**20.** 2-Deoxy-2-(3R-[9-(1-naphthyl)nonanoyloxy]tetradecanoyl)amino-3-O-[9-(1-naphthyl)octanoyl]-4-O-sulfo-1,5-anhydro-D-glucitol.

**21.** Verbindung nach Anspruch 2, wobei R eine Methoxygruppe ist.

**22.** Methyl-2-deoxy-2-[3R-(9-phenylnonanoyloxy)tetradecanoyl]amino-3-O-(9-phenylnonanoyl)-4-O-sulfo-beta-D-glucopyranosid.

**23.** Verbindung nach Anspruch 1, wobei sowohl $R^7$ als auch R Wasserstoffatome sind.

**24.** 2,6-Deoxy-2-[3S-(9-phenylnonanoyloxy)tetradecanoyl]amino-3-O-(9-phenylnonanoyl)-4-O-sulfo-1,5-anhydro-D-glucitol.

**25.** Verfahren zur Herstellung der Verbindung der allgemeinen Formel

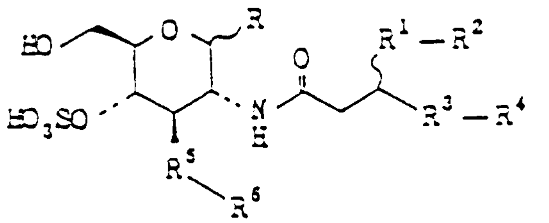

(Ia)

[wobei R ein Wasserstoffatom, eine Hydroxygruppe oder eine Alkoxygruppe mit 1 bis 4 Kohlenstoffatomen darstellt; $R^1$ eine Einfachbindung oder eine Oxycarbonylalkylgruppe mit 2 bis 20 Kohlenstoffatomen ist; $R^2$ und $R^6$ unabhängig voneinander ein Wasserstoffatom oder eine Gruppe der allgemeinen Formel

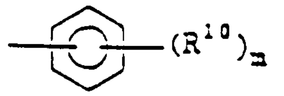 , 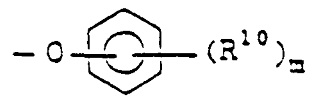 ,

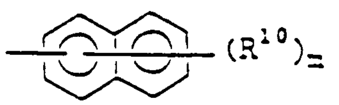 oder 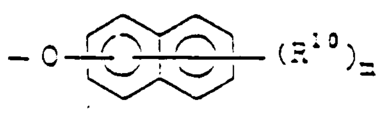

bedeutet (wobei $R^{10}$ ein Wasserstoffatom, eine Alkyl- oder Alkoxygruppe mit 1 bis 7 Kohlenstoffatomen oder ein Halogenatom ist, und m 1, 2 oder 3 darstellt); $R^3$ eine Alkylengruppe mit 1 bis 20 Kohlenstoffatomen ist; $R^4$ ein Wasserstoffatom oder eine Gruppe der allgemeinen Formel

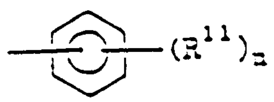 , 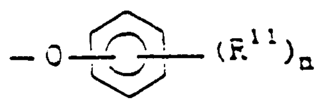 ,

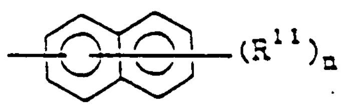 oder 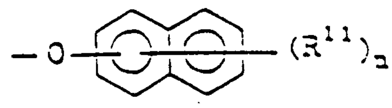

darstellt (wobei $R^{11}$ ein Wasserstoffatom, eine Alkyl- oder Alkoxygruppe mit 1 bis 7 Kohlenstoffatomen oder ein Halogenatom bedeutet, und n 1, 2 oder 3 ist); $R^5$ eine Oxycarbonylgruppe mit 2 bis 20 Kohlenstoffatomen ist, unter der Voraussetzung, dass $R^2$, $R^4$ und $R^6$ nicht gleichzeitig Wasserstoffatome darstellen), dadurch **gekennzeichnet,** dass man eine Verbindung der allgemeinen Formel (II)

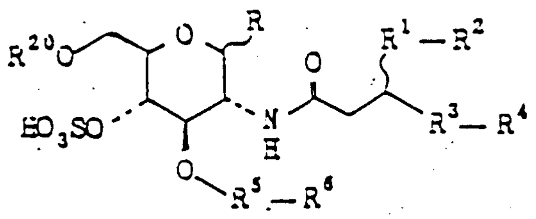

(II)

[wobei $R^{20}$ eine Trialkylsilylgruppe darstellt und die anderen Symbole wie zuvor definiert sind] unter sauren Bedingungen hydrolysiert.

**26.** Verfahren zur Herstellung der Verbindung der allgemeinen Formel (Ib)

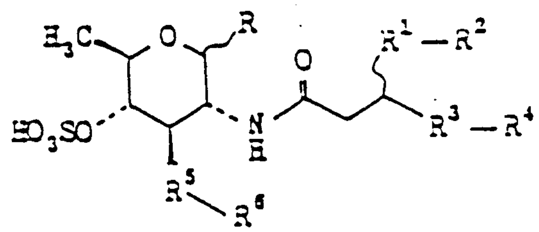

(Ib)

[wobei alle Symbole wie in Anspruch 25 definiert sind], dadurch **gekennzeichnet,** dass man eine Sulfogruppe in die Hydroxygruppe an der Position 4 einer Verbindung der allgemeinen Formel (III)

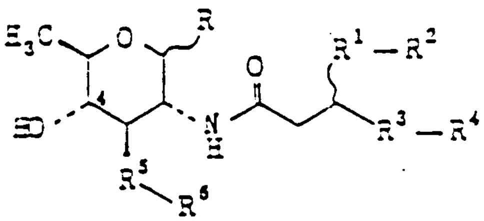

(III)

[wobei alle Symbole wie in Anspruch 25 definiert sind] einführt.

**27.** Pharmazeutische Zusammensetzung zur Verwendung bei der Steigerung der Immunität, dadurch **gekennzeichnet,** dass sie als Wirkverbindung eine Verbindung der allgemeinen Formel (I) zusammen mit einem pharmazeutischen Träger und/oder einer pharmazeutischen Beschichtung umfasst.

**28.** Pharmazeutiche Zusammensetzung zur Verwendung bei der Behandlung von Tumoren, dadurch **gekennzeichnet,** dass sie als Wirkverbindung eine Verbindung der allgemeinen Formel (I) zusammen mit einem pharmazeutischen Träger und/oder pharmazeutischen Beschichtung umfasst.

**29.** Verbindung gemäss einem der Ansprüche 1 bis 24 zur Verwendung als ein Arzneimittel zur Steigerung der Immunität.

**30.** Verbindung nach einem der Ansprüche 1 bis 24 zur Verwendung als ein Arzneimittel zur Behandlung eines Tumors.

**Patentansprüche für folgende Vertragsstaaten: AT, ES, GR**

**1.** Verfahren zur Herstellung eines Glucopyranosederivates der allgemeinen Formel (I)

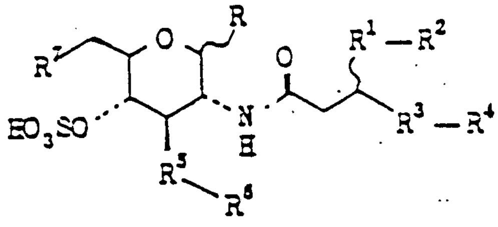

(I)

[wobei R ein Wasserstoffatom, eine Hydroxygruppe oder eine Alkoxygruppe mit 1 bis 4 Kohlenstoffatomen darstellt; $R^1$ eine Einfachbindung oder eine Oxycarbonylalkylgruppe mit 2 bis 20 Kohlenstoffatomen bedeutet; $R^2$ und $R^6$ unabhängig voneinander ein Wasserstoffatom oder eine Gruppe der allgemeinen Formel

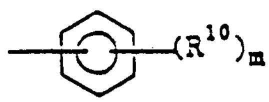 , 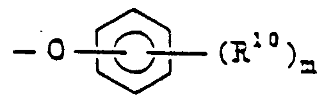 ,

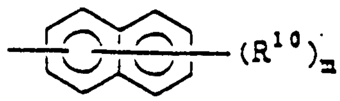 oder 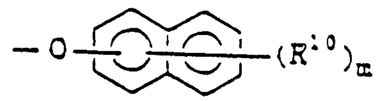

bedeuten (wobei $R^{10}$ ein Wasserstoffatom, eine Alkyl- oder Alkoxygruppe mit 1 bis 7 Kohlenstoffatomen oder ein Halogenatom ist, und m 1, 2 oder 3 bedeutet); $R^3$ eine Alkylengruppe mit 1 bis 20 Kohlenstoffatomen ist; $R^4$ ein Wasserstoffatom oder eine Gruppe der allgemeinen Formel

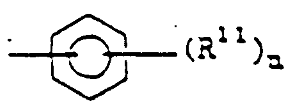 , 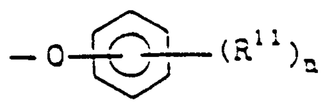 ,

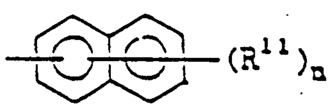 oder 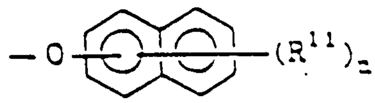

darstellt (wobei $R^{11}$ ein Wasserstoffatom, eine Alkyl- oder Alkoxygruppe mit 1 bis 7 Kohlenstoffatomen oder ein Halogenatom bedeutet, und n 1, 2 oder 3 ist); $R^5$ eine Oxycarbonylgruppe mit 2 bis 20 Kohlenstoffatomen bedeutet; $R^7$ ein Wasserstoffatom oder eine Hydroxygruppe ist, unter der Voraussetzung, dass $R^2$, $R^4$ und $R^6$ nicht gleichzeitig Wasserstoffatome darstellen); oder eines nicht-toxischen Salzes hiervon, dadurch **gekennzeichnet,** dass

(a) wenn $R^7$ eine Hydroxygruppe ist, eine Verbindung der allgemeinen Formel (II)

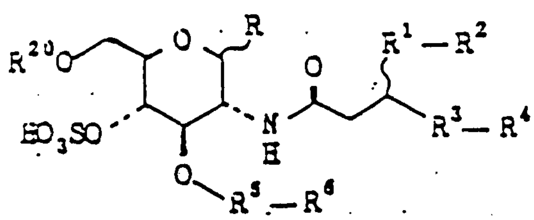

(II)

[wobei $R^{20}$ eine Trialkylsilylgruppe darstellt und die anderen Symbole wie zuvor definiert sind] unter sauren Bedingungen hydrolysiert wird; oder

(b) wenn $R^7$ ein Wasserstoffatom ist, eine Sulfogruppe in die Hydroxygruppe an der Position 4 einer Verbindung der allgemeinen Formel (III)

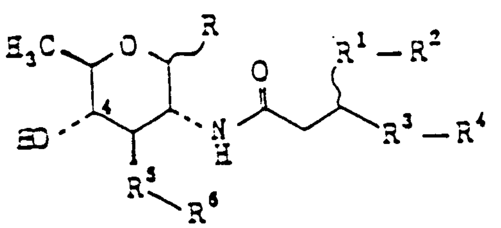

(III)

[wobei alle Symbole wie oben definiert sind] eingeführt wird.

**2.** Verfahren nach Anspruch 1, wobei $R^7$ eine Hydroxygruppe ist.

3. Verfahren nach Anspruch 2, wobei R eine Hydroxygruppe ist.

4. Verfahren nach Anspruch 3, wobei $R^2$ eine Phenyl- oder Phenoxygruppe ist und $R^4$ ein Wasserstoffatom ist.

5. Verfahren nach Anspruch 4, wobei die Verbindung aus der Reihe

2-Deoxy-2-[3R-(4-phenylbutyryloxy)tetradecanoyl]amino-3-O-(4-phenylbutyryl)-4-O-sulfo-D-glucopyranose,
2-Deoxy-2-[3S-(5-phenylpentanoyloxy)tetradecanoyl]amino-3-O-(5-phenylpentanoyl)-4-O-sulfo-D-glucopyranose,
2-Deoxy-2-[3S-(7-phenylheptanoyloxy)tetradecanoyl]amino-3-O-(7-phenylheptanoyl)-4-O-sulfo-D-glucopyranose,
2-Deoxy-2-[3S-(9-phenylnonanyloxy)tetradecanoyl]amino-3-O-(9-phenylnonanyl)-4-O-sulfo-D-glucopyranose,
2-Deoxy-2-[3S-(10-phenyldecanoyloxy)tetradecanoyl]amino-3-O-(10-phenyldecanoyl)-4-O-sulfo-D-glucopyranose,
2-Deoxy-2-[3R-(10-phenyldecanoyloxy)tetradecanoyl]amino-3-O-(10-phenyldecanoyl)-4-O-sulfo-D-glucopyranose,
2-Deoxy-2-[3S-(11-phenylundecanoyloxy)tetradecanoyl]amino-3-O-(11-phenylundecanoyl)-4-O-sulfo-D-glucopyranose,
2-Deoxy-2-[3S-(13-phenyltridecanoyloxy)tetradecanoyl]amino-3-O-(13-phenyltridecanoyl)-4-O-sulfo-D-glucopyranose,
2-Deoxy-2-[3R-(9-phenylnonanoyloxy)tetradecanoyl]amino-3-O-(9-phenylnonanoyl)-4-O-sulfo-D-glucopyranose, und
2-Deoxy-2-[3S-(8-phenoxyoctanoyloxy)tetradecanoyl]amino-3-O-(8-phenoxyoctanoyl)-4-O-sulfo-D-glucopyranose,

ausgewählt wird.

6. Verfahren nach Anspruch 3, wobei sowohl $R^2$ als auch $R^4$ Phenylgruppen sind.

7. Verfahren nach Anspruch 6, wobei die Verbindung aus der Reihe

2-Deoxy-2-[3R-(9-phenylnonanoyloxy)-9-phenylnonanoyl]amino-3-O-(9-phenylnonanoyl)-4-O-sulfo-D-glucopyranose, und
2-Deoxy-2-[3S-(9-phenylnonanoyloxy)-9-phenylnonanoyl]amino-3-O-(9-phenylnonanoyl)-4-O-sulfo-D-glucopyranose

ausgewählt wird.

8. Verfahren nach Anspruch 3, wobei $R^2$ eine substituierte Phenyl- oder Phenoxygruppe ist.

9. Verfahren nach Anspruch 8, wobei die Verbindung aus der Reihe

2-Deoxy-2-(3S-[8-(4-methoxyphenyl)octanoyloxy]tetradecanoyl)amino-3-O-[8-(4-methoxyphenyl)-octanoyl]-4-O-sulfo-D-glucopyranose,
2-Deoxy-2-(3S-[9-(4-chlorophenyl)nonanoyloxy]tetradecanoyl)amino-3-O-[9-(4-chlorophenyl)nonanoyl]-4-O-sulfo-D-glucopyranose,
2-Deoxy-2-(3S-[5-(4-pentylphenyl)pentanoyloxy]tetradecanoyl)amino-3-O-[5-(4-pentylphenyl)pentanoyl]-4-O-sulfo-D-glucopyranose,
2-Deoxy-2-(3S-[8-(4-chlorophenoxy)octanoyloxy]tetradecanoyl)amino-3-O-[8-(4-chlorophenoxy)-octanoyl]-4-O-sulfo-D-glucopyranose und
2-Deoxy-2-(3S-[8-(3,5-dichlorophenoxy)octanoyloxy]tetradecanoyl)amino-3-O-[8-(3,5-dichlorophenoxy)-octanoyl]-4-O-sulfo-D-glucopyranose

ausgewählt wird.

**10.** Verfahren nach Anspruch 3, wobei $R^2$ eine Naphthyl- oder Naphthyloxygruppe ist.

**11.** Verfahren nach Anspruch 10, wobei die Verbindung aus der Reihe

2-Deoxy-2-(3S-[9-(1-naphthyl)nonanoyloxy]tetradecanoyl)amino-3-O-[9-(1-naphthyl)nonanoyl]-4-O-sulfo-D-glucopyranose und
2-Deoxy-2-(3S-[9-(2-naphthyl)nonanoyloxy]tetradecanoyl)amino-3-O-[9-(2-naphthyl)nonanoyl]-4-O-sulfo-D-glucopyranose

ausgewählt wird.

**12.** Verfahren nach Anspruch 3, wobei $R^1$ eine Einfachbindung ist, $R^2$ ein Wasserstoffatom bedeutet und $R^4$ eine Naphthyl- oder Naphthyloxygruppe ist.

**13.** Verfahren nach Anspruch 12, wobei die Verbindung aus der Reihe

2-Deoxy-2-[9-(1-naphthyl)nonanoyl]amino-3-O-[9-(1-naphthyl)nonanoyl]-4-O-sulfo-D-glucopyranose und
2-Deoxy-2-[9-(2-naphthyl)nonanoyl]amino-3-O-[9-(2-naphthyl)nonanoyl]-4-O-sulfo-D-glucopyranose

ausgewählt wird.

**14.** Verfahren nach Anspruch 2, wobei R ein Wasserstoffatom ist.

**15.** Verfahren nach Anspruch 14, wobei $R^2$ eine Phenyl- oder Phenoxygruppe ist, und $R^4$ ein Wasserstoffatom ist.

**16.** Verfahren nach Anspruch 15, wobei die Vrbindung aus der Reihe

2-Deoxy-2-[3R-(9-phenylnonanoyloxy)tetradecanoyl]amino-3-O-(9-phenylnonanoyl)-4-O-sulfo-1,5-anhydro-D-glucitol und
2-Deoxy-2-[3R-(8-phenoxyoctanoyloxy)tetradecanoyl]amino-3-O-(8-phenoxyoctanoyl)-4-O-sulfo-1,5-anhydro-D-glucitol

ausgewählt wird.

**17.** Verfahren nach Anspruch 14, wobei $R^2$ eine substituierte Phenyl- oder Phenoxygruppe ist.

**18.** Verfahren nach Anspruch 17, wobei die Verbindung aus der Reihe

2-Deoxy-2-(3R-[8-(4-methoxyphenyl)octanoyloxy]tetradecanoyl)amino-3-O-[8-(4-methoxyphenyl)-octanoyl]-4-O-sulfo-1,5-anhydro-D-glucitol,
2-Deoxy-2-(3R-[8-(4-chlorophenoxy)octanoyloxy]tetradecanoyl)amino-3-O-[8-(4-chlorophenoxy)-octanoyl]-4-O-sulfo-1,5-anhydro-D-glucitol und
2-Deoxy-2-(3R-[5-(4-pentylphenyl)pentanoyloxy]tetradecanoyl)amino-3-O-[5-(4-pentylphenyl)pentanoyl]-4-O-sulfo-1,5-anhydro-D-glucitol

ausgewählt wird.

**19.** Verfahren nach Anspruch 14, wobei $R^2$ eine Naphthyl- oder Naphthyloxygruppe ist.

**20.** Verfahren nach Anspruch 19, wobei die Verbindung 2-Deoxy-2-(3R-[9-(1-naphthyl)nonanoyloxy]-tetradecanoyl)amino-3-O-[9-(1-naphthyl)octanoyl]-4-O-sulfo-1,5-anhydro-D-glucitol ist.

**21.** Verfahren nach Anspruch 2, wobei R eine Methoxygruppe ist.

**22.** Verfahren nach Anspruch 16, wobei die Verbindung Methyl-2-deoxy-2-[3R-(9-phenylnonanoyloxy)-tetradecanoyl]amino-3-O-(9-phenylnonanoyl)-4-O-sulfo-beta-D-glucopyranosid ist.

**23.** Verfahren nach Anspruch 1, wobei sowohl $R^7$ als auch R Wasserstoffatome sind.

**24.** Verfahren nach Anspruch 23, wobei die Verbindung 2,6-Deoxy-2-[3S-(9-phenylnonanoyloxy)-tetradecanoyl]amino-3-O-(9-phenylnonanoyl)-4-O-sulfo-1,5-anhydro-D-glucitol ist.

**25.** Verfahren zur Herstellung einer pharmazeutischen Zusammensetzung, das das Bereitstellen einer Verbindung der allgemeinen Formel

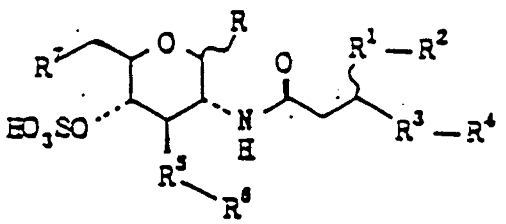

(I)

wobei alle Symbole wie in Anspruch 1 definiert sind, mit einem pharmazeutischen Träger und/oder einer pharmazeutischen Beschichtung umfasst.